(19) Europäisches Patentamt
European Patent Office
Office européen des brevets

(11) EP 4 768 496 A1

(12) **EUROPEAN PATENT APPLICATION**
published in accordance with Art. 153(4) EPC

(43) Date of publication:
**01.07.2026 Bulletin 2026/27**

(21) Application number: **24912968.5**

(22) Date of filing: **26.12.2024**

(51) International Patent Classification (IPC):
***C07K 1/02*** *(2006.01)* ***A61K 38/02*** *(2006.01)*
***A61K 38/08*** *(2019.01)* ***A61K 38/10*** *(2006.01)*
***C07K 1/10*** *(2006.01)*

(52) Cooperative Patent Classification (CPC):
**A61K 38/02; A61K 38/08; A61K 38/10; C07K 1/02; C07K 1/10**

(86) International application number:
**PCT/JP2024/046067**

(87) International publication number:
**WO 2025/143082 (03.07.2025 Gazette 2025/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.12.2023 JP 2023221705**

(71) Applicant: **Chugai Seiyaku Kabushiki Kaisha**
**Kita-ku**
**Tokyo 115-8543 (JP)**

(72) Inventor: **KOMIYA, Shio**
**Tokyo 115-8543 (JP)**

(74) Representative: **Vossius & Partner**
**Patentanwälte Rechtsanwälte mbB**
**Siebertstraße 3**
**81675 München (DE)**

(54) **METHOD FOR PRODUCING PEPTIDE COMPOUND USING TWO OR MORE ADDITIVES**

(57) Provided is a method for producing a peptide compound or a salt thereof, comprising a step (linking step) of linking an amino group of a first amino acid or peptide and a carboxy group of a second amino acid or peptide with an amide bond, wherein a condensing agent and two or more additives are used in the linking step, and pKa of the additive is between 0 and 10.0.

EP 4 768 496 A1

## Description

### Technical Field

The present invention relates to a method for producing a peptide compound using two or more additives.

### Background Art

Recently, it has been found that the metabolic stability and membrane permeability of the peptide is improved by using non-natural amino acids such as cyclic peptides (Non Patent Literatures 1 and 2). Especially, among them, cyclic peptide compounds containing N-substituted amino acids have been suggested to have drug-likeness such as metabolic stability and membrane permeability, and to be useful for the creation of inhibitors of protein-protein interactions (Patent Literature 1, Non Patent Literature 3).

As peptide compounds have attracted attention as pharmaceuticals, the methods for producing peptide compounds have also been viewed as important (Non Patent Literature 4). Synthesis of peptide compounds is generally carried out by extending the peptide chain by repeating the step of linking the carboxy group at the C-terminus of an amino acid to the amino group at the N-terminus of another amino acid or peptide. Known examples of the method for extending a peptide chain include a method using a phosphate-based condensing agent as a condensing agent, a method using HOPO (2-hydroxypyridine-N-oxide) as an additive (Non Patent Literature 5), and a method using Oxyma (ethyl 2-cyano-2-(hydroxyimino)acetate) (Non Patent Literature 6). It is also known a method using two types, HOPO and Oxyma, as additives, and using carbodiimide as a condensing agent (Patent Literature 2). However, all of these methods are a method of subjecting an amino acid to an amide bond formation reaction sequentially by one residue at a time.

Another known method of extending the peptide chain is a method called fragment coupling, which subjects peptide fragments each consisting of a plurality of amino acid residues to an amide bond formation reaction to each other. The fragment coupling method has the advantage of the high total yield of the final product. The greater the number of amino acid residues in the desired peptide compound, the better the advantage. However, under conventional amide bond formation conditions, the increase of impurities due to the racemization at the $\alpha$-position of the amino acid residue and the resulting decrease in the yield and purity of the target product significantly increases the difficulty of purification, resulting in an increase in the time and effort involved in production. In addition, when the N-terminus or the C-terminus is N-substituted amino acid and it becomes the reaction point of the amide bond formation reaction in the fragment coupling, reduction in reactivity has been a problem (Non Patent Literature 7). Thus, when synthesizing a peptide compound containing an N-substituted amino acid, the synthesis method has been limited to a method of subjecting the amino acids to amide bond formation reactions sequentially by one residue at a time or a method of fragment coupling with residues that do not cause epimerization.

### Citation List

#### Patent Literature

Patent Literature 1: International Publication No. WO 2013/100132
Patent Literature 2: International Publication No. WO 2023/117904

#### Non Patent Literature

Non Patent Literature 1: Acc. Chem. Res., 2008, 41, 1331-1342.
Non Patent Literature 2: Angew. Chem. Int. Ed., 2013, 52, 254-269.
Non Patent Literature 3: Chem. Rev., 2019, 119, 10360-10391.
Non Patent Literature 4: Amino Acids, Peptides and Proteins in Organic Chemistry: Building Blocks, Catalysis and Coupling Chemistry, Volume 3, 2011.
Non Patent Literature 5: Tetrahedron Letters., 2020, 152299.
Non Patent Literature 6: Tetrahedron Letters., 2021, 153462.
Non Patent Literature 7: J. Peptide Res., 2005, 65, 153-166.

### Summary of Invention

Technical Problem

The present invention has been made in view of such circumstances. In one aspect, a problem to be solved is to establish a novel synthetic method for a peptide compound. In one aspect, a problem to be solved is to establish a novel synthetic method suitable for fragment coupling of peptide compounds. In one aspect, a problem to be solved is to establish a synthetic method in which all amino acid residues constituting a peptide compound retains high diastereoselectivity even by fragment coupling. In one aspect, a problem to be solved is to establish a method for synthesizing a peptide compound with high yield even by fragment coupling. In one aspect, a problem to be solved is to establish a method for synthesizing a peptide compound with high yield by fragment coupling while preventing racemization at the $\alpha$-carbon of the carboxy group of the reaction substrate. In one aspect, a problem to be solved is to establish a synthetic method suitable for fragment coupling a peptide compound containing an N-substituted amino acid at the N-terminus or the C-terminus. In one aspect, a problem to be solved is to establish a synthetic method suitable for fragment coupling a peptide compound in which a nitrogen atom bound to the $\alpha$-carbon of the carboxy group of the reaction substrate is substituted with an alkyl.

Solution to Problem

The present inventors have intensively studied to solve the above problem. As a result, the present inventors have found a method of solving at least one of the problems above by performing an amide bond formation reaction in a reaction system where a condensing agent and two or more additives are present. In addition, the present inventors have found reaction conditions that can inhibit the production of byproducts in condensation reactions.

In one non-limiting specific aspect, the present invention encompasses the following.

[1] A method for producing a peptide compound or a salt thereof, comprising a step (linking step) of linking an amino group of a first amino acid or peptide and a carboxy group of a second amino acid or peptide with an amide bond, wherein a condensing agent and two or more additives are used in the linking step, and pKa of the additive is between 0 and 10.0.

[1-1] A method for producing a peptide compound or a salt thereof, comprising a step (linking step) of linking an amino group of a first amino acid or peptide and a carboxy group of a second amino acid or peptide with an amide bond, wherein a condensing agent and two or more additives are used in the linking step.

[2] The method according to [1] or [1-1], wherein the condensing agent is a phosphorus-based condensing agent.

[3] The method according to [2], wherein the phosphorus-based condensing agent is represented by the following formula (1):

[Formula 1]

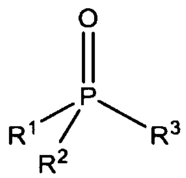

(1)

wherein,

$R^1$ is $C_1$-$C_6$ alkoxy, $C_6$-$C_{10}$ aryloxy, $C_6$-$C_{10}$ aryl, or di-$C_1$-$C_6$ alkylamino;
$R^2$ is $C_1$-$C_6$ alkoxy, $C_6$-$C_{10}$ aryloxy, $C_6$-$C_{10}$ aryl, or di-$C_1$-$C_6$ alkylamino; and
$R^3$ is halogen, optionally substituted $C_6$-$C_{10}$ aryloxy, or azide.

[4] The method according to [3], wherein

$R^1$ is ethoxy, phenyloxy, phenyl, or dimethylamino;

$R^2$ is ethoxy, phenyloxy, phenyl, or dimethylamino; and

$R^3$ is chloro, pentafluorophenyloxy, or azide.

[5] The method according to any of [2] to [4], wherein the phosphorus-based condensing agent is at least one selected from the group consisting of diphenyl chlorophosphate (DPPC), diethyl chlorophosphate (DEPC), dimethyl chlorophosphate, diisopropyl chlorophosphate, dibenzyl chlorophosphate, diphenylphosphoryl azide (DPPA), pentafluorophenyl diphenyl phosphinate (FDPP), 2-chloro-2-oxo-1,3,2-disoxaphosphorane, dimethylphosphinic chloride, diethylphosphinic chloride, dicyclohexylphosphinic chloride, and diphenylphosphinic chloride ($Ph_2P(O)Cl$).

[5-1] The method according to any of [2] to [5], wherein the phosphorus-based condensing agent is at least one selected from the group consisting of diphenyl chlorophosphate (DPPC), diethyl chlorophosphate (DEPC), diphenylphosphoryl azide (DPPA), pentafluorophenyl diphenyl phosphinate (FDPP), and diphenylphosphinic chloride ($Ph_2P(O)Cl$).

[6] The method according to any of [2] to [5-1], wherein the phosphorus-based condensing agent is at least one selected from the group consisting of diphenyl chlorophosphate (DPPC) and diethyl chlorophosphate (DEPC).

[7] The method according to any of [1] to [6], wherein the condensing agent is used in 0.5 to 5.0 molar equivalents relative to the first amino acid or peptide.

[8] The method according to any of [1] to [7], wherein the additive is a nucleophile that reacts with the carboxy group to form an active ester.

[8-1] The method according to any of [1] to [8], wherein the additives are two or more selected from the group consisting of 2-hydroxypyridine-N-oxide (HOPO), a HOPO derivative, 1-hydroxy-7-azabenzotriazole (HOAt), 1-hydroxybenzotriazole (HOBt), 3,4-dihydro-3-hydroxy-4-oxo-benzotriazine (HOOBt), ethyl 1-hydroxy-1H-1,2,3-triazole-4-carboxylate (HOCt), 2,2,3,3,3-pentafluoro-1-propanol (PfpOH), ethyl 2-cyano-2-(hydroxyimino)acetate (Oxyma), 5-(hydroxyimino)-1,3-dimethylpyrimidine-2,4,6-(1H,3H,5H)-trion (Oxyma-B), 4-nitrophenol, pentafluorophenol, ethyl (hydroxyimino)cyanoacetate potassium salt (K-Oxyma), 4,6-dimethoxy-1,3,5-triazin-2-ol, 2-hydroxypyridine, trimethylsilanol, and 2-hydroxy-5-nitropyridine.

[8-2] The method according to any of [1] to [8-1], wherein the additives are two or more selected from the group consisting of 2-hydroxypyridine-N-oxide (HOPO), a HOPO derivative, 3,4-dihydro-3-hydroxy-4-oxo-benzotriazine (HOOBt), 2,2,3,3,3-pentafluoro-1-propanol (PfpOH), ethyl 2-cyano-2-(hydroxyimino)acetate (Oxyma), 5-(hydroxyimino)-1,3-dimethylpyrimidine-2,4,6-(1H,3H,5H)-trion (Oxyma-B), and pentafluorophenol.

[8-3] The method according to [8], [8-1] or [8-2], wherein the additives are two additives.

[9] The method according to any of [1] to [8-3], wherein one of the additives is HOPO or a HOPO derivative.

[9-1] The method according to any of [1] to [8-3], wherein one of the additives is HOPO.

[9-2] The method according to any of [1] to [8-3], wherein one of the additives is HOPO and the other one or more is selected from the group consisting of Oxyma, HOAt, HOOBt, HOCt, PfpOH, Oxyma-B, pentafluorophenol, and K-Oxyma.

[9-3] The method according to any of [1] to [8-3], wherein one of the additives is Oxyma.

[9-4] The method according to any of [1] to [8-3], wherein one of the additives is Oxyma and the other one or more is selected from the group consisting of HOPO, a HOPO derivative, HOOBt, HOCt, Oxyma-B, pentafluorophenol, PfpOH, 4,6-dimethoxy-1,3,5-triazin-2-ol, 2-hydroxypyridine, and 2-hydroxy-5-nitropyridine.

[9-5] The method according to any of [1] to [8-3], wherein one of the additives is Oxyma and the other one or more is selected from the group consisting of HOPO, HOOBt, HOCt, Oxyma-B, and pentafluorophenol.

[9-6] The method according to any of [1] to [8-3], wherein the additives are Oxyma and HOPO.

[9-7] The method according to any of [8-1] to [9], wherein the HOPO derivative is selected from the group consisting of 5-F-HOPO, 5-Cl-HOPO, 5-Br-HOPO, 5-CF3-HOPO, 5-CO2Me-HOPO, and 4-Br-HOPO.

[10] The method according to any of [1] to [9-7], wherein each of the one or two or more additives is used in 0.1 to 5.0 molar equivalents relative to the first amino acid or peptide.

[11] The method according to any of [1] to [10], wherein the second amino acid or peptide is a peptide containing two or more amino acid residues.

[12] The method according to any of [1] to [11], wherein an amino group containing a nitrogen atom located at β position of the carboxy group of the second amino acid or peptide is represented by formula: $-NR^5-$, wherein $R^5$ is a hydrogen atom, linear $C_1$-$C_6$ alkyl, branched $C_3$-$C_6$ alkyl, or $C_3$-$C_8$ cycloalkyl.

[13] The method according to any of [1] to [12], wherein an amino group containing a nitrogen atom located at β position of the carboxy group of the second amino acid or peptide is represented by formula: $-NR^5-$, wherein $R^5$ is a hydrogen atom or linear $C_1$-$C_4$ alkyl.

[14] The method according to any of [1] to [13], wherein an amino group containing a nitrogen atom located at β position of the carboxy group of the second amino acid or peptide is represented by formula: $-NR^5-$, wherein $R^5$ is a hydrogen atom or a methyl group.

[15] The method according to any of [1] to [14], wherein the first amino acid or peptide is a peptide containing two or more amino acid residues.

[16] The method according to any of [1] to [15], wherein the amino group of the first amino acid or peptide is an amino group represented by formula: $-NR^6R^7$, wherein $R^6$ is a hydrogen atom, and $R^7$ is a hydrogen atom, linear $C_1$-$C_6$ alkyl,

branched $C_3$-$C_6$ alkyl, or $C_3$-$C_8$ cycloalkyl.

[17] The method according to any of [1] to [16], wherein the amino group of the first amino acid or peptide is an amino group represented by formula: $-NR^6R^7$, wherein $R^6$ is a hydrogen atom, and $R^7$ is a hydrogen atom or linear $C_1$-$C_4$ alkyl.

[18] The method according to any of [1] to [17], wherein the amino group of the first amino acid or peptide is an amino group represented by formula: $-NR^6R^7$, wherein $R^6$ is a hydrogen atom, and $R^7$ is a hydrogen atom, methyl, or ethyl.

[19] The method according to any of [1] to [18], wherein the amino group of the first amino acid or peptide is an amino group represented by formula: $-NR^6R^7$, wherein $R^6$ is a hydrogen atom, and $R^7$ is a hydrogen atom or methyl.

[20] The method according to any of [1] to [19], wherein the amino group of the first amino acid or peptide is an amino group represented by formula: $-NR^6R^7$, wherein $R^6$ is a hydrogen atom, and $R^7$ is methyl.

[21] The method according to any of [1] to [20], wherein $\alpha$-carbon of the carboxy group of the second amino acid or peptide is represented by formula: $-CR^8R^9-$, wherein $R^8$ and $R^9$ are identical or different and each is a hydrogen atom, linear $C_1$-$C_4$ alkyl, optionally substituted branched $C_3$-$C_6$ alkyl, optionally substituted phenyl $C_1$-$C_2$ alkyl, or optionally substituted 5- to 6-membered heteroaryl $C_1$-$C_2$ alkyl.

[22] The method according to any of [1] to [21], wherein $\alpha$-carbon of the carboxy group of the second amino acid or peptide is represented by formula: $-CR^8R^9-$, wherein $R^8$ and $R^9$ are identical or different and each is a hydrogen atom, linear $C_1$-$C_3$ alkyl, isopropyl, or optionally substituted benzyl.

[23] The method according to any of [1] to [22], wherein $\alpha$-carbon of the carboxy group of the second amino acid or peptide is represented by formula: $-CR^8R^9-$, wherein $R^8$ is a hydrogen atom, and $R^9$ is a hydrogen atom, methyl, benzyl, or p-fluorobenzyl.

[24] The method according to any of [1] to [23], wherein the carboxy group of the first amino acid or peptide is protected.

[25] The method according to any of [1] to [24], wherein the amino group of the second amino acid or peptide is protected.

[26] The method according to any of [1] to [25], wherein a base is used in the linking step.

[27] The method according to [26], wherein pKa of a conjugate acid of the base used in the linking step is between 0 and 15.0, more preferably between 3 and 13.0, and further preferably between 5 and 11.5.

[27-1] The method according to any of [1] to [27], wherein the pKa is a measured value as pKa when water at 25°C is used as a solvent.

[28] The method according to [26] or [27], wherein the base used in the linking step is an organic base.

[28-1] The method according to [28], wherein the organic base used in the linking step is at least one selected from the group consisting of dicyclohexylmethylamine, diisopropylethylamine, triethylamine, 1-methylpiperidine, N-methylmorpholine, 4-N,N-dimethylaminopyridine, 2,6-lutidine, 2,4,6-collidine, N-methylimidazole, 1,8-diazabicyclo [5.4.0]-7-undecene, 1,5-diazabicyclo[4.3.0]-5-nonene, 1,1,3,3-tetramethylguanidine, 2-tert-butyl-1,1,3,3-tetramethylguanidine, and 7-methyl-1,5,7-triazabicyclo[4.4.0]deca-5-ene.

[28-2] The method according to [28] or [28-1], wherein the organic base used in the linking step is at least one selected from the group consisting of dicyclohexylmethylamine, diisopropylethylamine, triethylamine, 1-methylpiperidine, and 2-tert-butyl-1,1,3,3-tetramethylguanidine.

[29] The method according to [26] or [27], wherein the base used in the linking step is an inorganic base, preferably an alkali metal carbonate or alkali metal phosphate.

[29-1] The method according to [29], wherein the inorganic base used in the linking step is potassium carbonate or potassium phosphate.

[29-2] The method according to any of [28] to [29-1], wherein the organic base or inorganic base used in the linking step is used in 1.1 to 10.0 molar equivalents, preferably 2.0 to 9.0 molar equivalents, and further preferably 3.0 to 8.0 molar equivalents, relative to the first amino acid or peptide.

[30] The method according to any of [1] to [29], wherein the linking step is performed by a liquid-phase synthesis.

[31] The method according to [30], wherein the liquid-phase synthesis is performed in an organic solvent.

[32] The method according to [31], wherein the organic solvent used in the linking step is at least one selected from the group consisting of acetonitrile, isopropyl acetate, ethyl acetate, butyl acetate, methyl t-butyl ether, diethyl ether, dichloromethane, tetrahydrofuran, 2-methyltetrahydrofuran, N,N-dimethylformamide, N,N-dimethylacetamide, N-methylpyrrolidone, dimethylsulfoxide, $\alpha,\alpha,\alpha$-trifluorotoluene, toluene, hexane, and cyclopentyl methyl ether, or a mixed solvent thereof.

[33] The method according to [32], wherein the organic solvent used in the linking step is at least one selected from the group consisting of acetonitrile, isopropyl acetate, methyl t-butyl ether, dichloromethane, tetrahydrofuran, 2-methyltetrahydrofuran, N,N-dimethylformamide, N,N-dimethylacetamide, $\alpha,\alpha,\alpha$-trifluorotoluene, toluene, and cyclopentyl methyl ether, or a mixed solvent thereof.

[33-1] The method according to [32] or [33], wherein the organic solvent used in the linking step is at least one selected from the group consisting of isopropyl acetate, tetrahydrofuran, 2-methyltetrahydrofuran, $\alpha,\alpha,\alpha$-trifluorotoluene, and toluene, or a mixed solvent thereof.

[33-2] The method according to any of [32] to [33-1], wherein the organic solvent used in the linking step is at least one selected from the group consisting of 2-methyltetrahydrofuran, $\alpha,\alpha,\alpha$-trifluorotoluene, and toluene, or a mixed solvent thereof.

[33-3] The method according to any of [32] to [33-2], wherein the organic solvent used in the linking step is used at 50 to 100 mg/mL relative to the first amino acid or peptide.

[34] The method according to any of [1] to [33], further comprising a deprotection step.

[35] The method according to any of [1] to [34], wherein the linking step is repeated two or more times.

[36] The method according to any of [1] to [35], wherein the peptide compound or a salt thereof produced contains 8 to 20 amino acid residues.

[37] The method according to any of [1] to [36], wherein the peptide compound or a salt thereof produced contains 11 to 14 amino acid residues.

[38] The method according to any of [1] to [37], wherein the peptide compound produced contains at least one non-natural amino acid residue.

[39] The method according to any of [1] to [38], wherein the peptide compound produced contains at least four non-natural amino acid residues.

[40] The method according to any of [1] to [39], wherein the peptide compound produced contains at least five non-natural amino acid residues.

[41] The method according to any of [38] to [40], wherein the non-natural amino acid residue is an N-methylamino acid residue.

[42] The method according to any of [1] to [41], wherein the peptide compound or a salt thereof produced contains a cyclic portion composed of 4 or more amino acid residues, and wherein an amide bond with which the amino group of the first amino acid or peptide and the carboxy group of the second amino acid or peptide are linked is contained in the cyclic portion at 1 to 7 locations.

[43] The method according to any of [1] to [42], which is performed using a flow reaction apparatus.

[44] A peptide compound or a salt thereof produced by the method according to any of [1] to [43].

[45] A pharmaceutical composition comprising the peptide compound or a salt thereof produced by the method according to any of [1] to [43].

[46] A linking agent comprising a condensing agent and two or more additives, for use in a step of linking an amino group of a first amino acid or peptide and a carboxy group of a second amino acid or peptide with an amide bond.

[47] The linking agent according to [46], wherein the amino group of the first amino acid or peptide is an amino group represented by formula: $-NR^6R^7$, wherein $R^6$ is a hydrogen atom, and $R^7$ is a hydrogen atom, linear $C_1$-$C_6$ alkyl, branched $C_3$-$C_6$ alkyl, or $C_3$-$C_8$ cycloalkyl, and $\alpha$-carbon of the carboxy group of the second amino acid or peptide is represented by formula: $-CR^8R^9-$, wherein $R^8$ and $R^9$ are identical or different and each is a hydrogen atom, linear $C_1$-$C_4$ alkyl, optionally substituted branched $C_3$-$C_6$ alkyl, optionally substituted phenyl $C_1$-$C_2$ alkyl, or optionally substituted 5- to 6-membered heteroaryl $C_1$-$C_2$ alkyl.

[48] The linking agent according to [47], wherein the amino group of the first amino acid or peptide is an amino group represented by formula: $-NR^6R^7$, wherein $R^6$ is a hydrogen atom, and $R^7$ is a hydrogen atom or linear $C_1$-$C_4$ alkyl.

[49] The linking agent according to [47] or [48], wherein the amino group of the first amino acid or peptide is an amino group represented by formula: $-NR^6R^7$, wherein $R^6$ is a hydrogen atom, and $R^7$ is a hydrogen atom, methyl, or ethyl.

[50] The linking agent according to any of [47] to [49], wherein the amino group of the first amino acid or peptide is an amino group represented by formula: $-NR^6R^7$, wherein $R^6$ is a hydrogen atom, and $R^7$ is a hydrogen atom or methyl.

[51] The linking agent according to any of [47] to [50], wherein the amino group of the first amino acid or peptide is an amino group represented by formula: $-NR^6R^7$, wherein $R^6$ is a hydrogen atom, and $R^7$ is methyl.

[52] The linking agent according to any of [47] to [51], wherein $\alpha$-carbon of the carboxy group of the second amino acid or peptide is represented by formula: $-CR^8R^9-$, wherein $R^8$ and $R^9$ are identical or different and each is a hydrogen atom, linear $C_1$-$C_3$ alkyl, isopropyl, or optionally substituted benzyl.

[53] The linking agent according to any of [47] to [52], wherein $\alpha$-carbon of the carboxy group of the second amino acid or peptide is represented by formula: $-CR^8R^9-$, wherein $R^8$ is a hydrogen atom, and $R^9$ is a hydrogen atom, methyl, benzyl, or p-fluorobenzyl.

In the above numberings, the number cited in the dependent item includes the branch number of the number, unless otherwise mentioned. For example, the [1] cited in the dependent item shows that not only [1] but also its branch number [1-1] is included. The same applies to other numberings.

## Advantageous Effects of Invention

According to the present invention, a peptide compound can be synthesized with high diastereo-selectivity and high yield even by fragment coupling.

Description of Embodiments

The abbreviations used herein are listed below.

2-MeTHF: 2-methyltetrahydrofuran
IPAC: isopropyl acetate
EtOAc: ethyl acetate
MeCN: acetonitrile
THF: tetrahydrofuran
MeOH: methanol
MTBE: methyl tert-butyl ether
MTHP: 4-methyltetrahydropyran
CPME: cyclopentyl methyl ether
DCM: dichloromethane
DMF: N,N-dimethylformamide
DMA: N,N-dimethylacetamide
DMI: 1,3-dimethyl-2-imidazolidinone
NMP: N-methylpyrrolidone
DMSO: dimethylsulfoxide
DBN: 1,5-diazabicyclo[4.3.0]-5-nonene
DBU: 1,8-diazabicyclo[5.4.0]-7-undecene
DIPEA: N,N-diisopropylethylamine
TEA: triethylamine
NMM: N-methylmorpholine
NMI: N-methylimidazole
Cy2NMe: dicyclohexylmethylamine
DMAP: 4-N,N-dimethylaminopyridine
T3P: propylphosphonic acid anhydride
HATU: O-(7-azabenzotriazol-1-yl)-N,N,N',N'-tetramethyluronium hexafluorophosphate or 1-[bis(dimethylamino)methylene]-1H-1,2,3-triazolo[4,5-b]pyridinium 3-oxide hexafluorophosphate
COMU: (1-cyano-2-ethoxy-2-oxoethylideneaminooxy)dimethylaminomorpholinocarbenium hexafluorophosphate or ethyl 2-cyano-2-((dimethylimino)(morpholino)methyloximino)acetate hexafluorophosphate
DEPC: diethyl chlorophosphate
DPPC: diphenyl chlorophosphate
DPPA: diphenylphosphoryl azide
FDPP: pentafluorophenyl diphenyl phosphinate
$Ph_2P(O)Cl$: diphenylphosphinic chloride
HOPO: 1-hydroxypyridin-2-one
3-Cl-5-CF3-HOPO: 3-chloro-1-hydroxy-5-(trifluoromethyl)pyridin-2-one
5-F-HOPO: 5-fluoro-1-hydroxypyridin-2-one
5-Cl-HOPO: 5-chloro-1-hydroxypyridin-2-one
5-Br-HOPO: 5-bromo-1-hydroxypyridin-2-one
5-CF3-HOPO: 1-hydroxy-5-(trifluoromethyl)pyridin-2-one
5-CO2Me-HOPO: 1-hydroxy-5-(methoxycarbonyl)pyridin-2-one
4-Br-HOPO: 4-bromo-1-hydroxypyridin-2-one
$H_3PO_4$: phosphoric acid
NaCl: sodium chloride
$Na_2CO_3$: sodium carbonate
$K_2CO_3$: potassium carbonate
$NaHCO_3$: sodium hydrogen carbonate
$NaHSO_4$: sodium hydrogen sulfate
$Na_2SO_4$: sodium sulfate
$H_2O$: water
TFA: trifluoroacetic acid
HMDS: hexamethyldisilazane
TMSOTf: trimethylsilyl trifluoromethanesulfonate
Pd/C: palladium on carbon
HOAt: 1-hydroxy-7-azabenzotriazole (HOAt)

HOBt: 1-hydroxybenzotriazole
HOOBt: 3-hydroxy-1,2,3-benzotriazin-4-one
HOCt: ethyl 1-hydroxy-1H-1,2,3-triazole-4-carboxylate
HOSu: N-hydroxysuccinimide
HONB: N-hydroxy-5-norbornene-2,3-dicarboximide
HOPht: N-hydroxyphthalimide
EDC·HCl: 1-(3-dimethylaminopropyl)-3-ethylcarbodiimide hydrochloride
DIC: N,N'-diisopropylcarbodiimide
CDI: 1,1'-carbonyl diimidazole
MNBA: 2-methyl-6-nitrobenzoic anhydride
TCFH: chloro-N,N,N',N'-tetramethylformamidinium hexafluorophosphate
DEPBT: 3-(diethoxyphosphoryloxy)-1,2,3-benzotriazine-4(3H)-one
TDBTU: O-(3,4-dihydro-4-oxo-1,2,3-benzotriazin-3-yl)-N,N,N',N'-tetramethyluronium tetrafluoroborate
DPPA: diphenylphosphoryl azide
FDPP: pentafluorophenyl diphenyl phosphinate
p-TsCl: para-toluenesulfonyl chloride
HOTU: O-[(ethoxycarbonyl)cyanomethylenamino]-N,N,N',N'-tetramethyluronium hexafluorophosphate
Oxyma: ethyl 2-cyano-2-(hydroxyimino)acetate
Oxyma-B: 5-(hydroxyimino)-1,3-dimethylpyrimidine-2,4,6-(1H,3H,5H)-thrion
K-Oxyma: ethyl (hydroxyimino)cyanoacetate potassium salt
PfpOH: 2,2,3,3,3-pentafluoro-1-propanol
TMG: 1,1,3,3-tetramethylguanidine
MTBD: 7-methyl-1,5,7-triazabicyclo[4.4.0]dec-5-en
BTMB: 2-tert-butyl-1,1,3,3-tetramethylguanidine

Definitions of functional groups and the like (The terms explained below are illustrative and not intended to be particularly limiting, and are those that can be commonly understood by those skilled in the art.)

The "halogen atom" as used herein include fluorine, chlorine, bromine, and iodine. As used herein, F refers to fluorine, Cl refers to chlorine, Br refers to bromine, and I refers to iodine. Examples of the halogen include fluorine, chlorine and bromine, and preferably fluorine and chlorine.

The "alkyl" as used herein is a linear or branched monovalent saturated hydrocarbon group derived from an aliphatic saturated hydrocarbon by removing any one hydrogen atom, the group having a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear alkyl but also a branched alkyl. Specifically, the alkyl is an alkyl having 1 to 20 carbon atoms ($C_1$-$C_{20}$), preferably $C_1$-$C_{10}$ alkyl, more preferably $C_1$-$C_6$ alkyl. Hereinafter, "$C_p$-$C_q$" means that it has p to q carbon atoms. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl. Specific examples of the linear $C_1$-$C_6$ alkyl include methyl, ethyl, n-propyl, n-butyl, n-pentyl, and n-hexyl. Specific examples of the linear $C_1$-$C_3$ alkyl include methyl, ethyl, and n-propyl. Specific examples of the branched $C_3$-$C_6$ alkyl include i-propyl, s-butyl, t-butyl, isobutyl (2-methylpropyl), s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

The "alkenyl" as used herein is a linear or branched monovalent unsaturated hydrocarbon group having one or more carbon-carbon double bonds (bonds by two adjacent sp2 carbon atoms). Depending on the conformation of the atoms or groups of atoms attached to the sp2 carbon atoms, the geometric morphology of the double bond can assume entgegen (E) or zusammen (Z) and cis or trans conformations. The alkenyl is, for example, $C_2$-$C_{10}$ alkenyl, preferably $C_2$-$C_8$ alkenyl, more preferably $C_2$-$C_7$ alkenyl, most preferably $C_2$-$C_6$ alkenyl. Specific examples of the alkenyl include ethenyl (vinyl), 1-propenyl, 2-propenyl (allyl), isopropenyl, 1-butenyl, 2-butenyl (including cis, trans), 3-butenyl, pentenyl, and hexenyl.

The "alkynyl" as used herein is a linear or branched monovalent unsaturated hydrocarbon group having one or more carbon-carbon triple bonds (bonds by two adjacent sp carbon atoms). The alkynyl is, for example, $C_2$-$C_{10}$ alkynyl, preferably $C_2$-$C_8$ alkynyl, more preferably $C_2$-$C_7$ alkynyl, most preferably $C_2$-$C_6$ alkynyl. Specific examples of the alkynyl include ethynyl, 1-propynyl, propargyl (2-propynyl), 1-butynyl, 2-butynyl, 3-butynyl, pentynyl, and hexynyl.

The "cycloalkyl" as used herein is a saturated or partially saturated cyclic monovalent non-aromatic hydrocarbon

ring group (alicyclic ring group). A carbon atom constituting the ring may be oxidated to form carbonyl. The cycloalkyl may be selected from the group consisting of a monocyclic ring, a condensed ring, and a spiro ring. As used herein, the cycloalkyl containing a monocyclic ring is referred to as monocyclic cycloalkyl or a monocyclic alicyclic ring group; the cycloalkyl containing a condensed ring is referred to as condensed cyclic cycloalkyl or a condensed cyclic alicyclic ring group; and the cycloalkyl containing a spiro ring is referred to as spirocyclic cycloalkyl or a spirocyclic alicyclic ring group. The cycloalkyl may form a condensed ring with a saturated alicyclic ring such as a cyclopentane ring or a cyclohexane ring, an unsaturated alicyclic ring such as a cyclopentene ring or a cyclohexene ring, or an aromatic hydrocarbon ring such as a benzene ring or a naphthalene ring. The cycloalkyl may form a spiro ring with a saturated alicyclic ring such as a cyclopropane ring, a cyclobutane ring, a cyclopentane ring, or a cyclohexane ring. The cycloalkyl is, for example, $C_3$-$C_{10}$ cycloalkyl, preferably $C_3$-$C_8$ cycloalkyl, more preferably $C_3$-$C_7$ cycloalkyl, most preferably $C_3$-$C_6$ cycloalkyl. Specific examples of the cycloalkyl include cyclopropyl, cyclobutyl, cyclopentyl, cyclohexyl, cycloheptyl, cyclooctyl, bicyclo[2.2.1]heptyl, spiro[3.3]heptyl, and cyclohexenyl.

The "aryl" as used herein is a monovalent aromatic hydrocarbon ring group composed of a monovalent, monocyclic or condensed ring that exhibits aromaticity. As used herein, the aryl composed of a monocyclic ring is referred to as a monocyclic aryl, and an aryl composed of a condensed ring is referred to as a condensed cyclic aryl. The aryl is, for example, $C_6$-$C_{14}$ aryl, preferably $C_6$ aryl, $C_{10}$ aryl and $C_{14}$ aryl, more preferably $C_6$ aryl and $C_{10}$ aryl, most preferably $C_6$ aryl. Specific examples of the aryl include phenyl, 1-naphthyl, 2-naphthyl, tolyl, and xylyl.

The "heterocyclyl" as used herein is a heterocyclic group that contains, as an atom constituting a ring, a hetero atom selected from the group consisting of nitrogen atoms, oxygen atoms and sulfur atoms, in addition to the carbon atom, preferably 1 to 5 hetero atoms, more preferably 1 to 3 hetero atoms, and that may have a double and/or triple bond in the ring. A carbon atom in the ring of the heterocyclyl may be oxidated to form carbonyl. As used herein, the heterocyclyl containing a monocyclic ring is referred to as a monocyclic heterocyclyl; the heterocyclyl containing a condensed ring is referred to as a condensed cyclic heterocyclyl; and the heterocyclyl containing a spiro ring is referred to as a spirocyclic heterocyclyl. The heterocyclyl may form a condensed ring or a spiro ring with a saturated alicyclic ring such as a cyclopentane ring or a cyclohexane ring, or a saturated heterocycle such as a tetrahydropyran ring, a dioxane ring, or a pyrrolidine ring. The number of atoms constituting the ring of heterocyclyl is, for example, 3 to 14 (3- to 14-membered heterocyclyl), preferably 3 to 12 (3- to 12-membered heterocyclyl), more preferably 3 to 10 (3- to 10-membered heterocyclyl), most preferably 4 to 7 (4- to 7-membered heterocyclyl). Specific examples of the heterocyclyl include azetidinyl, oxiranyl, oxetanyl, thietanyl, tetrahydrofuranyl, pyrrolidinyl, pyrazolidinyl, imidazolidinyl, oxazolidinyl, isoxazolidinyl, thiazolidinyl, isothiazolidinyl, thiadiazolidinyl, oxazolidonyl, dioxolanyl, tetrahydropyranyl, morpholinyl, thiomorpholinyl, 4-oxopyrrolidinyl, piperidinyl, 4-oxopiperidinyl, piperazinyl, and dioxanyl, and rings in which one or more single bonds in these saturated heterocycles are replaced with a double bond or a triple bond.

The "heteroaryl" as used herein is a monovalent aromatic heterocyclic group that contains at least one heteroatom in addition to the carbon atom and is composed of a monocyclic or condensed ring that exhibits aromaticity. As used herein, a heteroaryl composed of a monocyclic ring is referred to as a monocyclic heteroaryl, and a heteroaryl composed of a condensed ring is referred to as a condensed cyclic heteroaryl. The number of atoms constituting the ring of heteroaryl is, for example, 5 to 14 (5- to 14-membered heteroaryl), preferably 5 to 13 (5- to 13-membered heteroaryl), more preferably 5 to 10 (5- to 10-membered heteroaryl), most preferably 5 to 7 (5- to 7-membered heteroaryl). Specific examples of the heteroaryl include 5-membered heteroaryl such as furyl, thienyl, pyrrolyl, imidazolyl, pyrazolyl, thiazolyl, oxazolyl, triazolyl, or tetrazolyl; 6-membered heteroaryl such as pyridyl, pyrimidyl, pyridazinyl, pyrazinyl, or triazinyl; 9-membered heteroaryl such as benzofuranyl, benzothienyl, benzothiazolyl, benzimidazolyl, benzotriazolyl, indolyl, indazolyl, or pyrazolopyridyl; and 10-membered heteroaryl such as quinolyl, isoquinolyl, cinnolinyl, quinazolinyl, or quinoxalinyl.

The "alkoxy" as used herein is a group (-OR, wherein R is alkyl) in which "alkyl" as defined herein is attached to an oxygen atom. The alkoxy is, for example, $C_1$-$C_{20}$ alkoxy, preferably $C_1$-$C_{10}$ alkoxy, more preferably $C_1$-$C_8$ alkoxy, most preferably $C_1$-$C_6$ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The "alkenyloxy" as used herein is a group (-OR, wherein R is alkenyl) in which "alkenyl" as defined herein is attached to an oxygen atom. The alkenyloxy is, for example, $C_2$-$C_{10}$ alkenyloxy, preferably $C_2$-$C_8$ alkenyloxy, more preferably $C_2$-$C_7$ alkenyloxy, most preferably $C_2$-$C_6$ alkenyloxy. Specific examples of the alkenyloxy include ethenyl (vinyl) oxy, 1-propenyloxy, 2-propenyl (allyl) oxy, isopropenyloxy, 1-butenyloxy, 2-cis-butenyloxy, 2-trans-butenyloxy, 3-butenyloxy, pentenyloxy, and hexenyloxy.

The "cycloalkoxy" as used herein is a group (-OR, wherein R is cycloalkyl) in which "cycloalkyl" as defined herein is attached to an oxygen atom. The cycloalkoxy is, for example, $C_3$-$C_{10}$ cycloalkoxy, preferably $C_3$-$C_8$ cycloalkoxy, more preferably $C_3$-$C_7$ cycloalkoxy, most preferably $C_3$-$C_6$ cycloalkoxy. Specific examples of the cycloalkoxy include cyclopropoxy, cyclobutoxy, and cyclopentyloxy.

The "aryloxy" as used herein is a group (-OAr, wherein Ar is aryl) in which "aryl" as defined herein is attached to an oxygen atom. The aryloxy is, for example, $C_6$-$C_{14}$ aryloxy, preferably $C_6$ aryloxy, $C_{10}$ aryloxy and $C_{14}$ aryloxy, more preferably $C_6$ aryloxy and $C_{10}$ aryloxy, most preferably $C_6$ aryloxy. Specific examples of the aryloxy include phenoxy, 1-naphthyloxy, 2-naphthyloxy, tolyloxy, and xylyloxy.

The "amino" as used herein means -NRR' wherein N represents a nitrogen atom and R and R' are each independently selected from the group consisting of a hydrogen atom, alkyl, alkenyl, alkynyl, cycloalkyl, heterocyclyl, aryl, and heteroaryl, or R and R' form a ring together with the nitrogen atom to which they are attached. Examples of the amino include $-NH_2$, mono-$C_1$-$C_6$ alkylamino, di-$C_1$-$C_6$ alkylamino, and 4- to 8-membered cyclic amino.

The "monoalkylamino" as used herein means a group of the "amino (-NRR')" as defined herein, in which R is a hydrogen atom and R' is the "alkyl". The monoalkylamino is, for example, mono-$C_1$-$C_{20}$ alkylamino, preferably mono-$C_1$-$C_{15}$ alkylamino, more preferably mono-$C_1$-$C_{10}$ alkylamino, most preferably mono-$C_1$-$C_6$ alkylamino. Specific examples of the monoalkylamino include methylamino, ethylamino, n-propylamino, i-propylamino, n-butylamino, s-butylamino, and t-butylamino.

The "dialkylamino" as used herein means a group of the "amino (-NRR')" as defined herein, in which R and R' are each independently the "alkyl". The dialkylamino is, for example, di-$C_1$-$C_{20}$ alkylamino, preferably di-$C_1$-$C_{15}$ alkylamino, more preferably di-$C_1$-$C_{10}$ alkylamino, most preferably di-$C_1$-$C_6$ alkylamino. Specific examples of the dialkylamino include dimethylamino, diethylamino, and methyl ethylamino.

The "cyclic amino" as used herein is a group of the "amino (-NRR')" as defined herein, in which R and R' form a ring together with the nitrogen atom to which they are attached. The cyclic amino is, for example, a 3- to 14-membered cyclic amino, preferably a 3- to 12-membered cyclic amino, more preferably a 3- to 10-membered cyclic amino, most preferably a 4- to 7-membered cyclic amino. Specific examples of the cyclic amino include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

The "protected amino" as used herein means an amino group protected with an optional protecting group. Specific examples of the protected amino include an amino protected by a protecting group such as Boc (tert-butoxycarbonyl), Fmoc (9-fluorenylmethyloxycarbonyl), Cbz (benzyloxycarbonyl), Troc (2,2,2-trichloroethoxycarbonyl), Alloc (allyloxycarbonyl), Teoc (2-(trimethylsilyl)ethoxycarbonyl), or trifluoroacetyl.

The "aminocarbonyl" as used herein is a group in which an "amino" as defined herein is attached to a carbon atom of a carbonyl. It is also sometimes referred to as amide. Examples of the aminocarbonyl include $-CONH_2$, mono-$C_1$-$C_6$ alkylaminocarbonyl, di-$C_1$-$C_6$ alkylaminocarbonyl, and 4- to 8-membered cyclic aminocarbonyl. Specific examples of the aminocarbonyl include $-CONH_2$, methylaminocarbonyl, ethylaminocarbonyl, dimethylaminocarbonyl, diethylaminocarbonyl, 1-azetidinylcarbonyl, 1-pyrrolidinylcarbonyl, 1-piperidinylcarbonyl, 1-piperazinylcarbonyl, 4-morpholinylcarbonyl, and 3-oxazolidinylcarbonyl.

The "alkenyloxycarbonyl" as used herein is a carbonyl group to which "alkenyloxy" as defined herein is attached. The alkenyloxycarbonyl is, for example, $C_2$-$C_{10}$ alkenyloxycarbonyl, preferably $C_2$-$C_8$ alkenyloxycarbonyl, more preferably $C_2$-$C_7$ alkenyloxycarbonyl, most preferably $C_2$-$C_6$ alkenyloxycarbonyl. Specific examples of the alkenyloxycarbonyl include ethenyl (vinyl) oxycarbonyl, 1-propenyloxycarbonyl, 2-propenyl (allyl) oxycarbonyl, isopropenyloxycarbonyl, 1-butenyloxycarbonyl, 2-cis-butenyloxycarbonyl, 2-trans-butenyloxycarbonyl, 3-butenyloxycarbonyl, pentenyloxycarbonyl, and hexenyloxycarbonyl.

The "alkylsulfonyl" as used herein is a sulfonyl group to which "alkyl" as defined herein is attached. The alkylsulfonyl is, for example, $C_1$-$C_{20}$ alkylsulfonyl, preferably $C_1$-$C_{10}$ alkylsulfonyl, more preferably $C_1$-$C_8$ alkylsulfonyl, most preferably $C_1$-$C_6$ alkylsulfonyl. Specific examples of the alkylsulfinyl include methylsulfonyl, ethylsulfonyl, 1-propylsulfonyl, 2-propylsulfonyl, n-butylsulfonyl, i-butylsulfonyl, s-butylsulfonyl, t-butylsulfonyl, pentylsulfonyl, and 3-methylbutylsulfonyl.

The "hydroxyalkyl" as used herein is a group in which one or more hydrogen atoms of the "alkyl" as defined herein are substituted with a hydroxy group. The hydroxyalkyl is preferably a group in which one hydrogen atom of the alkyl is substituted with a hydroxy group. The hydroxyalkyl is, for example, hydroxy $C_1$-$C_{20}$ alkyl, preferably hydroxy $C_1$-$C_{10}$ alkyl, more preferably hydroxy $C_1$-$C_8$ alkyl, most preferably hydroxy $C_1$-$C_6$ alkyl. Specific examples of the hydroxyalkyl include hydroxyethyl, 1-hydroxyethyl, 2-hydroxyethyl, 2-hydroxy-2-methylpropyl, and 5-hydroxypentyl.

The "haloalkyl" as used herein is a group in which one or more hydrogen atoms of the "alkyl" as defined herein are substituted with halogen. The haloalkyl is preferably a group in which one or more and six or less hydrogen atoms that are allowed to be substituted of the hydrogen atoms of the alkyl are substituted with halogen. The haloalkyl is, for example, halo-$C_1$-$C_{20}$ alkyl, preferably halo-$C_1$-$C_{10}$ alkyl, more preferably halo-$C_1$-$C_8$ alkyl, most preferably halo-$C_1$-$C_6$ alkyl. The halo-$C_1$-$C_6$ alkyl is, for example, a group in which one or more and six or less hydrogen atoms allowed to be substituted of the hydrogen atoms of the alkyl are substituted with fluorine, preferably a group in which one or more and five or less hydrogen atoms are substituted with fluorine, more preferably a group in which one or more and four or less hydrogen atoms are substituted with fluorine, most preferably a group in which one or more and three or less hydrogen atoms are substituted with fluorine. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

The "cyanoalkyl" as used herein is a group in which one or more hydrogen atoms of the "alkyl" as defined herein are substituted with cyano. The cyanoalkyl is preferably a group in which one hydrogen atom of the alkyl is substituted with

cyano. The cyanoalkyl is, for example, cyano $C_1$-$C_{20}$ alkyl, preferably cyano $C_1$-$C_{10}$ alkyl, more preferably cyano $C_1$-$C_8$ alkyl, most preferably cyano $C_1$-$C_6$ alkyl. Specific examples of the cyanoalkyl include cyanomethyl and 2-cyanoethyl.

The "aminoalkyl" as used herein is a group in which one or more hydrogen atoms of the "alkyl" as defined herein are substituted with amino. The aminoalkyl is preferably a group in which one hydrogen atom of the alkyl is substituted with amino. The aminoalkyl is, for example, amino $C_1$-$C_{20}$ alkyl, preferably amino $C_1$-$C_{10}$ alkyl, more preferably amino $C_1$-$C_8$ alkyl, most preferably amino $C_1$-$C_6$ alkyl. Specific examples of the aminoalkyl include aminomethyl, aminoethyl, 4-aminobutyl, methylaminomethyl, dimethylaminomethyl, methylaminoethyl, and dimethylaminoethyl.

The "carboxyalkyl" as used herein is a group in which one or more hydrogen atoms of the "alkyl" as defined herein are substituted with "carboxy". The carboxyalkyl is preferably a group in which one hydrogen atom of the alkyl is substituted with carboxy. The carboxyalkyl is, for example, carboxy $C_1$-$C_{20}$ alkyl, preferably carboxy $C_1$-$C_{15}$ alkyl, more preferably carboxy $C_1$-$C_{10}$ alkyl, most preferably carboxy $C_1$-$C_6$ alkyl. Specific examples of the carboxyalkyl include carboxymethyl and carboxyethyl.

The "alkenyloxycarbonylalkyl" as used herein is a group in which one or more hydrogen atoms of the "alkyl" as defined herein are substituted with "alkenyloxycarbonyl". The alkenyloxycarbonylalkyl is preferably a group in which one hydrogen atom of the alkyl is substituted with alkenyloxycarbonyl. The alkenyloxycarbonylalkyl is, for example, $C_2$-$C_{10}$ alkenyloxycarbonyl-$C_1$-$C_6$ alkyl, preferably $C_2$-$C_8$ alkenyloxycarbonyl-$C_1$-$C_6$ alkyl, more preferably $C_2$-$C_7$ alkenyloxycarbonyl-$C_1$-$C_6$ alkyl, most preferably $C_2$-$C_6$ alkenyloxycarbonyl-$C_1$-$C_6$ alkyl. Specific examples of the alkenyloxycarbonylalkyl include ethenyl (vinyl) oxycarbonylmethyl, 1-propenyloxycarbonylethyl, 2-propenyl (allyl) oxycarbonylmethyl, isopropenyloxycarbonylmethyl, 1-butenyloxycarbonylethyl, 2-cis-butenyloxycarbonylmethyl (including cis, trans), 2-trans-butenyloxycarbonylmethyl, 3-butenyloxycarbonylethyl, pentenyloxycarbonylmethyl, and hexenyloxycarbonylethyl.

The "alkoxyalkyl" as used herein is a group in which one or more hydrogen atoms of the "alkyl" as defined herein are substituted with "alkoxy". The alkoxyalkyl is preferably a group in which one hydrogen atom of the alkyl is substituted with alkoxy. The alkoxyalkyl is, for example, $C_1$-$C_6$ alkoxy-$C_1$-$C_{20}$ alkyl, preferably $C_1$-$C_6$ alkoxy-$C_1$-$C_{15}$ alkyl, more preferably $C_1$-$C_6$ alkoxy-$C_1$-$C_{10}$ alkyl, most preferably $C_1$-$C_6$ alkoxy-$C_1$-$C_6$ alkyl. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

The "cycloalkylalkyl" as used herein is a group in which one or more hydrogen atoms of the "alkyl" as defined herein are substituted with "cycloalkyl". The cycloalkylalkyl is preferably a group in which one hydrogen atom of the alkyl is substituted with cycloalkyl. The cycloalkylalkyl is, for example, $C_3$-$C_{10}$ cycloalkyl-$C_1$-$C_{20}$ alkyl, preferably $C_3$-$C_{10}$ cycloalkyl-$C_1$-$C_6$ alkyl, more preferably $C_3$-$C_8$ cycloalkyl-$C_1$-$C_6$ alkyl, most preferably $C_3$-$C_6$ cycloalkyl-$C_1$-$C_2$ alkyl. Specific examples of the cycloalkylalkyl include cyclopropylmethyl, cyclobutylmethyl, cyclopentylmethyl, cyclohexylmethyl, cyclohexylethyl, and cyclohexylpropyl.

The "cycloalkoxyalkyl" as used herein is a group in which one or more hydrogen atoms of the "alkyl" as defined herein are substituted with "cycloalkoxy". The cycloalkoxyalkyl is preferably a group in which one hydrogen atom of the alkyl is substituted with cycloalkoxy. The cycloalkoxyalkyl is, for example, $C_3$-$C_{10}$ cycloalkoxy-$C_1$-$C_6$ alkyl, preferably $C_3$-$C_8$ cycloalkoxy-$C_1$-$C_6$ alkyl, more preferably $C_3$-$C_7$ cycloalkoxy-$C_1$-$C_6$ alkyl, most preferably $C_3$-$C_6$ cycloalkoxy-$C_1$-$C_6$ alkyl. Specific examples of the cycloalkoxyalkyl include cyclopropoxymethyl, cyclobutoxymethyl, and cyclopentyloxymethyl.

The "heterocyclylalkyl" as used herein is a group in which one or more hydrogen atoms of the "alkyl" as defined herein are substituted with "heterocyclyl". The heterocyclylalkyl is preferably a group in which one hydrogen atom of the alkyl is substituted with heterocyclyl. The heterocyclylalkyl is, for example, 3- to 14-membered heterocyclyl-$C_1$-$C_6$ alkyl, preferably 3-to 12-membered heterocyclyl-$C_1$-$C_6$ alkyl, more preferably 3- to 10-membered heterocyclyl-$C_1$-$C_4$ alkyl, most preferably 4- to 7-membered heterocyclyl-$C_1$-$C_3$ alkyl. Specific examples of the heterocyclylalkyl include azetidin-1-ylmethyl, oxetan-3-ylmethyl, 2-(tetrahydrofuran-3-yl)ethyl, (1-methylpyrrolidin-3-yl)methyl, 2-morpholinoethyl, 3-(1-piperidinyl)propyl, and 3-(4-methylpiperazin-1-yl)propyl.

The "alkylsulfonylalkyl" as used herein is a group in which one or more hydrogen atoms of the "alkyl" as defined herein are substituted with "alkylsulfonyl". The alkylsulfonylalkyl is preferably a group in which one hydrogen atom of the alkyl is substituted with alkylsulfonyl. The alkylsulfonylalkyl is, for example, $C_1$-$C_{20}$ alkylsulfonyl-$C_1$-$C_4$ alkyl, preferably $C_1$-$C_{10}$ alkylsulfonyl-$C_1$-$C_4$ alkyl, more preferably $C_1$-$C_8$ alkylsulfonyl-$C_1$-$C_4$ alkyl, most preferably $C_1$-$C_6$ alkylsulfonyl-$C_1$-$C_4$ alkyl. Specific examples of the alkylsulfonylalkyl include methylsulfonylmethyl, methylsulfonylpropyl, methylsulfonylbutyl, ethylsulfonylethyl, 1-propylsulfonylmethyl, 2-propylsulfonylethyl, n-butylsulfonylpropyl, i-butylsulfonylmethyl, s-butylsulfonylmethyl, t-butylsulfonylmethyl, pentylsulfonylmethyl, and 3-methylbutylsulfonylmethyl.

The "aminocarbonylalkyl" as used herein means a group in which one or more hydrogen atoms of the "alkyl" as defined herein are substituted with the "aminocarbonyl" as defined herein. The aminocarbonylalkyl is preferably a group in which one hydrogen atom of the alkyl is substituted with aminocarbonyl. The aminocarbonylalkyl is, for example, aminocarbonyl-$C_1$-$C_{10}$ alkyl, preferably aminocarbonyl-$C_1$-$C_6$ alkyl, more preferably aminocarbonyl-$C_1$-$C_4$ alkyl, most preferably aminocarbonyl-$C_1$-$C_2$ alkyl. Specific examples of the aminocarbonyl alkyl include $-CH_2CONH_2$, methylaminocarbonylimethyl, ethylaminocarbonylethyl, dimethylaminocarbonylpropyl, diethylaminocarbonylmethyl, 1-azetidinylcarbonylmethyl, 1-pyrrolidinylcarbonylethyl, 1-piperidinylcarbonylpropyl, 1-piperazinylcarbonylbutyl, 4-morpholinylcarbonylmethyl, and 3-oxazolidinylcarbonylethyl.

The "aryloxyalkyl" as used herein means a group in which one or more hydrogen atoms of the "alkyl" as defined herein are substituted with the "aryloxy" as defined herein. The aryloxyalkyl is preferably $C_6$-$C_{10}$ aryloxy-$C_1$-$C_6$ alkyl, more preferably $C_6$-$C_{10}$ aryloxy-$C_1$-$C_2$ alkyl. Specific examples of the aryloxyalkyl include phenoxymethyl and 2-phenoxyethyl.

The "aralkyl (arylalkyl)" as used herein is a group in which one or more hydrogen atoms of the "alkyl" as defined herein are substituted with the "aryl" as defined herein. The aralkyl is, for example, $C_7$-$C_{20}$ aralkyl, preferably $C_7$-$C_{18}$ aralkyl, more preferably $C_7$-$C_{16}$ aralkyl, most preferably $C_7$-$C_{14}$ aralkyl. The $C_7$-$C_{20}$ aralkyl is, for example, $C_6$-$C_{10}$ aryl-$C_1$-$C_{10}$ alkyl, preferably $C_6$-$C_{10}$ aryl-$C_1$-$C_8$ alkyl, more preferably $C_6$ aryl-$C_1$-$C_8$ alkyl or $C_{10}$ aryl-$C_1$-$C_8$ alkyl, most preferably $C_6$ aryl-$C_1$-$C_8$ alkyl. The $C_7$-$C_{18}$ aralkyl is, for example, $C_6$-$C_{10}$ aryl-$C_1$-$C_8$ alkyl, preferably $C_6$-$C_{10}$ aryl-$C_1$-$C_6$ alkyl, more preferably $C_6$ aryl-$C_1$-$C_6$ alkyl or $C_{10}$ aryl-$C_1$-$C_6$ alkyl, most preferably $C_6$ aryl-$C_1$-$C_6$ alkyl. The $C_7$-$C_{16}$ aralkyl is, for example, $C_6$-$C_{10}$ aryl-$C_1$-$C_6$ alkyl, preferably $C_6$-$C_{10}$ aryl-$C_1$-$C_4$ alkyl, more preferably $C_6$ aryl-$C_1$-$C_4$ alkyl or $C_{10}$ aryl-$C_1$-$C_4$ alkyl, most preferably $C_6$ aryl-$C_1$-$C_4$ alkyl. The $C_7$-$C_{14}$ aralkyl is, for example, $C_6$-$C_{10}$ aryl-$C_1$-$C_4$ alkyl, preferably $C_6$-$C_{10}$ aryl-$C_1$-$C_3$ alkyl, more preferably $C_6$ aryl-$C_1$-$C_3$ alkyl or $C_{10}$ aryl-$C_1$-$C_3$ alkyl, most preferably $C_6$ aryl-$C_1$-$C_3$ alkyl. Specific examples of the aralkyl include benzyl, phenethyl, and 3-phenylpropyl.

The "aralkoxy" as used herein is a group (-O-R-Ar, wherein R is alkylene) in which an alkyl moiety in "aralkyl" as defined herein is attached to an oxygen atom. The aralkoxy is, for example, $C_7$-$C_{20}$ aralkoxy, preferably $C_7$-$C_{18}$ aralkoxy, more preferably $C_7$-$C_{16}$ aralkoxy, most preferably $C_7$-$C_{14}$ aralkoxy. The $C_7$-$C_{20}$ aralkoxy is, for example, $C_6$-$C_{10}$ aryl-$C_1$-$C_{10}$ alkoxy, preferably $C_6$-$C_{10}$ aryl-$C_1$-$C_8$ alkoxy, more preferably $C_6$ aryl-$C_1$-$C_8$ alkoxy or $C_{10}$ aryl-$C_1$-$C_8$ alkoxy, most preferably $C_6$ aryl-$C_1$-$C_8$ alkoxy. The $C_7$-$C_{18}$ aralkoxy is, for example, $C_6$-$C_{10}$ aryl-$C_1$-$C_8$ alkoxy, preferably $C_6$-$C_{10}$ aryl-$C_1$-$C_6$ alkoxy, more preferably $C_6$ aryl-$C_1$-$C_6$ alkoxy or $C_{10}$ aryl-$C_1$-$C_6$ alkoxy, most preferably $C_6$ aryl-$C_1$-$C_6$ alkoxy. The $C_7$-$C_{16}$ aralkoxy is, for example, $C_6$-$C_{10}$ aryl-$C_1$-$C_6$ alkoxy, preferably $C_6$-$C_{10}$ aryl-$C_1$-$C_4$ alkoxy, more preferably $C_6$ aryl-$C_1$-$C_4$ alkoxy or $C_{10}$ aryl-$C_1$-$C_4$ alkoxy, most preferably $C_6$ aryl-$C_1$-$C_4$ alkoxy. The $C_7$-$C_{14}$ aralkoxy is, for example, $C_6$-$C_{10}$ aryl-$C_1$-$C_4$ alkoxy, preferably $C_6$-$C_{10}$ aryl-$C_1$-$C_3$ alkoxy, more preferably $C_6$ aryl-$C_1$-$C_3$ alkoxy or $C_{10}$ aryl-$C_1$-$C_3$ alkoxy, most preferably $C_6$ aryl-$C_1$-$C_8$ alkoxy. Specific examples of the aralkoxy include benzyloxy, phenethyloxy, and 3-phenylpropoxy.

The "aralkoxyalkyl" as used herein means a group in which one or more hydrogen atoms of the "alkyl" as defined herein are substituted with the "aralkoxy" as defined herein. The aralkoxyalkyl is preferably $C_7$-$C_{14}$ aralkoxy-$C_1$-$C_6$ alkyl, more preferably $C_7$-$C_{14}$ aralkoxy-$C_1$-$C_2$ alkyl. Specific examples of the aralkoxyalkyl include benzyloxymethyl and 1-(benzyloxy)ethyl.

The "heteroarylalkyl" as used herein is a group in which one or more hydrogen atoms of the "alkyl" as defined herein are substituted with "heteroaryl". The heteroarylalkyl is preferably a group in which one hydrogen atom of the alkyl is substituted with heteroaryl. The heteroarylalkyl is, for example, 5- to 10-membered heteroaryl-$C_1$-$C_6$ alkyl, preferably 5- to 10-membered heteroaryl-$C_1$-$C_4$ alkyl, more preferably 5- to 10-membered heteroaryl-$C_1$-$C_3$ alkyl, most preferably 5- to 10-membered heteroaryl-$C_1$-$C_2$ alkyl. Specific examples of the heteroarylalkyl include 2-pyridylmethyl, 3-pyridylmethyl, 4-pyridylmethyl, 2-furanylmethyl, 2-thienylmethyl, 3-thienylmethyl, and 4-thiazolylmethyl.

The "heteroarylalkoxy" as used herein is a group in which an alkyl moiety in the "heteroarylalkyl" as defined herein is attached to an oxygen atom. The heteroarylalkoxy is, for example, 5- to 10-membered heteroaryl-$C_1$-$C_6$ alkoxy, preferably 5- to 10-membered heteroaryl-$C_1$-$C_4$ alkoxy, more preferably 5- to 10-membered heteroaryl-$C_1$-$C_3$ alkoxy, most preferably 5-to 10-membered heteroaryl-$C_1$-$C_2$ alkoxy. Specific examples of the heteroarylalkoxy include 2-pyridylmethoxy, 3-pyridylmethoxy, 4-pyridylmethoxy, 2-furanylmethoxy, 2-thienylmethoxy, 3-thienylmethoxy, and 4-thiazolylmethoxy.

The "heteroarylalkoxyalkyl" as used herein means a group in which one or more hydrogen atoms of the "alkyl" as defined herein are substituted with the "heteroarylalkoxy" as defined herein. The heteroarylalkoxyalkyl is preferably a group in which one hydrogen atom of the alkyl is substituted with heteroarylalkoxy. The heteroarylalkoxyalkyl is, for example, 5- to 10-membered heteroaryl-$C_1$-$C_6$ alkoxy-$C_1$-$C_6$ alkyl, preferably 5- to 10-membered heteroaryl-$C_1$-$C_4$ alkoxy-$C_1$-$C_6$ alkyl, more preferably 5- to 10-membered heteroaryl-$C_1$-$C_3$ alkoxy-$C_1$-$C_6$ alkyl, most preferably 5- to 10-membered heteroaryl-$C_1$-$C_2$ alkoxy-$C_1$-$C_6$ alkyl. Specific examples of the heteroarylalkoxyalkyl include 2-pyridylmethoxymethyl, 3-pyridylmethoxymethyl, 4-pyridylmethoxymethyl, 2-furanylmethoxymethyl, 2-thienylmethoxymethyl, 3-thienylmethoxymethyl, and 4-thiazolylmethoxymethyl.

The "heterocycloalkylidenealkyl" as used herein means a group in which one or more hydrogen atoms of the "alkyl" as defined herein are substituted with the "heterocycloalkylidene" as defined herein. The heterocycloalkylidenealkyl is preferably 4- to 7-membered heterocycloalkyliden-$C_1$-$C_6$ alkyl, more preferably 4- to 7-membered heterocycloalkyliden-$C_1$-$C_2$ alkyl. Specific examples of the heteroarylalkoxyalkyl include tetrahydro-4H-pyran-4-ylidenemethyl and azetidin-3-ylidenemethyl.

The "alkoxyalkenyl" as used herein is a group in which one or more hydrogen atoms of the "alkenyl" as defined herein are substituted with "alkoxy". The alkoxyalkenyl is preferably a group in which one hydrogen atom of the alkenyl is substituted with alkoxy. The alkoxyalkenyl is, for example, $C_1$-$C_6$ alkoxy-$C_2$-$C_{10}$ alkenyl, preferably $C_1$-$C_6$ alkoxy-$C_2$-$C_8$

alkenyl, more preferably $C_1$-$C_6$ alkoxy-$C_2$-$C_7$ alkenyl, most preferably $C_1$-$C_6$ alkoxy-$C_2$-$C_6$ alkenyl. Specific examples of the alkoxyalkenyl include (E)-4-methoxybut-2-en-1-yl.

The "aminocarbonylalkenyl" as used herein is a group in which one or more hydrogen atoms of the "alkenyl" as defined herein are substituted with "aminocarbonyl". The aminocarbonylalkenyl is preferably a group in which one hydrogen atom of the alkenyl is substituted with aminocarbonyl. The aminocarbonylalkenyl is, for example, aminocarbonyl-$C_2$-$C_{10}$ alkenyl, preferably aminocarbonyl-$C_2$-$C_8$ alkenyl, more preferably aminocarbonyl-$C_2$-$C_6$ alkenyl, most preferably aminocarbonyl-$C_2$-$C_4$ alkenyl. Specific examples of the aminocarbonylalkenyl include (E)-3-(dimethylaminocarbonyl)-prop-2-en-1-yl.

The "haloalkoxy" as used herein is a group in which one or more hydrogen atoms of the "alkoxy" as defined herein are substituted with halogen. The haloalkoxy is preferably a group in which one or more and six or less hydrogen atoms allowed to be substituted of the hydrogen atoms of the alkoxy are substituted with halogen. The haloalkoxy is, for example, halo-$C_1$-$C_{20}$ alkoxy, preferably halo-$C_1$-$C_{10}$ alkoxy, more preferably halo-$C_1$-$C_8$ alkoxy, most preferably halo-$C_1$-$C_6$ alkoxy. The halo-$C_1$-$C_6$ alkoxy is, for example, a group in which one or more and six or less hydrogen atoms allowed to be substituted of the hydrogen atoms of the alkoxy are substituted with fluorine, preferably a group in which one or more and five or less hydrogen atoms are substituted with fluorine, more preferably a group in which one or more and four or less hydrogen atoms are substituted with fluorine, most preferably a group in which one or more and three or less hydrogen atoms are substituted with fluorine. Specific examples of the haloalkoxy include difluoromethoxy, trifluoromethoxy, 2,2-difluoroethoxy, 2,2,2-trifluoroethoxy, 3-fluoropropoxy, and 2,2-difluoropropoxy.

The "alkylene" as used herein means a divalent group induced by the further removal of any one hydrogen atom from the "alkyl" described herein. The alkylene is preferably $C_1$-$C_8$ alkylene, more preferably $C_4$-$C_8$ alkylene. Examples of the alkylene specifically include $-CH_2-$, $-(CH_2)_2-$, $-(CH_2)_3-$, $-CH(CH_3)CH_2-$, $-C(CH_3)_2-$, $-(CH_2)_4-$, $-CH(CH_3)CH_2CH_2-$, $-C(CH_3)_2CH_2-$, $-CH_2CH(CH_3)CH_2-$, $-CH_2C(CH_3)_2-$, $-CH_2CH_2CH(CH_3)-$, $-CH_2CH(CH_2CH_3)-$, $-(CH_2)_5-$, $-CH(CH_3)CH(CH_2CH_3)-$, $-(CH_2)_6-$, $-(CH_2)_7-$, and $-(CH_2)_8-$.

The "alicyclic ring" herein is a saturated or partially saturated non-aromatic hydrocarbon ring. A carbon atom constituting the ring may be oxidated to form carbonyl. The alicyclic ring may be selected from the group consisting of a monocyclic ring, a condensed ring, and a spiro ring. As used herein, an alicyclic ring containing a monocyclic ring is referred to as a monocyclic alicyclic ring; an alicyclic ring containing a condensed ring is referred to as a condensed cyclic alicyclic ring; and an alicyclic ring containing a spiro ring is referred to as a spirocyclic alicyclic ring. The alicyclic ring may form a condensed ring with a saturated alicyclic ring such as a cyclopentane ring or a cyclohexane ring; an unsaturated alicyclic ring such as a cycloheptene ring or a cyclohexene ring; or an aromatic hydrocarbon ring such as a benzene ring or a naphthalene ring. The alicyclic ring may form a spiro ring with a saturated alicyclic ring such as a cyclopropane ring, a cyclobutane ring, a cyclopentane ring, or a cyclohexane ring. The alicyclic ring is, for example, a $C_3$-$C_{10}$ alicyclic ring, preferably a $C_3$-$C_8$ alicyclic ring, more preferably a $C_3$-$C_7$ alicyclic ring, most preferably a $C_3$-$C_6$ alicyclic ring. Specific examples of the alicyclic ring include a cyclopropane ring, a cyclobutane ring, a cyclopentane ring, a cyclohexane ring, a cycloheptane ring, a cyclooctane ring, a bicyclo[2.2.1]heptane ring, and a cyclohexene ring.

The "saturated heterocycle" as used herein is a ring of the "heterocycle" that does not have an unsaturated bond in the ring. The number of atoms constituting the saturated heterocycle is from 3 to 14 (3- to 14-membered heterocycles), preferably from 3 to 12 (3- to 12-membered heterocycles), more preferably from 3 to 10 (3- to 10-membered heterocycles), most preferably from 4 to 7 (4- to 7-membered heterocycles). Specific examples of the saturated heterocycle include an azetidine ring, an epoxy ring, an oxetane ring, a thietane ring, a tetrahydrofuran ring, a pyrrolidine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolan ring, a tetrahydropyran ring, a morpholine ring, a thiomorpholine ring, a 4-oxopyrrolidine rings, a piperidine ring, a 4-oxopiperidine ring, a piperazine ring, and a dioxane ring.

The "peptide compound" as used herein means a compound wherein two or more amino acids are linked by an amide bond. As long as two or more amino acids are linked by an amide bond, the peptide chain may contain another bond such as an ester bond or a thioester bond. Examples of the number of residues of the amino acid contained in the peptide include 5 to 30 residues. The peptide may be linear, branched, or cyclic.

The "peptide chain" as used herein means a chain-like portion of a peptide in which two or more amino acids are linked by an amide bond. As long as two or more amino acids are linked by an amide bond, the peptide chain may contain another bond such as an ester bond or a thioester bond. Examples of the number of residues of the amino acid contained in the peptide chain include 5 to 30 residues.

The "optionally substituted" as used herein means that a certain group and/or certain atom may be substituted with an optional substituent and/or optional atom. That is, either of the state in which the certain group and the certain atom are neither substituted by an optional substituent nor an optional atom, or the state in which the certain group and/or certain atom are substituted by an optional substituent and/or optional atom can be selected. Each of the certain group and/or certain atom may be further substituted with an optional substituent and/or optional atom. That is, either of the state in which the certain group and the certain atom have no substitutions of an optional substituent or an optional atom, or the state in which the certain group and/or certain atom have a substitution of an optional substituent and/or optional atom can

be selected. The certain group and the optional substituent are not limited and may be, for example, freely selected from groups containing atoms selected from the group consisting of a hydrogen atom, a halogen atom, a carbon atom, an oxygen atom, a sulfur atom, a nitrogen atom, a boron atom, a silicon atom, and a phosphorus atom. The certain atom and the optional atom are not limited and may be, for example, freely selected from a halogen atom, a carbon atom, an oxygen atom, a sulfur atom, a nitrogen atom, a boron atom, a silicon atom, and a phosphorus atom. Examples of the optional substituent include alkyl, alkoxy, fluoroalkyl, fluoroalkoxy, oxo, aminocarbonyl, alkylsulfonyl, alkylsulfonylamino, cycloalkyl, aryl, heteroaryl, heterocyclyl, arylalkyl, heteroarylalkyl, halogen, nitro, amino, monoalkylamino, dialkylamino, cyano, carboxyl, alkoxycarbonyl, and formyl.

The "optionally protected" as used herein means that a group may be protected with an optional protecting group.

The "one or more" as used herein means the number of 1 or 2 or more. When the "one or more" is used in the context associated with a substituent of a group, the term means a number from one to the maximum number of substituents allowed by the group. Specifically, the "one or more" includes, for example, 1, 2, 3, 4, 5, 6, 7, 8, 9, 10, and/or a greater number.

The compound described herein or a salt thereof can be a solvate thereof. Examples of the salt of the compound include hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salts and potassium salts; alkaline earth metal salts such as magnesium salt and calcium salt; and ammonium salts such as ammonium salt, alkylammonium salt, dialkylammonium salt, trialkylammonium salt, and tetraalkylammonium salt. These salts can be produced by, for example, bringing the compound into contact with an acid or a base. The solvate as used herein means one in which a compound and a solvent together form a molecular aggregate, and is not particularly limited as long as it is a solvate formed by a solvent acceptable for ingestion along with administration of a medicament. Specific examples of the solvate include not only solvates formed with a single solvent such as water, alcohol (ethanol, methanol, 1-propanol, 2-propanol, or the like), or dimethyl sulfoxide, but also solvates formed with a plurality of solvents per one molecule of compound, or solvates formed with a plurality of types of solvents per one molecule of compound. For example, a solvate formed by a compound with water is called a hydrate. As the solvate of the compound of the present invention, the hydrate is preferred. Specifically, the hydrate is preferably 1 to 20 hydrates, more preferably 1 to 10 hydrates, further preferably 1 to 5 hydrates, most preferably 1 to 3 hydrates.

The "amino acid" as used herein includes a natural amino acid and a non-natural amino acid (sometimes also referred to as an amino acid derivative). The "amino acid" as used herein may mean an amino acid residue. The "natural amino acid" as used herein is any L-type amino acid selected from Gly (glycine), L-Ala (alanine), L-Ser (serine), L-Thr (threonine), L-Val (valine), L-Leu (leucine), L-Ile (isoleucine), L-Phe (phenylalanine), L-Tyr (tyrosine), L-Trp (tryptophan), L-His (histidine), L-Glu (glutamic acid), L-Asp (aspartate), L-Gln (glutamine), L-Asn (asparagine), L-Cys (cysteine), L-Met (methionine), L-Lys (lysine), L-Arg (arginine), or L-Pro (proline). The "non-natural amino acid" as used herein is an amino acid other than the natural amino acid. Examples of the non-natural amino acid include a $\beta$-amino acid, a $\gamma$-amino acid, a D-type amino acid, an N-substituted amino acid (excluding Pro), an $\alpha,\alpha$-disubstituted amino acid, and an amino acid having a side chain different from that of natural amino acids. As the amino acid herein, amino acids having any conformation are acceptable. The side chains of the amino acids are not particularly limited, and each side chain is freely selected from groups such as alkyl, alkenyl, alkynyl, aryl, heteroaryl, aralkyl, heteroaralkyl, cycloalkyl, or spiro-bonded cycloalkyl, and/or atoms such as a hydrogen atom. Each group and/or atom is further optionally substituted. In one non-limiting aspect, the amino acid as used herein may be a compound having a carboxy group and an amino group in the same molecule. Even in this case, compounds in which the nitrogen atom of the amino group and any atom of a side chain of the amino acid together form a ring, such as proline, hydroxyproline, and azetidine-2-carboxylic acid, are also included in the amino acid.

The backbone amino group of the amino acid may be unsubstituted (i.e., an $NH_2$ group) or may be substituted (i.e., a -NHR group wherein R represents alkyl, alkenyl, alkynyl, aryl, heteroaryl, aralkyl, or cycloalkyl optionally having a substituent, and one or two non-adjacent methylene groups in any of these groups may be substituted by an oxygen atom, a carbonyl group (-CO-), or a sulfonyl group ($-SO_2-$); and a carbon chain bonded to an N atom and a carbon atom at $\alpha$-position may form a ring, as in proline). The substituent of the R is selected in the same manner as in the substituent for the amino acid side chain mentioned above. In the case of a substituted backbone amino group, the R is included in the "side chain of the amino acid" as used herein. Such an amino acid having the substituted backbone amino group is referred to herein as the "N-substituted amino acid". Examples of the "N-substituted amino acid" as used herein preferably include, but are not limited to, N-alkyl amino acids, N-$C_1$-$C_6$ alkyl amino acids, N-$C_1$-$C_4$ alkyl amino acids, and N-methyl amino acids.

The "amino acid" as used herein includes all isotopes corresponding to each. The isotope of the "amino acid" is a form in which at least one atom is replaced with an atom having the same atomic number (proton number) and a different mass number (total number of protons and neutrons) at an abundance ratio different from the natural abundance ratio. Examples of the isotope contained in the "amino acid" herein include a hydrogen atom, a carbon atom, a nitrogen atom, an oxygen atom, a phosphorus atom, a sulfur atom, a fluorine atom, and a chlorine atom, and they include $^{2}H$, $^{3}H$, $^{13}C$, $^{14}C$, $^{15}N$, $^{17}O$, $^{18}O$, $^{32}P$, $^{35}S$, $^{18}F$, $^{36}Cl$, and the like, respectively. For the compounds as used herein, all the compounds

containing any proportions of radioactive or non-radioactive isotopic elements are encompassed within the scope of the present invention.

Examples of the "halogen-derived substituent" as used herein include fluoro (-F), chloro (-Cl), bromo (-Br), and iodo (-I).

Examples of the "oxygen atom-containing substituent" as used herein include hydroxy (-OH), oxy (-OR), oxo (=O), carbonyl (-C(=O)-R), carboxy (-$CO_2$H), oxycarbonyl (-C(=O)-OR), carbonyloxy (-O-C(=O)-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino (-NH-$SO_2$-R), aminosulfonyl (-$SO_2$-NHR), sulfamoylamino (-NH-$SO_2$-NHR), thiocarboxy (-C(=O)-SH), and carboxycarbonyl (-C(=O)-$CO_2$H).

Examples of the oxy (-OR) include alkoxy, cycloalkoxy, alkenyloxy, alkynyloxy, aryloxy, heteroaryloxy, and aralkyloxy. The alkoxy is preferably $C_1$-$C_4$ alkoxy or $C_1$-$C_2$ alkoxy, particularly preferably methoxy or ethoxy.

Examples of the carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of the oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of the carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of the thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of the carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of the aminocarbonyl (-C(=O)-NHR) include alkylaminocarbonyl (e.g., $C_1$-$C_6$ or $C_1$-$C_4$ alkylaminocarbonyl, specifically, ethylaminocarbonyl or methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Additional examples thereof include groups in which the H atom bonded to the N atom in -C(=O)-NHR is further substituted with alkyl, cycloalkyl, alkenyl, alkynyl, aryl, heteroaryl, or aralkyl.

Examples of the carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Additional examples thereof include groups in which the H atom bonded to the N atom in -NH-C(=O)-R is further substituted with alkyl, cycloalkyl, alkenyl, alkynyl, aryl, heteroaryl, or aralkyl.

Examples of the oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Additional examples thereof include groups in which the H atom bonded to the N atom in -NH-C(=O)-OR is further substituted with alkyl, cycloalkyl, alkenyl, alkynyl, aryl, heteroaryl, or aralkyl.

Examples of the sulfonylamino (-NH-$SO_2$-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Additional examples thereof include groups in which the H atom bonded to the N atom in -NH-$SO_2$-R is further substituted with alkyl, cycloalkyl, alkenyl, alkynyl, aryl, heteroaryl, or aralkyl.

Examples of the aminosulfonyl (-$SO_2$-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Additional examples thereof include groups in which the H atom bonded to the N atom in -$SO_2$-NHR is further substituted with alkyl, cycloalkyl, alkenyl, alkynyl, aryl, heteroaryl, or aralkyl.

Examples of the sulfamoylamino (-NH-$SO_2$-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Further, the two H atoms bonded to the N atom in -NH-$SO_2$-NHR is optionally substituted with substituents independently selected from the group consisting of alkyl, cycloalkyl, alkenyl, alkynyl, aryl, heteroaryl, and aralkyl, and the two substituents may form a ring.

Examples of the "sulfur atom-containing substituent" as used herein include thiol (-SH), thio (-S-R), sulfinyl (-S=O-R), sulfonyl (-S(O)$_2$-R), sulfo (-$SO_3$H), pentafluorosulfanyl (-$SF_5$), and disulfanyl (-S-S-R).

Examples of the thio (-S-R) include alkylthio, cycloalkylthio, alkenylthio, alkynylthio, arylthio, heteroarylthio, and aralkylthio.

Examples of the sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of the sulfonyl (-S(O)$_2$-R) include alkylsulfonyl, cycloalkylsulfonyl, alkenylsulfonyl, alkynylsulfonyl, arylsulfonyl, heteroarylsulfonyl, and aralkylsulfonyl.

Examples of the "nitrogen atom-containing substituent" as used herein include azide (-$N_3$, also referred to as "azide group"), cyano (-CN), primary amino (-$NH_2$), secondary amino (-NH-R), tertiary amino (-NR(R')), amidino (-C(=NH)-$NH_2$), substituted amidino (-C(=NR)-NR'R"), guanidino (-NH-C(=NH)-$NH_2$), substituted guanidino (-NR-

C(=NR‴)-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

Examples of the tertiary amino (-NR(R')) include an amino group having optional two substituents each independently selected from alkyl, cycloalkyl, alkenyl, alkynyl, aryl, heteroaryl, aralkyl, and the like, such as alkyl(aralkyl)amino, and the optional two substituents may form a ring.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R, R', R", and R‴ are each independently selected from alkyl, cycloalkyl, alkenyl, alkynyl, aryl, heteroaryl, and aralkyl, and groups in which these substituents form a ring.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

As used herein, the "amino acid residue" constituting a peptide compound is sometimes referred to simply as an "amino acid".

As used herein, the "to" that indicates a numerical range includes values of both ends thereof. For example, "A to B" means the numerical range of A or more and B or less.

As used herein, the term "approximately", when used in combination with a numeric value, means the value range of +10% and -10% of the numeric value.

As used herein, the term "and/or" is meant to include every combination of the terms "and" and "or" appropriately combined. Specifically, for example, the term "A, B, and/or C" includes the following seven variations: (i) A, (ii) B, (iii) C, (iv) A and B, (v) A and C, (vi) B and C, and (vii) A, B, and C.

## Method for producing peptide compound

In an aspect, the present invention relates to a method for producing a peptide compound or a salt thereof. The method comprises a step (linking step) of linking an amino group of a first amino acid or peptide and a carboxy group of a second amino acid or peptide with an amide bond, wherein a condensing agent and two or more additives are present in a reaction system of the linking step, and pKa of the additive is between 0 and 10.0, more preferably between 0 and 8.0, further preferably between 0 and 5.0, and most preferably between 1 and 5.0 (hereinafter also referred to as "aspect 1").

In an aspect, the present invention relates to a method for producing a peptide compound or a salt thereof. The method comprises a step (linking step) of linking an amino group of a first amino acid or peptide and a carboxy group of a second amino acid or peptide with an amide bond, wherein a condensing agent and two or more additives are used in the linking step, and pKa of the additive is between 0 and 10.0, more preferably between 0 and 8.0, further preferably between 0 and 5.0, and most preferably between 1 and 5.0 (hereinafter also referred to as "aspect 2").

In an aspect, the additive used in the linking step has pKa of between 0 and 10.0, more preferably between 0 and 8.0, further preferably between 0 and 5.0, and most preferably between 1 and 5.0. The "pKa" as used herein can be measured using water as a solvent. As the pKa used herein, a measured value that has already been reported as pKa when using water as a solvent may be employed. A value measured at 25°C is applied as the measured value of pKa. When the measured value of pKa is not available, pKa may be calculated with ADMETPredictor (Simulations Plus Inc., ver8.0) and employed as pKa herein. The pKa of the representative additives contemplated in this aspect are listed below:

HOAt (pKa = 3.28; Chem. Eur. J. 2009, 15, 9394-9403);
HOBt (pKa = 4.60; Chem. Eur. J. 2009, 15, 9394-9403);
oxyma (pKa = 4.60; Chem. Eur. J. 2009, 15, 9394-9403);
tert-Butyl2-cyano-2-(hydroxyimino)acetate (pKa = 4.60; ACS Omega. 2022, 7, 6007-6023);
(2,2-Dimethyl0-1,3-dioxolan-4-yl)methyl2-cyano-2-(hydroxyimino)acetate (pKa = 4.5; ACS Omega. 2022, 7, 6007-6023);
2-Amino-N-hydroxy-oxoacetimidoylcyanide (pKa = 6.3; ACS Omega. 2022, 7, 6007-6023);
2-(Ethylamino)-N-hydroxy-2-oxoacetimidoylcyanide (pKa = 6.3; ACS Omega. 2022, 7, 6007-6023);
2-(Dimethylamino)-N-ydroxy-2-oxoacetimidoylcyanide (pKa = 6.3; ACS Omega. 2022, 7, 6007-6023);
N-Hydroxy-2-oxo-2-(Piperidin-1-yl)acetimidoyl cyanide (pKa = 6.3; ACS Omega. 2022, 7, 6007-6023);
N-Hydroxy-2-morpholino-2-oxoacetimidoylcyanide (pKa = 6.1; ACS Omega. 2022, 7, 6007-6023);
Oxyma-B (5-(Hydroxyimino)-1,3-dimethylpyrimidine-2,4,6(1H,3H,5H)-trione) (pKa = 8.2; ACS Omega. 2022, 7, 6007-6023);
Oxyma-T (1,3-diethyl-5-hydroxyimino-2-thioxo-hexahydropyrimidine-4,6-dione) (pKa = 8.2; ACS Omega. 2022, 7,

6007-6023);
Hydroxyimino-2-phenylacetonitrile (pKa = 8.2; ACS Omega. 2022, 7, 6007-6023);
Hydroxyimino-2-(4-chloro)phenylacetonitrile (pKa = 8.0; ACS Omega. 2022, 7, 6007-6023);
Hydroxypicolinimidoyl cyanide (pKa = 8.2; ACS Omega. 2022, 7, 6007-6023);
Hydroxyimino-2-(1-naphytyl)phenylacetonitrile (pKa = 8.2; ACS Omega. 2022, 7, 6007-6023);
Hydroxycarbonimidoyl dicyanide (pKa = 3.8; ACS Omega. 2022, 7, 6007-6023);
1-Ethyl-3-methyl-2-(hydroxyamino)malonate (pKa = 7.2; ACS Omega. 2022, 7, 6007-6023);
Diethyl 2-(hydroxyamino)malonate (pKa = 7.2; ACS Omega. 2022, 7, 6007-6023);
Diisopyrplyl 2-(hydroxyimino)malonate (pKa = 7.2; ACS Omega. 2022, 7, 6007-6023);
HONM (5-(Hyrdoxyimino)-2,2-dimethyl-1,3-dioxane-4,6-dione) (pKa = 6.1; ACS Omega. 2022, 7, 6007-6023);
HOSu (1-Hydroxypyrrolidine-2,5-dione) (pKa = 7.7; ACS Omega. 2022, 7, 6007-6023);
HONB ((4S,7S)-2-Hydroxy-3a,4,7,7a-tetrahydro-1H-4,7-methanoisoindole-1,3-(2H)-dione) (pKa = 7.7; ACS Omega. 2022, 7, 6007-6023);
HOBI (2-Phenyl-1H-benzo[d]imidazoyl-1-ol) (pKa = 7.7; ACS Omega. 2022, 7, 6007-6023);
6-C1-HOBI (6-Chloro-2-phanyl-1H-benzo[dimidazol]-1-ol) (pKa = 7.1; ACS Omega. 2022, 7, 6007-6023);
HODhad (3-Hydroxypyrido[3,2-d]pyrimidin-4(3H)-one) (pKa = 5.8; ACS Omega. 2022, 7, 6007-6023);
2H-Tetrazol-2-ol (pKa = 8.2; ACS Omega. 2022, 7, 6007-6023);
HOI (1-Hydroxyindolin-2-one) (pKa = 8.3; ACS Omega. 2022, 7, 6007-6023);
2-(Hydroxyimino)-2-(pyrazin-2-yl)acetamide (pKa = 9.4; ACS Omega. 2022, 7, 6007-6023);
Ethyl-2-(hydroxyimino)-2-(pyrazin-2-yl)acetate (pKa = 9.4; ACS Omega. 2022, 7, 6007-6023);
Ethyl-2-(furan-2-yl)-2-(hydroxyimino)acetate (pKa = 9.6; ACS Omega. 2022, 7, 6007-6023);
2-(Furan-2-yl)-2-(hydroxyimino)acetamide (pKa = 9.4; ACS Omega. 2022, 7, 6007-6023);
Ethyl 2-(hydroxyimino)-2-(thianol-2-yl)acetate (pKa = 8.7; ACS Omega. 2022, 7, 6007-6023);
2(Hydroxyimino)-2-(thiazol-2-yl)acetamide (pKa = 8.7; ACS Omega. 2022, 7, 6007-6023);
Ethyl 2-(hydroxyimino)-2-(pyrimidin-2-yl)acetate (pKa = 9.6; ACS Omega. 2022, 7, 6007-6023);
2-(Hydroxyimino)-2-(pyrimidin-2-yl)acetamide (pKa = 9.4; ACS Omega. 2022, 7, 6007-6023);
2-(Hydroxyimino)-2-(pyrimidin-2-yl)ethanethioamide (pKa = 10.5; ACS Omega. 2022, 7, 6007-6023);
Ethyl 2-(furan-2-yl)-2-(hydroxyimino)acetate (pKa = 9.6; ACS Omega. 2022, 7, 6007-6023);
2-(Hydroxyimino)-2-(1H-pyrrol-2-yl)acetamide (pKa = 9.4; ACS Omega. 2022, 7, 6007-6023);
2-(Hydroxyimino)-2-(thiophen-2-yl)acetamide (pKa = 9.4; ACS Omega. 2022, 7, 6007-6023);
Ethyl 2-(hydroxyimino)-(1H-pyrrol-2-yl)acetate (pKa = 9.6; ACS Omega. 2022, 7, 6007-6023);
Ethyl 2-(hydroxyimino)-2-(thiophen-2-yl)acetate (pKa = 9.6; ACS Omega. 2022, 7, 6007-6023);
Ethyl 2-(hydroxyimino)-2-(pyridine-2-yl)acetate (pKa = 9.6; ACS Omega. 2022, 7, 6007-6023);
Ehyl2-(hydroxyimino)-2-(pyridine-4-yl)acetate (pKa = 9.6; ACS Omega. 2022, 7, 6007-6023);
2-(Hydroxyimino)but-3-ynamide (pKa = 9.4; ACS Omega. 2022, 7, 6007-6023);
Ethyl 2-(hydroxyimino)but-3-ynoate (pKa = 9.6; ACS Omega. 2022, 7, 6007-6023);
Ethyl 2-(hydroxyamino)-2-nitroacetate (pKa = 4.7; ACS Omega. 2022, 7, 6007-6023);
Dinitromethanoneoxime (pKa = 2.7; ACS Omega. 2022, 7, 6007-6023);
Nitro(phenyl)methanoneoxime (pKa = 7.6; ACS Omega. 2022, 7, 6007-6023);
1-Nitro-3-phenylpropan-1-oneoxime (pKa = 7.4; ACS Omega. 2022, 7, 6007-6023);
1-(Hydroxyimino)-N,N-dimethyl-1-phenylmethanesulfinamide (pKa = 7.4; ACS Omega. 2022, 7, 6007-6023);
Ethyl 2-(hydroxyimino)-2-sulfamoylacetate (pKa = 5.8; ACS Omega. 2022, 7, 6007-6023);
(Hydroxyimino)(methylsulfonyl)methanesulfinamide (pKa = 9.4; ACS Omega. 2022, 7, 6007-6023);
2-(Hydroxyimino)-2-(methylsulfonyl)acetamide (pKa = 9.4; ACS Omega. 2022, 7, 6007-6023);
Ethyl 2-(hydroxyimino)-2-((trifluoromethyl)sulfonyl)acetate (pKa = 4.2; ACS Omega. 2022, 7, 6007-6023);
2-Ethoxy-N-hydroxy-2-oxoacetimidictrifluoromethaneslfonic anhydride (pKa = 7.74; ACS Omega. 2022, 7, 6007-6023);
Diphenylmethaneoxime (pKa = 11.1; ACS Omega. 2022, 7, 6007-6023);
2-((Hydroxyimino)(pyridin-2-yl)methyl)-1-methylpyridin-1-iumiodide (pKa = 5.6; ACS Omega. 2022, 7, 6007-6023);
9H-Fluoren-9-oneoxime (pKa = 11.1; ACS Omega. 2022, 7, 6007-6023);
Anthracene-9,10-dionedioxime (pKa = 7.1; ACS Omega. 2022, 7, 6007-6023);
Pyrido[3,4-g]isoquinoline-5,10-dioxime (pKa = 5.5; ACS Omega. 2022, 7, 6007-6023);
Imidazolidin-2-oneoxime (pKa = 12.5; ACS Omega. 2022, 7, 6007-6023);
1,3-Dihydro-2H-imidazol-2-oneoxime (pKa = 8.2; ACS Omega. 2022, 7, 6007-6023);
Oxazolidin-2-oneoxime (pKa = 12.5; ACS Omega. 2022, 7, 6007-6023);
Oxazol-2(3H)-oneoxime (pKa = 6.6; ACS Omega. 2022, 7, 6007-6023);
2-(Hydroxyimino)malonamide (pKa = 9.4; ACS Omega. 2022, 7, 6007-6023);
N',N'-Dihydroxy-2-(hydroxyamino)malonamide (pKa = 7.7; ACS Omega. 2022, 7, 6007-6023);

Butane-2,3-dionedioxime (pKa = 12.1; ACS Omega. 2022, 7, 6007-6023);
N'-Hydroxypyrazine-2-carboximidamide (pKa = 12.5; ACS Omega. 2022, 7, 6007-6023);
3-Methyl-1,2,4-oxadiazole-5-carbaldehydeoxime (pKa = 8.0; ACS Omega. 2022, 7, 6007-6023);
3-(Hydroxyimino)-1-phenylindoline-2-one (pKa = 9.0; ACS Omega. 2022, 7, 6007-6023);
3-(Hydroxyimino)-1-methylindolin-2-one (pKa = 9.4; ACS Omega. 2022, 7, 6007-6023);
2-((Hydroxyimino)methyl)-1-methylpyridine-1-ium (pKa = 8.0; ACS Omega. 2022, 7, 6007-6023);
2,3,4,5,6,-Pentafluorophenol (pKa = 4.9; ACS Omega. 2022, 7, 6007-6023);
2,3,5-Trichlorophenol (pKa = 6.4; ACS Omega. 2022, 7, 6007-6023);
4-Nitrophenol (pKa = 7.2; ACS Omega. 2022, 7, 6007-6023);
Phenol (pKa = 10.0; ACS Omega. 2022, 7, 6007-6023);
HFIP (1,1,1,3,3,3-Hexafluoropropan-2-ol) (pKa = 9.3; Tetrahedron Asymmetry, 2012, 23, 1023-1027)

In aspects 1 and 2, the condensing agent and the two or more additives may be added to the reaction system comprising the first amino acid or peptide and the second amino acid or peptide, or the first amino acid or peptide and the second amino acid or peptide may be added to the reaction systems comprising the condensing agent and the two or more additives separately.

In an aspect of aspects 1 and 2, the condensing agent is a phosphorus-based condensing agent. Herein, the phosphorus-based condensing agent is not particularly limited as long as it is a compound having a phosphorus atom in the molecule and facilitating the linking (condensation reaction) of the amino group and the carboxy group, and, for example, the phosphorus-based condensing agent may be a compound represented by the following formula (1):

[Formula 2]

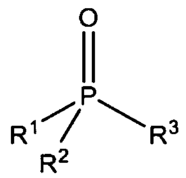

(1)

wherein, $R^1$ is $C_1$-$C_6$ alkoxy, $C_6$-$C_{10}$ aryloxy, $C_6$-$C_{10}$ aryl, or di-$C_1$-$C_6$ alkylamino;
$R^2$ is $C_1$-$C_6$ alkoxy, $C_6$-$C_{10}$ aryloxy, $C_6$-$C_{10}$ aryl, or di-$C_1$-$C_6$ alkylamino; and
$R^3$ is halogen, optionally substituted $C_6$-$C_{10}$ aryloxy, or azide.

In the phosphorus-based condensing agent represented by formula (1), those in which $R^1$ is ethoxy, phenyloxy, phenyl, or dimethylamino; $R^2$ is ethoxy, phenyloxy, phenyl, or dimethylamino; and $R^3$ is chloro, pentafluorophenyloxy, or azide are preferred.

In an aspect of aspects 1 and 2, specific examples of the phosphorus-based condensing agent include diphenyl chlorophosphate (DPPC), diethyl chlorophosphate (DEPC), dimethyl chlorophosphate, diisopropyl chlorophosphate, dibenzyl chlorophosphate, diphenylphosphoryl azide (DPPA), pentafluorophenyl diphenyl phosphinate (FDPP), 2-chloro-2-oxo-1,3,2-disoxaphosphorane, dimethylphosphinic chloride, diethylphosphinic chloride, dicyclohexylphosphinic chloride, and diphenylphosphinic chloride ($Ph_2P(O)Cl$). Among them, diphenyl chlorophosphate (DPPC), diethyl chlorophosphate (DEPC), diphenylphosphoryl azide (DPPA), pentafluorophenyl diphenyl phosphinate (FDPP), and diphenylphosphinic chloride ($Ph_2P(O)Cl$) are preferred, and diphenyl chlorophosphate (DPPC) and diethyl chlorophosphate (DEPC) are more preferred.

In aspects 1 and 2, the additive is a nucleophile that reacts with a carboxy group to form an active ester. Preferably, the additives are two or more selected from the group consisting of 2-hydroxypyridine-N-oxide (HOPO), a HOPO derivative, 1-hydroxy-7-azabenzotriazole (HOAt), 1-hydroxybenzotriazole (HOBt), 3,4-dihydro-3-hydroxy-4-oxo-benzotriazine (HOOBt), ethyl 1-hydroxy-1H-1,2,3-triazole-4-carboxylate (HOCt), 2,2,3,3,3-pentafluoro-1-propanol (PfpOH), ethyl 2-cyano-2-(hydroxyimino)acetate (Oxyma), 5-(hydroxyimino)-1,3-dimethylpyrimidine-2,4,6-(1H,3H,5H)-trion (Oxyma-B), 4-nitrophenol, pentafluorophenol, ethyl(hydroxyimino)cyanoacetate potassium salt (K-Oxyma), 4,6-dimethoxy-1,3,5-triazin-2-ol, 2-hydroxypyridine, trimethylsilanol, and 2-hydroxy-5-nitropyridine, and more preferably two or more selected from the group consisting of 2-hydroxypyridine-N-oxide (HOPO), a HOPO derivative, 3,4-dihydro-3-hydroxy-4-oxo-benzotriazine (HOOBt), 2,2,3,3,3-pentafluoro-1-propanol (PfpOH), ethyl 2-cyano-2-(hydroxyimino)acetate (Oxyma), 5-(hydroxyimino)-1,3-dimethylpyrimidine-2,4,6-(1H,3H,5H)-trion (Oxyma-B), and pentafluorophenol. Specific examples of the HOPO derivative include 5-F-HOPO, 5-Cl-HOPO, 5-Br-HOPO, 5-CF3-HOPO, 5-CO2Me-HOPO, and 4-Br-HOPO.

In aspects 1 and 2, the additives are preferably two additives.

In an aspect of aspects 1 and 2, the condensing agent is used in 0.1 to 5.0 molar equivalents, more preferably 0.5 to 3.0 molar equivalents, further preferably 0.5 to 2.6 molar equivalents, most preferably 0.5 to 2.0 molar equivalents, relative to the first amino acid or peptide.

In an aspect of aspects 1 and 2, one of the two or more additives is HOPO and the other one or more is selected from the group consisting of Oxyma, HOAt, HOOBt, HOCt, PfpOH, Oxyma-B, pentafluorophenol, and K-Oxyma.

In another aspect of aspects 1 and 2, one of the two or more additives is Oxyma and the other one or more is selected from the group consisting of HOPO, a HOPO derivative, HOOBt, HOCt, Oxyma-B, pentafluorophenol, PfpOH, 4,6-dimethoxy-1,3,5-triazin-2-ol, 2-hydroxypyridine, and 2-hydroxy-5-nitropyridine. In this aspect, it is preferred that one of the two or more additives is Oxyma and the other one or more is selected from the group consisting of HOPO, HOOBt, HOCt, Oxyma-B, and pentafluorophenol, and it is more preferred that the two or more additives are two additives, Oxyma and HOPO.

In an aspect of aspects 1 and 2, the additives are used in 0.1 to 5.0 molar equivalents, more preferably 0.3 to 3.0 molar equivalents, further preferably 0.5 to 2.6 molar equivalents, most preferably 0.5 to 2.0 molar equivalents, relative to the first amino acid or peptide.

In an aspect, the linking step can be carried out, for example, by stirring the reaction mixture at a reaction temperature of -50°C to 30°C, preferably -20°C to 20°C, further preferably -18°C to 20°C, most preferably 0°C to 15°C, for 10 minutes to 24 hours.

There is also a known method of separating peptide components from other unnecessary components by selectively dissolving only peptide components bound to the dissolved tag in a specific phase along with liquid-phase separation (hereinafter referred to as "liquid-phase tag method"). According to this method, unnecessary components can be separated without solidification, which can contribute to the speed and simplification of the reaction process. Among them, in particular, examples of the liquid-phase tag method using the hydrophobic tag include the methods described in Y. Okada, H. Suzuki, T. Nakae, S. Fujita, H. Abe, K. Nagano, T. Yamada, N. Ebata, S. Kim and K. Chiba, Tag-Assisted Liquid-Phase Peptide Synthesis Using Hydrophobic Benzyl Alcohols as Supports, J. Org. Chem., 2013, 78, 320-327., or S. Yano et al., Molecules 2021, 26 (12), 3497.

In an aspect, the linking step may be carried out in solid-phase synthesis or liquid-phase synthesis, but preferably is carried out in liquid-phase synthesis. When the linking step is carried out in a liquid-phase synthesis, the liquid-phase synthesis may be carried out in an organic solvent, and a liquid-phase tag method may be used.

In an aspect, specific examples of the organic solvent used in the linking step include acetonitrile, isopropyl acetate, ethyl acetate, butyl acetate, methyl t-butyl ether, diethyl ether, dichloromethane, tetrahydrofuran, 2-methyltetrahydrofuran, N,N-dimethylformamide, N,N-dimethylacetamide, N-methylpyrrolidone, dimethylsulfoxide, α,α,α,-trifluorotoluene, toluene, hexane, cyclopentyl methyl ether, or a mixed solvent thereof. Among them, the organic solvent is preferably at least one selected from the group consisting of acetonitrile, isopropyl acetate, methyl t-butyl ether, dichloromethane, tetrahydrofuran, 2-methyltetrahydrofuran, N,N-dimethylformamide, N,N-dimethylacetamide, α,α,α-trifluorotoluene, toluene, and cyclopentyl methyl ether, or a mixed solvent thereof, more preferably at least one selected from the group consisting of isopropyl acetate, tetrahydrofuran, 2-methyltetrahydrofuran, α,α,α-trifluorotoluene, and toluene, or a mixed solvent thereof, and more preferably at least one selected from the group consisting of 2-methyltetrahydrofuran, α,α,α-trifluorotoluene, and toluene, or a mixed solvent thereof. As the mixed solvent, a mixed solvent of isopropyl acetate and methyl t-butyl ether or a mixed solvent of isopropyl acetate and acetonitrile is preferred.

In an aspect, the organic solvent is used at 10 to 1000 mg/mL, preferably at 20 to 500 mg/mL, and further preferably at 50 to 200 mg/mL, relative to the first amino acid or peptide.

In an aspect, a base is used in the linking step.

In an aspect, the base used in the linking step has pKa of the conjugate acid of 15.0 or less, preferably 13.0 or less, more preferably 11.5 or less. In an aspect, the base used in the linking step has pKa of the conjugate acid of 0 or more, preferably 3 or more, more preferably 5 or more. In an aspect, the base used in the linking step has pKa of the conjugate acid of between 0 and 15.0, preferably between 0 and 11.5, more preferably between 3 and 11.5, further preferably between 5 and 11.5, most preferably between 6 and 11.5. The "pKa" as used herein can be measured using water as a solvent. As the pKa used herein, measured values that have already been reported as pKa when using water as a solvent may be employed. A value measured at 25°C is applied as the measured value of pKa. When the measured value of pKa is not available, pKa may be calculated with ADMETPredictor (Simulations Plus Inc., ver8.0) and employed as pKa herein. Listed below are pKa of representative reagents:

Conjugate acid of DBU (pKa = 11.9; R. Srivastava, J. Mol. Catal. A: Chem. 264 (2007) 146-152);
Conjugate acid of piperidine (pKa = 11.22; Hall, H.K., Jr. J. A.m. Chem. Soc. 1957, 79, 5441);
Conjugate acid of triethylamine (pKa = 10.65; Hall, H.K., Jr. J. A.m. Chem. Soc. 1957, 79, 5441);
Conjugate acid of diisopropylethylamine (pKa = 11.44; Chemical and Pharmaceutical Bulletin, 1995, 43, 1872-1877);
Conjugate acid of 2,4,6-collidine (pKa = 7.48; Clarke, K., Rothwell, K. J. Chem. Soc. 1960, 1885);
Conjugate acid of 2,6-lutidine (pKa = 6.77; Clarke, K., Rothwell, K. J. Chem. Soc. 1960, 1885);

Conjugate acid of N-methylmorpholine (pKa = 7.38; Analytical Sciences, 1996, vol.12)
Conjugated acids of pyridine (pKa = 5.21; D.H. Ripin, D.A. Evans, pKa's of Nitrogen Acids, [online], [searched on September 22, 2023],);

HCl (pKa = -8.0; D.H. Ripin, D.A. Evans, pKa's of Inorganic and Oxo-Acids,[online], [searched on December 5, 2017], internet
<URL:http://evans.rc.fas.harvard.edu/pdf/evans_pKa_table.pdf>);
HFIP(1,1,1,3,3,3-Hexafluoropropan-2-ol)(pKa = 9.3; Tetrahedron Asymmetry, 2012, 23, 1023-1027)

In an aspect, the base used in the linking step is an organic base. Examples of the organic base include dicyclohexylmethylamine, diisopropylethylamine, triethylamine, 1-methylpiperidine, N-methylmorpholine, 4-N,N-dimethylaminopyridine, 2,6-lutidine, 2,4,6-collidine, N-methylimidazole, 1,8-diazabicyclo[5.4.0]-7-undecene, 1,5-diazabicyclo[4.3.0]-5-nonene, 1,1,3,3-tetramethylguanidine, 2-tert-butyl-1,1,3,3-tetramethylguanidine, and 7-methyl-1,5,7-triazabicyclo[4.4.0]deca-5-ene. The organic base used in the linking step is preferably one selected from the group consisting of dicyclohexylmethylamine, diisopropylethylamine, triethylamine, 1-methylpiperidine, and 2-tert-butyl-1,1,3,3-tetramethylguanidine.

In an aspect, the base used in the linking step is an inorganic base, preferably an alkali metal carbonate or alkali metal phosphate. Examples of the alkali metal carbonate include sodium carbonate, potassium carbonate, and cesium carbonate. Examples of the alkali metal phosphate include sodium phosphate and potassium phosphate. The inorganic base used in the linking step is preferably potassium carbonate or potassium phosphate.

In an aspect, the organic base or inorganic base used in the linking step is used in 1.1 to 10.0 molar equivalents, preferably 2.0 to 9.0 molar equivalents, and further preferably 3.0 to 8.0 molar equivalents, relative to the first amino acid or peptide.

The "first amino acid" and "second amino acid" as used herein are terms used only to distinguish reaction points between amino acids, which are the substrates in the linking step. Specifically, the "first amino acid" means an amino acid in which the amino group is the reaction point, and the "second amino acid" means an amino acid in which the carboxy group is the reaction point.

In an aspect, the first amino acid or peptide is a peptide containing two or more amino acid residues.

In an aspect, the amino group of the first amino acid or peptide is an amino group represented by formula: $-NR^6R^7$, wherein $R^6$ is a hydrogen atom, and $R^7$ is linear $C_1$-$C_6$ alkyl, branched $C_3$-$C_6$ alkyl, or $C_3$-$C_8$ cycloalkyl. In a certain aspect, the amino group of the first amino acid or peptide is an amino group represented by formula: $-NR^6R^7$, wherein $R^6$ is a hydrogen atom, and $R^7$ is a hydrogen atom or linear $C_1$-$C_4$ alkyl. In a certain aspect, the amino group of the first amino acid or peptide is an amino group represented by formula: $-NR^6R^7$, wherein $R^6$ is a hydrogen atom, and $R^7$ is a hydrogen atom, methyl, or ethyl. In a certain aspect, the amino group of the first amino acid or peptide is an amino group represented by formula: $-NR^6R^7$, wherein $R^6$ is a hydrogen atom, and $R^7$ is a hydrogen atom or methyl. In a certain aspect, the amino group of the first amino acid or peptide is an amino group represented by formula: $-NR^6R^7$, wherein $R^6$ is a hydrogen atom, and $R^7$ is methyl.

In a certain aspect, the carboxy group of the first amino acid or peptide is optionally protected with a protecting group. Any protecting group known in the art can be used as the protecting group for the carboxy group as long as it does not reduce the solubility of the peptide in the solvent. Specific examples of such a protecting group for the carboxy group include a methyl group, an ethyl group, a t-butyl group, a trityl group, a cumyl group, a benzyl group, and an allyl group, and among these, a t-butyl group is preferred.

In an aspect, the second amino acid or peptide is a peptide containing two or more amino acid residues.

In an aspect, the $\alpha$-carbon of the carboxy group of the second amino acid or peptide is optionally substituted. In an aspect, the $\alpha$-carbon of the carboxy group of the second amino acid or peptide is represented by formula: $-CR^8R^9-$, wherein $R^8$ and $R^9$ are identical or different and each is a hydrogen atom, linear $C_1$-$C_4$ alkyl, optionally substituted branched $C_3$-$C_6$ alkyl, optionally substituted phenyl $C_1$-$C_2$ alkyl, or optionally substituted 5- to 6-membered heteroaryl $C_1$-$C_2$ alkyl.

When $R^1$ or $R^2$ is branched $C_3$-$C_6$ alkyl, the branched $C_3$-$C_6$ alkyl is optionally substituted with one or more halogens.

When $R^1$ or $R^2$ is phenyl-$C_1$-$C_2$ alkyl, the phenyl-$C_1$-$C_2$ alkyl is optionally substituted with one or more selected from the group consisting of methyl, ethyl, propyl, halogen, methoxy, ethoxy and trifluoromethyl.

When $R^1$ or $R^2$ is 5- to 6-membered heteroaryl-$C_1$-$C_2$ alkyl, the 5- to 6-membered heteroaryl-$C_1$-$C_2$ alkyl is optionally substituted with one or more selected from the group consisting of methyl, ethyl, propyl, halogen, methoxy, ethoxy and trifluoromethyl.

In a certain aspect, the $\alpha$-carbon of the carboxy group of the second amino acid or peptide is represented by formula: $-CR^8R^9-$, wherein $R^8$ and $R^9$ are identical or different and each is a hydrogen atom, linear $C_1$-$C_3$ alkyl, isopropyl, or optionally substituted benzyl.

When $R^1$ or $R^2$ is benzyl, the benzyl or phenethyl is optionally substituted with one or more selected from the

group consisting of fluorine, methyl, ethyl, methoxy, ethoxy, and trifluoromethyl.

In a certain aspect, the α-carbon of the carboxy group of the second amino acid or peptide is represented by formula: $-CR^8R^9-$, wherein $R^8$ is a hydrogen atom, and $R^9$ is a hydrogen atom, methyl, benzyl, or p-fluorobenzyl.

In an aspect, an amino group containing a nitrogen atom located at β position of the carboxy group of the second amino acid or peptide is represented by formula: $-NR^5-$, wherein $R^5$ is a hydrogen atom, linear $C_1$-$C_6$ alkyl, branched $C_3$-$C_6$ alkyl, or $C_3$-$C_8$ cycloalkyl.

In a certain aspect, an amino group containing a nitrogen atom located at β position of the carboxy group of the second amino acid or peptide is represented by formula: $-NR^5-$, wherein $R^5$ is a hydrogen atom or linear $C_1$-$C_4$ alkyl.

In a certain aspect, an amino group containing a nitrogen atom located at β position of the carboxy group of the second amino acid or peptide is represented by formula: $-NR^5-$, wherein $R^5$ is a hydrogen atom or methyl.

In an aspect, the amino group of the second amino acid or peptide is optionally protected with a protecting group. Any protecting group known in the art can be used as the protecting group for the amino group as long as it does not reduce the solubility of the peptide in the solvent. Specific examples of such a protecting group for the amino group include Cbz, p-nitrobenzyloxycarbonyl, 2-naphthylmethyloxycarbonyl, diphenylmethyloxycarbonyl, 9-anthrylmethyloxycarbonyl, Teoc, Fmoc, Boc, Alloc, trifluoroacetyl, triphenylmethyl, and methoxycarbonyl, and among these, Cbz, Teoc, Fmoc, Boc, Alloc, and trifluoroacetyl are preferred.

In an aspect, a peptide compound or a salt thereof produced by the method of the present invention contains amino acid residues of 8 to 20 residues, preferably 11 to 14 residues, more preferably 11 to 13 residues, most preferably 11 residues.

In an aspect, a peptide compound or a salt thereof produced by the method of the present invention can contain at least one, at least two, at least three, at least four, or at least five non-natural amino acid residues. In a certain aspect, the non-natural amino acid residues contained in the peptide compound or a salt thereof produced by the method of the present invention are N-methylamino acid residues.

In an aspect, the peptide compound produced by the method of the present invention may be a linear peptide compound. In another aspect, the peptide compound produced by the method of the present invention may be a cyclic peptide compound. In an aspect, the linear or cyclic peptide compound may comprise a cyclic structure (cyclic portion) as its moiety structure. Specific examples of the cyclic structure include those in which the side chain of one amino acid residue is linked to a side chain of another amino acid residue, those in which the N-substituent of one amino acid residue is linked to the side chain of another amino acid residue, and those in which the N-substituent of one amino acid residue is linked to the N-substituent of another amino acid residue. The two amino acid residues involved in the linkage for the cyclic structure may be adjacent, or any number of amino acid residues, for example, 1, 2, 3, 4, 5, 6, 7, 8, 9, 10, 11, 12, 13, 14, 15, 16, 17, 18, or 19 amino acid residues may be present therebetween. Examples of the size of the ring formed by the cyclic structure include, but are not intended to be particularly limited to, a 4-membered ring, a 5-membered ring, a 6-membered ring, a 7-membered ring, an 8-membered ring, a 9-membered ring, a 10-membered ring, an 11-membered ring, a 12-membered ring, a 13-membered ring, a 14-membered ring, a 15-membered ring, a 16-membered ring, a 17-membered ring, an 18-membered ring, a 19-membered ring, a 20-membered ring, a 21-membered ring, a 22-membered ring, a 23-membered ring, a 24-membered ring, a 25-membered ring, a 26-membered ring, a 27-membered ring, a 28-membered ring, a 29-membered ring, a 30-membered ring, a 31-membered ring, a 32-membered ring, a 33-membered ring, a 34-membered ring, and a 35-membered ring. Preferably, the ring formed by the cyclic structure is a 5-membered ring, a 6-membered ring, a 7-membered ring, an 8-membered ring, a 9-membered ring, a 10-membered ring, an 11-membered ring, a 12-membered ring, a 13-membered ring, a 14-membered ring, a 15-membered ring, a 16-membered ring, a 17-membered ring, an 18-membered ring, a 19-membered ring, or a 20-membered ring, more preferably a 10-membered ring, an 11-membered ring, a 12-membered ring, a 13-membered ring, a 14-membered ring, a 15-membered ring, a 16-membered ring, a 17-membered ring, or an 18-membered ring, most preferably an 11-membered ring, a 12-membered ring, a 13-membered ring, or a 14-membered ring. When a cyclic structure is present in the peptide compound, the number of cyclic structures is not limited, but it is preferable that one, two, three, four, or five cyclic structures be present.

In an aspect, the peptide compound or a salt thereof produced by the method of the present invention can contain a cyclic portion composed of 4 or more, 6 or more, 8 or more, or 11 or more amino acid residues. In an embodiment, the peptide compound or a salt thereof produced by the method of the present invention is a cyclic peptide compound or a salt thereof, consisting of a cyclic portion composed of 4 to 14, preferably 6 to 14, further preferably 8 to 14, most preferably 11 to 14 amino acid residues. In an embodiment, the peptide compound or a salt thereof produced by the method of the present invention is a cyclic peptide compound or a salt thereof, consisting of a cyclic portion composed of 11 amino acid residues. In these aspects, the amide bond with which the amino group of the first amino acid or peptide and the carboxy group of the second amino acid or peptide are linked is contained in the cyclic portion at 1, 2, 3, 4, 5, 6, or 7 locations.

In an embodiment, the method of the present invention may further include a step (deprotection step) of removing/deprotecting a protecting group of an amino group or carboxy group of the second amino acid or peptide and/or a protecting group of an amino group or carboxy group of the first amino acid or peptide in the peptide compound obtained in the linking step, immediately before or after and/or during the linking step. The deprotection step can be performed using

methods known in the art, for example, using the reagents and conditions described in "Greene's Protective Groups in Organic Synthesis, Fifth Edition, 2014".

In an aspect, the deprotection step of the protecting group of the amino group can be performed by contact hydrogenation, for example, when the protecting group is Cbz, p-nitrobenzyloxycarbonyl, 2-naphthylmethyloxycarbonyl, diphenylmethyloxycarbonyl, or 9-anthrylmethyloxycarbonyl. For the contact hydrogenation, any catalyst known in the art can be used. Specific examples of the catalyst include Pd/C, $Pd(OH)_2$/C, and $PtO_2$, and Pd/C is preferred.

In an aspect, the deprotection step of the protecting group of the amino group can be carried out using a reagent such as TBAF, $LiBH_4$, piperidine, trifluoroacetic acid, or methanesulfonic acid, for example, when the protecting group is Teoc, Fmoc, Boc, Alloc, or trifluoroacetyl.

In an aspect, the deprotection step of the protecting group of the carboxy group can be carried out under acidic conditions in the presence of a deprotection reagent, for example, when the protecting group is a methyl group, an ethyl group, a t-butyl group, a trityl group, a cumyl group, a benzyl group, or an allyl group.

In an aspect, the method of the present invention may further include a step (linking step A) of linking/condensing a third amino acid or peptide to a peptide compound obtained in the linking step or the deprotection step. That is, the method of the present invention may comprise repeating the linking step two or more times.

In an aspect, the method of the present invention may further include a step (linking step B) of linking/condensing an amino group and a carboxy group in a peptide compound obtained in the linking step or the deprotection step. In this case, the peptide compound obtained in the linking step B is a cyclic peptide compound.

In an aspect, the linking steps A and B can be performed, for example, in the presence or absence of a condensation reagent. Examples of the condensation reagent include T3P, EDCI, HATU, COMU, BEP, PyBOP, DMT-MM, and PyOxim.

In an aspect, the method of the present invention is performed using a flow reaction apparatus. The "flow reaction apparatus" as used herein refers to an apparatus that continuously supplies raw materials to a tubular reaction vessel, and mixes and reacts the raw materials to produce a target compound. The flow reaction apparatus may be a commercially available flow reaction apparatus (e.g., Africa flow chemistry system or Asia flow chemistry system manufactured by Syrris), or may be an apparatus that combines a tubular reaction vessel and a syringe pump.

## Peptide compound

In an aspect, the present invention is a peptide compound or a salt thereof produced by the "Method for producing peptide compound" described above. In an aspect, the present invention is a pharmaceutical composition containing a peptide compound or a salt thereof produced by the "Method for producing peptide compound" described above.

All references cited herein, including the following references, with inclusion of patent applications and publications, are incorporated herein by reference in their entirety: International Publication No. WO 2013/100132; International Publication No. WO 2018/225851; International Publication No. WO 2018/225864; International Publication No. WO 2019/117274; International Publication No. WO 2020/111238; International Publication No. WO 202012/2182; International Publication No. WO 2021/075478; International Publication No. WO 2021/090856; International Publication No. WO 2021132545; International Publication No. WO 2021246471; International Publication No. WO 2022/097540; International Publication No. WO 2022/138891; International Publication No. WO 20221/45444; International Publication No. WO 2022/234864; International Publication No. WO 2023/127869.

## Examples

The contents of the present invention will be further described by the following Examples, but the present invention is not limited to their contents. Except those as specifically described, starting substances, starting raw materials, solvents, and reagents were obtained from commercial suppliers, or synthesized using known methods. In Examples, "OxymaPure" is a trade name of a product obtained from a commercial supplier of ethyl 2-cyano-2-(hydroxyimino)acetate (Oxyma). $K_2CO_3$ Fine powder and $K_3PO_4$ Fine powder were obtained from a commercial supplier.

The $^1$H-NMR spectrum was measured using a nuclear magnetic resonance device JNM-ECZ 500 (manufactured by JEOL Ltd.), the chemical shift of tetramethylsilane used as an internal standard substance was set to 0 ppm, and a deuterium lock signal from a sample solvent was consulted. The chemical shift of a signal from a compound to be analyzed was expressed in ppm. Abbreviations for splitting of a signal were expressed as follows: s = singlet, brs = broad singlet, d = doublet, t = triplet, q = quartet, dd = double doublet, and m = multiplet. The splitting width of a signal was expressed as a J value (Hz). The integral value of signal was calculated on the basis of a ratio of the area intensity of each signal.

HPLC analysis was performed using H-Class system manufactured by Waters Corporation, by measuring at a wavelength of 210 nm with a PDA detector. The reactivity, selectivity, and purity of each substrate used in Examples were evaluated by the analytical method shown in Table 1 below. LCMS analysis was performed using a SQD2 or QDa detector.

[Table 1]

| Measurement method | Column | Measurement conditions |
|---|---|---|
| HPLC Method A | Waters Acquity CSH C18 (15 cm x 2.1 mm, 1.7 um) | Eluent A: 0.05% TFA in $H_2O$<br>Eluent B: 0.05% TFA in MeCN<br>Gradient(%B): 10%(0 min)→100%(19.0 min)→100%(21.0 min)→10%(21.01 min)→10%(25.0 min)<br>Flow rate: 0.3 mL/min<br>Column temperature: 50°C<br>Detection wavelength: 210 nm |
| HPLC Method B | Sigma Aldrich Ascentis Express C18 (5 cm x 4.6 mm, 2.7 um) | Eluent A: 0.05% TFA in $H_2O$<br>Eluent B: 0.05% TFA in MeCN<br>Gradient(%B): 5%(0 min)→100%(6.0 min)→100%(7.0 min)→5%(7.01 min)→5%(9.0 min)<br>Flow rate: 0.8 mL/min<br>Column temperature: 35°C<br>Detection wavelength: 210 nm |

Unless otherwise noted in the experiment section, HPLC samples were prepared as follows.
Condensation step: 3 to 10 μL of the reaction mixture was dissolved in MeCN (1.0 mL) and azetidine or 1-propylamine (0.1 mL).

The conversion rate of the condensation reaction and the diastereomer ratio were calculated by the following formula using the HPLC area value of each component in the reaction mixture. The substrate in the formula refers to the one with the lower molar number of filling amount among the two condensing substrates.

Conversion rate = [(desired product + epimer)/(substrate + desired product + epimer)] x 100 Diastereomer ratio = desired product/epimer

In the tables, the horizontal line (-) indicates that no calculations were made, "negligible" indicates that byproducts were detected in trace amounts, and "many" indicates that multiple byproducts were detected.

(Example 1) Condensation reaction of Cbz-MeLeu-MePhe(4-F)-OH and H-MeLeu-Ile-MeAla-Aze-EtPhe(4-Me)-Sar-OtBu

[Formula 3]

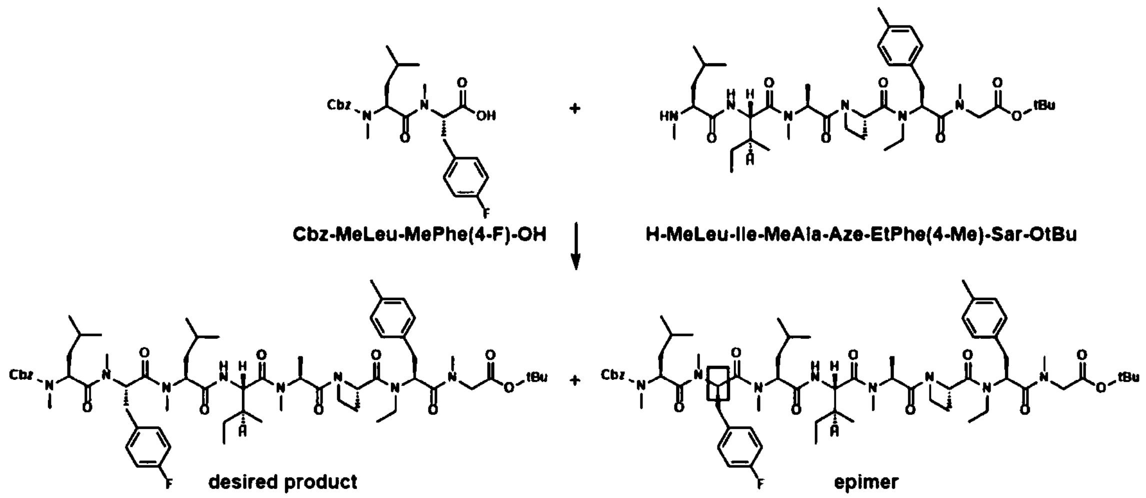

Cbz-MeLeu-MePhe(4-F)-OH

H-MeLeu-Ile-MeAla-Aze-EtPhe(4-Me)-Sar-OtBu

desired product

epimer

Reaction step

To a vial with a stirrer, HOPO (134.6 mg, 1.211 mmol, 2.0 equiv.), OxymaPure (86.1 mg, 0.606 mmol, 1.0 equiv.), a solution of Cbz-MeLeu-MePhe(4-F)-OH in 2-MeTHF (concentration 129.0 mg/mL, 2.60 mL, 335.0 mg, 0.731 mmol, 1.2 equiv.), a solution of H-MeLeu-Ile-MeAla-Aze-EtPhe(4-Me)-Sar-OtBu in 2-MeTHF (concentration 150.0 mg/mL, 3.00 mL, 450.0 mg, 0.606 mmol, 1.0 equiv.), and DIPEA (738 $\mu$L, 4.24 mmol, 7.0 equiv.) were sequentially added. The reaction mixture was cooled to 10°C with stirring. To the reaction mixture, DPPC (250 $\mu$L, 1.211 mmol, 2.0 equiv.) was added, and the mixture was stirred for 14 hours. The reaction mixture was analyzed by HPLC to confirm that reaction had progressed.

Conversion rate: 99.5%
Diastereomer ratio (dr): 99.9/0.1

Workup step

To the reaction mixture, 5% aqueous $Na_2CO_3$ solution (2.2 mL) was added, and the mixture was stirred for 1.5 hours. NMI (48 $\mu$L, 0.606 mmol) was added, and the mixture was further stirred for 1.5 hours. The aqueous layer was removed and 5% aqueous $Na_2CO_3$ solution (2.0 mL) was added to the resulting organic layer, and the mixture was stirred for 4 hours. The aqueous layer was removed, and the resulting organic layer was washed 3 times with 10% aqueous citric acid solution (2.0 mL x 3) and 2 times with 5% aqueous $Na_2CO_3$ solution (2.0 mL x 2). The resulting organic layer was concentrated to dryness to obtain 650.0 mg of the target.

Yield: 91%
Purity: 97.0%
Measurement method: HPLC Method A
Retention time: H-MeLeu-Ile-MeAla-Aze-EtPhe(4-Me)-Sar-OtBu: 10.2 min, desired product: 18.8 min, epimer: 19.0 min
Mass spectrometry: H-MeLeu-Ile-MeAla-Aze-EtPhe(4-Me)-Sar-OtBu: m/z 744.07 ([M+H]$^+$), desired product: m/z 1206.52 ([M+Na]$^+$), epimer: m/z 1206.35 ([M+Na]$^+$)

(Example 2) Condensation reaction of Cbz-MeLeu-MePhe(4-F)-OH and H-MeAla-Aze-EtPhe(4-Me)-Sar-OtBu

[Formula 4]

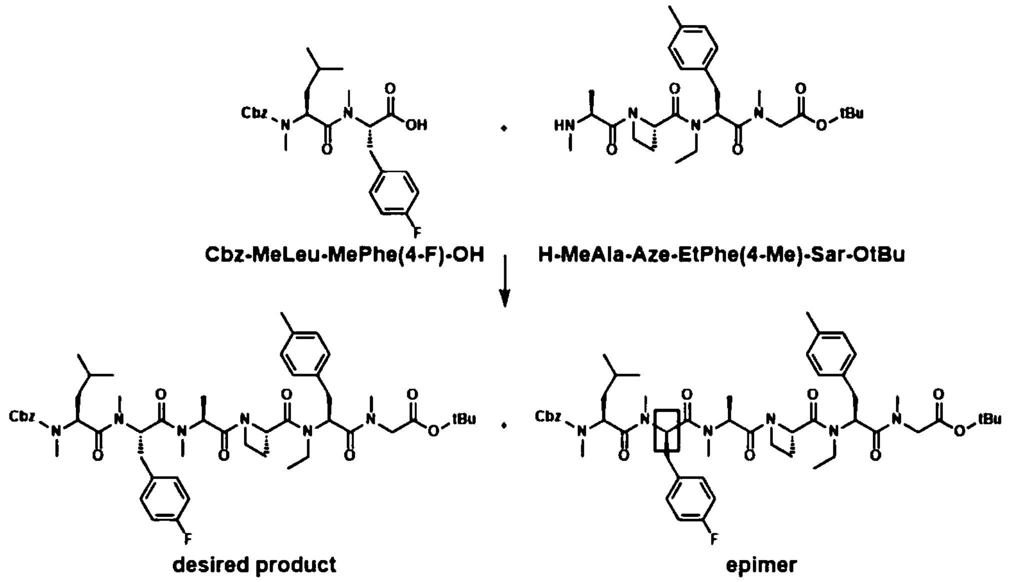

Cbz-MeLeu-MePhe(4-F)-OH H-MeAla-Aze-EtPhe(4-Me)-Sar-OtBu

desired product epimer

Under each condition shown in Table 2, to a test tube with a stirrer, additive-1, additive-2, a solution of Cbz-MeLeu-MePhe(4-F)-OH in 2-MeTHF (concentration 129.0 mg/mL, 0.300 mL, 38.7 mg, 0.084 mmol, 1.2 equiv.), a solution of H-MeAla-Aze-EtPhe(4-Me)-Sar-OtBu in 2-MeTHF (concentration 142.9 mg/mL, 0.245 mL, 35.0 mg, 0.070 mmol, 1.0 equiv.), and DIPEA (85.0 μL, 0.487 mmol, 7.0 equiv.) were sequentially added. The reaction mixture was cooled to 10°C with stirring. To the reaction mixture, DPPC (28.8 μL, 0.139 mmol, 2.0 equiv.) was added, and the mixture was stirred for 3 hours. The reaction mixture was analyzed by HPLC, and the conversion rate, the diastereomer ratio, and the byproduct amount were evaluated.

Measurement method: HPLC Method A
Retention time: H-MeAla-Aze-EtPhe(4-Me)-Sar-OtBu: 8.4 min, desired product: 17.5 min, epimer: 17.8 min
Mass spectrometry: H-MeAla-Aze-EtPhe(4-Me)-Sar-OtBu: m/z 503.81 ($[M+H]^+$), desired product: m/z 944.14 ($[M+H]^+$), epimer: m/z 944.14 ($[M+H]^+$)

[Table 2]

| Reaction conditions | | | Analysis results | | |
|---|---|---|---|---|---|
| Run | Additive-1 | Additive-2 | Conversion rate (%) | Diastereomer ratio | Byproduct amount |
| 1 | HOPO (2.0 eq.) | OxymaPure (1.0 eq.) | 99.7 | 99.7 / 0.3 | negligible |
| 2 | HOPO (3.0 eq.) | none | 99.5 | 96.6 / 3.4 | negligible |
| 3 | none | OxymaPure (3.0 eq.) | 99.8 | 99.9 / 0.1 | many |
| 4 | none | none | 98.7 | 84.0 / 16.0 | many |

As shown in Table 2, it was found that the highest conversion rate, diastereomer ratio, and purity of the target were obtained from the conditions of adding both HOPO and OxymaPure.

<u>Comparative experiments with HATU</u>

To a vial with a stirrer, a solution of Cbz-MeLeu-MePhe(4-F)-OH in 2-MeTHF (concentration 129.0 mg/mL, 0.300 mL, 38.7 mg, 0.084 mmol), a solution of H-MeAla-Aze-EtPhe(4-Me)-Sar-OtBu in MeCN (concentration 200.0 mg/mL, 0.175 mL, 35.0 mg, 0.069 mmol), and DIPEA (24.3 μL, 0.139 mmol) were sequentially added. The reaction mixture was cooled to 10°C with stirring, HATU (52.8 mg, 0.139 mmol) was added and the mixture was stirred for 5 hours.

Conversion rate: 99.7%

Diastereomer ratio: 90.5/9.5

(Example 3) Condensation reaction of Cbz-MeLeu-MePhe(4-F)-OH and H-MeLeu-Ile-MeAla-Aze-EtPhe(4-Me)-Sar-OtBu

[Formula 5]

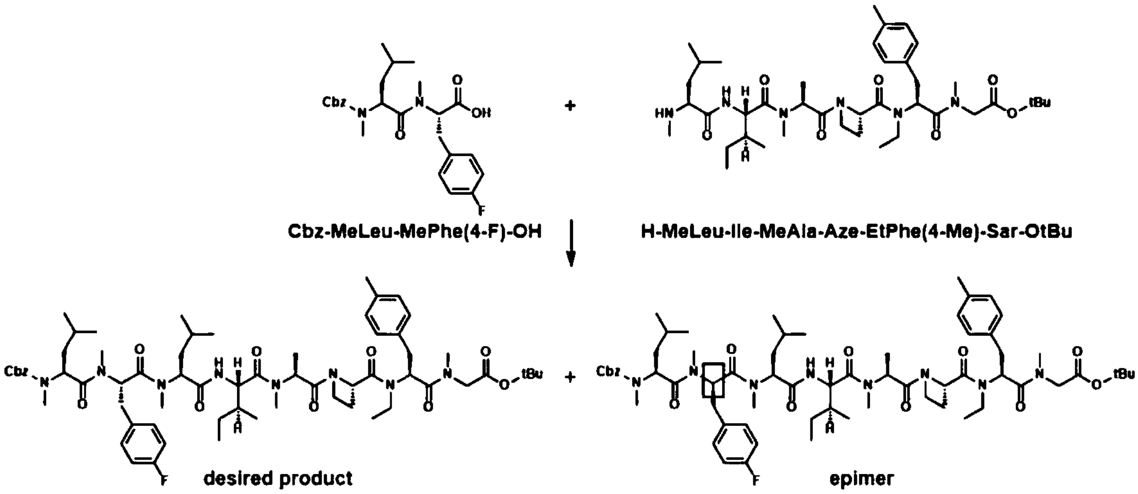


Under each condition shown in Table 3, to a test tube with a stirrer, additive-1, additive-2, a solution of Cbz-MeLeu-MePhe(4-F)-OH in 2-MeTHF (concentration 129.0 mg/mL, 0.260 mL, 33.5 mg, 0.073 mmol, 1.2 equiv.), a solution of H-MeLeu-Ile-MeAla-Aze-EtPhe(4-Me)-Sar-OtBu in 2-MeTHF (concentration 150.0 mg/mL, 0.300 mL, 45.0 mg, 0.061 mmol, 1.0 equiv.), and DIPEA (73.8 μL, 0.424 mmol, 7.0 equiv.) were sequentially added. The reaction mixture was cooled to 10°C with stirring. To the reaction mixture, DPPC (25.0 μL, 0.121 mmol, 2.0 equiv.) was added, and the mixture was stirred for 10 hours. The reaction mixture was analyzed by HPLC, and the conversion rate, the diastereomer ratio, and the byproduct amount were evaluated.

Measurement method: HPLC Method A
Retention time: H-MeLeu-Ile-MeAla-Aze-EtPhe(4-Me)-Sar-OtBu: 10.3 min, desired product: 18.9 min, epimer: 19.1 min
Mass spectrometry: H-MeLeu-Ile-MeAla-Aze-EtPhe(4-Me)-Sar-OtBu: m/z 744.07 ($[M+H]^+$), desired product: m/z 1206.23 ($[M+Na]^+$), epimer: m/z 1206.35 ($[M+Na]^+$)

[Table 3]

| Reaction conditions | | | Analysis results | | |
|---|---|---|---|---|---|
| Run | Additive-1 | Additive-2 | Conversion rate (%) | Diastereomer ratio | Byproduct amount |
| 1 | HOPO (2.0 eq.) | OxymaPure (1.0 eq.) | 98.3 | 99.8 / 0.2 | negligible |
| 2 | HOPO (3.0 eq.) | none | 96.4 | 96.8 / 3.2 | negligible |
| 3 | none | OxymaPure (3.0 eq.) | 80.9 | 96.0 / 4.0 | many |
| 4 | none | none | 88.2 | 47.2 / 52.8 | many |

As shown in Table 3, it was found that the highest conversion rate, diastereomer ratio, and purity of the target were obtained from the conditions of adding both HOPO and OxymaPure.

Comparative experiments with HATU

To a vial with a stirrer, a solution of Cbz-MeLeu-MePhe(4-F)-OH in 2-MeTHF (concentration 129.0 mg/mL, 0.260

mL, 33.5 mg, 0.073 mmol), a solution of H-MeLeu-Ile-MeAla-Aze-EtPhe(4-Me)-Sar-OtBu in MeCN (concentration 170.0 mg/mL, 0.265 mL, 45.1 mg, 0.061 mmol), and DIPEA (21.1 μL, 0.121 mmol) were sequentially added. The reaction mixture was cooled to 10°C, HATU (46.5 mg, 0.121 mmol) was added and the mixture was stirred for 5 hours.

Conversion rate: 99.6%
Diastereomer ratio: 79.5/20.5

(Example 4) Condensation reaction of Cbz-MeLeu-MePhe-OH and H-MeLeu-Ile-MeAla-Aze-EtPlie(4-Me)-Sar-OtBu

[Formula 6]

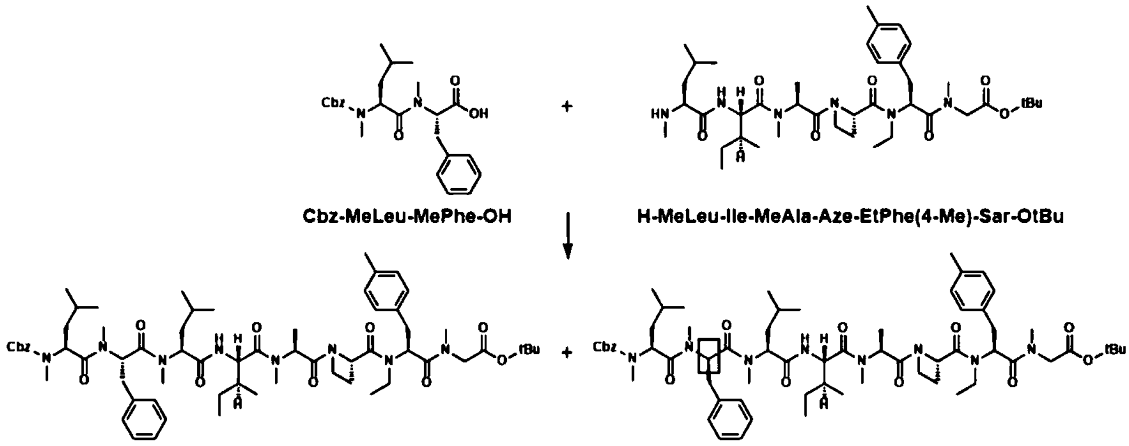

Cbz-MeLeu-MePhe-OH

H-MeLeu-Ile-MeAla-Aze-EtPhe(4-Me)-Sar-OtBu

desired product

epimer

Under each condition shown in Table 4, to a test tube with a stirrer, additive-1, additive-2, a solution of Cbz-MeLeu-MePhe-OH in 2-MeTHF (concentration 169.5 mg/mL, 0.210 mL, 35.6 mg, 0.081 mmol, 1.2 equiv.), a solution of H-MeLeu-Ile-MeAla-Aze-EtPhe(4-Me)-Sar-OtBu in 2-MeTHF (concentration 170.0 mg/mL, 0.295 mL, 50.2 mg, 0.067 mmol, 1.0 equiv.), and DIPEA (82.1 μL, 0.471 mmol, 7.0 equiv.) were sequentially added. The reaction mixture was cooled to 10°C with stirring. To the reaction mixture, DPPC (27.8 μL, 0.135 mmol, 2.0 equiv.) was added, and the mixture was stirred for 10 hours. The reaction mixture was analyzed by HPLC, and the conversion rate, the diastereomer ratio, and the byproduct amount were evaluated.

Measurement method: HPLC Method A
Retention time: H-MeLeu-Ile-MeAla-Aze-EtPhe(4-Me)-Sar-OtBu: 10.1 min, desired product: 18.7 min, epimer: 19.0 min
Mass spectrometry: H-MeLeu-Ile-MeAla-Aze-EtPhe(4-Me)-Sar-OtBu: m/z 744.12 ([M+H]$^+$), desired product: m/z 1188.73 ([M+Na]$^+$), epimer: m/z 1188.33 ([M+Na]$^+$)

[Table 4]

| Reaction conditions | | | Analysis results | | |
|---|---|---|---|---|---|
| Run | Additive-1 | Additive-2 | Conversion rate (%) | Diastereomer ratio | Byproduct amount |
| 1 | HOPO (2.0 eq.) | OxymaPure (1.0 eq.) | 97.1 | 99.7 / 0.3 | negligible |
| 2 | HOPO (2.0 eq.) | none | 96.4 | 89.4 / 10.6 | negligible |
| 3 | none | OxymaPure (3.0 eq.) | 75.0 | 96.3 / 3.7 | many |

As shown in Table 4, it was found that the highest conversion rate, diastereomer ratio, and purity of the target were obtained from the conditions of adding both HOPO and OxymaPure.

Comparative experiments with HATU

A solution of Cbz-MeLeu-MePhe-OH in 2-MeTHF (concentration 169.5 mg/mL, 0.210 mL, 35.6 mg, 0.081 mmol), a solution of H-MeLeu-Ile-MeAla-Aze-EtPhe(4-Me)-Sar-OtBu in MeCN (concentration 170.0 mg/mL, 0.295 mL, 50.2 mg, 0.067 mmol), and DIPEA (23.4 μL, 0.135 mmol) were sequentially added. The reaction mixture was cooled to 10°C, HATU (50.8 mg, 0.134 mmol) was added and the mixture was stirred for 5 hours.

Conversion rate: 99.5%
Diastereomer ratio: 76.5/23.5

(Example 5) Condensation reaction of Cbz-MeLeu-MePhe-OH and H-MeGly(cPent)-MeAsp(OtBu)-$NMe_2$

[Formula 7]

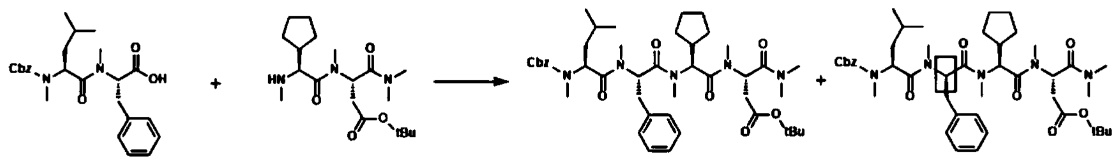

Cbz-MeLeu-MePhe-OH H-MeGly(cPent)-MeAsp(OtBu)-$NMe_2$ desired product epimer

Under each condition shown in Table 5, to a test tube with a stirrer, additive-1, additive-2, a solution of Cbz-MeLeu-MePhe-OH in 2-MeTHF (concentration 169.5 mg/mL, 0.210 mL, 35.6 mg, 0.081 mmol, 1.2 equiv.), a solution of H-MeGly(cPent)-MeAsp(OtBu)-$NMe_2$ in 2-MeTHF (concentration 129.6 mg/mL, 0.210 mL, 27.2 mg, 0.068 mmol, 1.0 equiv.), and DIPEA (82.6 μL, 0.474 mmol, 7.0 equiv.) were sequentially added. The reaction mixture was cooled to 10°C with stirring. To the reaction mixture, DPPC (28.0 μL, 0.135 mmol, 2.0 equiv.) was added, and the mixture was stirred for 10 hours. The reaction mixture was analyzed by HPLC, and the conversion rate, the diastereomer ratio, and the byproduct amount were evaluated.

Measurement method: HPLC Method A
Retention time: H-MeGly(cPent)-MeAsp(OtBu)-$NMe_2$: 5.7 min, desired product: 17.8 min, epimer: 18.2 min
Mass spectrometry: H-MeGly(cPent)-MeAsp(OtBu)-$NMe_2$: m/z 370.82 ($[M+H]^+$), desired product: m/z 748.28 ($[M-NMe_2]^+$), epimer: m/z 748.06 ($[M-NMe_2]^+$)

[Table 5]

| Reaction conditions | | | Analysis results | | |
|---|---|---|---|---|---|
| Run | Additive-1 | Additive-2 | Conversion rate (%) | Diastereomer ratio | Byproduct amount |
| 1 | HOPO (2.0 eq.) | OxymaPure (1.0 eq.) | 92.2*1 | 99.6 / 0.4*1 | negligible |
| 2 | HOPO (2.0 eq.) | none | 92.1 | 91.6 / 8.4 | negligible |
| 3 | none | OxymaPure (3.0 eq.) | 94.3 | 99.7 / 0.3 | many |
| *1 Conversion rate 96.5%, diastereomer ratio 99.6/0.4 at 23 hours | | | | | |

As shown in Table 5, it was found that the highest conversion rate, diastereomer ratio, and purity of the target were obtained from the conditions of adding both HOPO and OxymaPure.

Comparative experiments with HATU

To a vial with a stirrer, HATU (78.0 mg, 0.206 mmol), a solution of Cbz-MeLeu-MePhe-OH in IPAC (concentration 160.0 mg/mL, 0.300 mL, 48.0 mg, 0.109 mmol), a solution of H-MeGly(cPent)-MeAsp(OtBu)-$NMe_2$ in MeCN (concentration 75.7 mg/mL, 0.500 mL, 37.9 mg, 0.103 mmol), and DIPEA (90.0 μL, 0.514 mmol) were sequentially added, and the

mixture was stirred for 3.5 hours.

Conversion rate: 100.0%
Diastereomer ratio: 35.5/64.5

(Example 6) Condensation reaction of Cbz-MeLeu-MeAla-OH and H-MeAla-Aze-EtPhe(4-Me)-Sar-OtBu

[Formula 8]

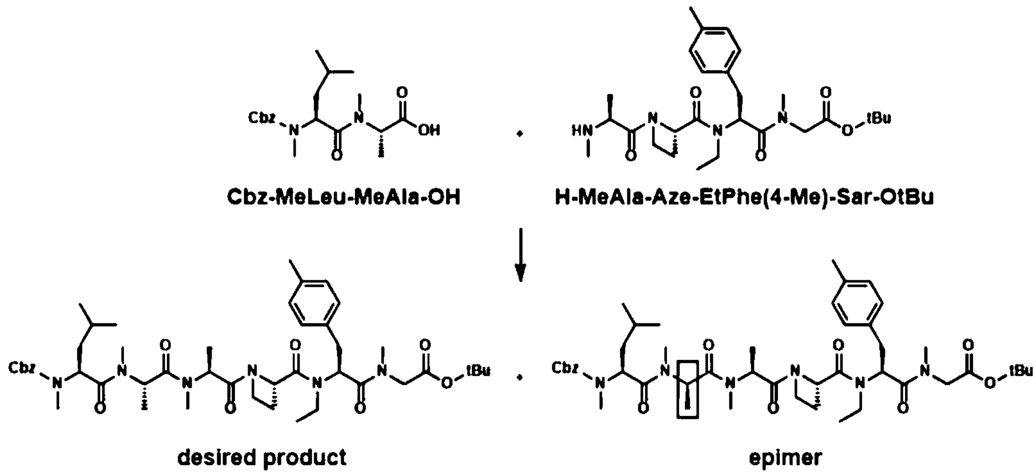

Cbz-MeLeu-MeAla-OH + H-MeAla-Aze-EtPhe(4-Me)-Sar-OtBu

desired product + epimer

To a test tube with a stirrer, Cbz-MeLeu-MeAla-OH (23.9 mg, 0.066 mmol, 1.1 equiv.), HOPO (13.3 mg, 0.119 mmol, 2.0 equiv.), OxymaPure (8.5 mg, 0.060 mmol, 1.0 equiv.), H-MeAla-Aze-EtPhe(4-Me)-Sar-OtBu (30.0 mg, 0.060 mmol, 1.0 equiv.), 2-MeTHF (0.300 mL), and DIPEA (72.8 μL, 0.418 mmol, 7.0 equiv.) were sequentially added. The reaction mixture was cooled to 10°C with stirring. To the reaction mixture, DEPC (17.2 μL, 0.119 mmol, 2.0 equiv.) was added, and the mixture was stirred for 30 hours.

Conversion rate: 98.4%
Diastereomer ratio: 99.2/0.8
Epimer synthesis: An epimer was synthesized according to the condition above except for using Cbz-MeLeu-D-MeAla-OH instead of Cbz-MeLeu-MeAla-OH.
Measurement method: HPLC Method A
Retention time: H-MeAla-Aze-EtPhe(4-Me)-Sar-OtBu: 8.0 min, desired product: 15.6 min, epimer: 15.9 min
Mass spectrometry: H-MeAla-Aze-EtPhe(4-Me)-Sar-OtBu: m/z 503.36 ($[M+H]^+$), desired product: m/z 849.69 ($[M+H]^+$), epimer: m/z 849.50 ($[M+H]^+$)

(Example 7) Condensation reaction of Cbz-MeLeu-MeAla-OH and H-MeLeu-Ile-MeAla-Aze-EtPhe(4-Me)-Sar-OtBu

[Formula 9]

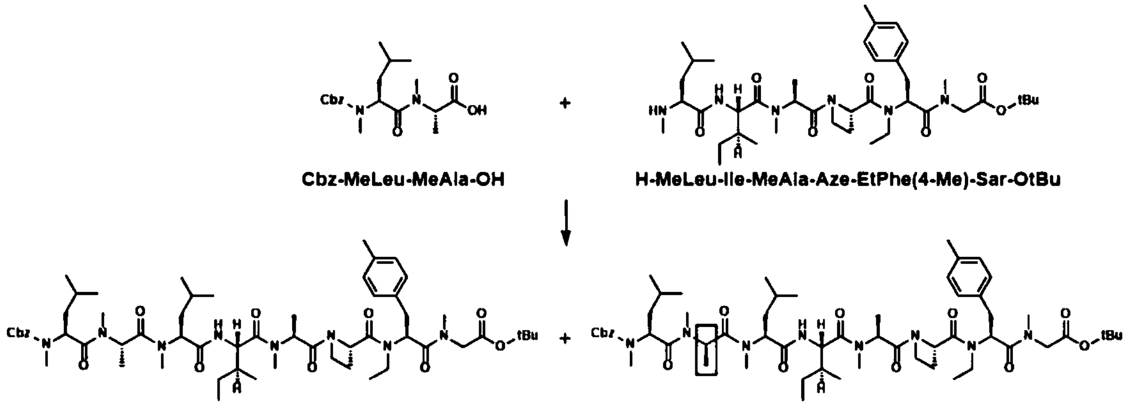

Cbz-MeLeu-MeAla-OH

H-MeLeu-Ile-MeAla-Aze-EtPhe(4-Me)-Sar-OtBu

desired product

epimer

To a test tube with a stirrer, Cbz-MeLeu-MeAla-OH (27.0 mg, 0.074 mmol, 1.1 equiv.), HOPO (15.0 mg, 0.135 mmol, 2.0 equiv.), OxymaPure (9.6 mg, 0.067 mmol, 1.0 equiv.), H-MeLeu-Ile-MeAla-Aze-EtPhe(4-Me)-Sar-OtBu (50.0 mg, 0.067 mmol, 1.0 equiv.), 2-MeTHF (0.300 mL), and DIPEA (81.1 μL, 0.471 mmol, 7.0 equiv.) were sequentially added. The reaction mixture was cooled to 10°C with stirring. To the reaction mixture, DPPC (27.9 μL, 0.135 mmol, 2.0 equiv.) was added, and the mixture was stirred for 30 hours.

Conversion rate: 99.0%
Diastereomer ratio: 99.5/0.5
Epimer synthesis: An epimer was synthesized according to the condition above except for using Cbz-MeLeu-D-MeAla-OH instead of Cbz-MeLeu-MeAla-OH.
Measurement method: HPLC Method A
Retention time: H-MeLeu-Ile-MeAla-Aze-EtPhe(4-Me)-Sar-OtBu: 9.9 min, desired product: 17.4 min, epimer: 17.7 min
Mass spectrometry: H-MeLeu-Ile-MeAla-Aze-EtPhe(4-Me)-Sar-OtBu: m/z 743.63 ($[M+H]^+$), desired product: m/z 1089.78 ($[M+H]^+$), epimer: m/z 1089.95 ($[M+H]^+$)

(Example 8) Condensation reaction of Cbz-MeLeu-MeAla-OH and H-MeGly(cPent)-MeAsp(OtBu)-$NMe_2$

[Formula 10]

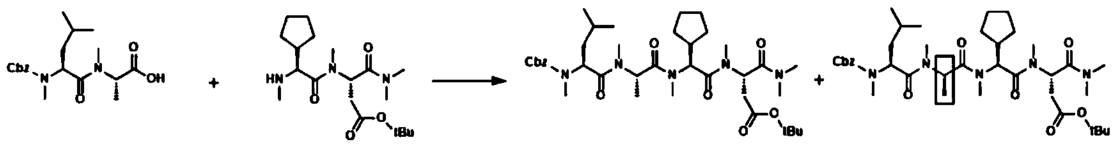

Cbz-MeLeu-MeAla-OH H-MeGly(cPent)-MeAsp(OtBu)-$NMe_2$ desired product epimer

To a test tube with a stirrer, Cbz-MeLeu-MeAla-OH (27.1 mg, 0.074 mmol, 1.1 equiv.), HOPO (15.0 mg, 0.135 mmol, 2.0 equiv.), OxymaPure (9.6 mg, 0.068 mmol, 1.0 equiv.), 2-MeTHF (0.152 mL), a solution of H-MeGly(c-Pent)-MeAsp(OtBu)-$NMe_2$ in 2-MeTHF (concentration 170.0 mg/mL, 0.147 mL, 25.0 mg, 0.068 mmol, 1.0 equiv.), and DIPEA (81.5 μL, 0.474 mmol, 7.0 equiv.) were sequentially added. The reaction mixture was cooled to 10°C with stirring. To the reaction mixture, DEPC (19.6 μL, 0.068 mmol, 2.0 equiv.) was added, and the mixture was stirred for 30 hours.

Conversion rate: 96.6%
Diastereomer ratio: 99.5/0.5
Epimer synthesis: An epimer was synthesized according to the condition above except for using Cbz-MeLeu-D-MeAla-OH instead of Cbz-MeLeu-MeAla-OH.
Measurement method: HPLC Method A
Retention time: H-MeGly(cPent)-MeAsp(OtBu)-$NMe_2$: 5.5 min, desired product: 16.1 min, epimer: 16.3 min

Mass spectrometry: H-MeGly(cPent)-MeAsp(OtBu)-$NMe_2$: m/z 370.31 ($[M+H]^+$), desired product: m/z 738.53 ($[M+Na]^+$), epimer: m/z 738.35 ($[M+Na]^+$)

(Example 9) Evaluation of additive-1

[Formula 11]

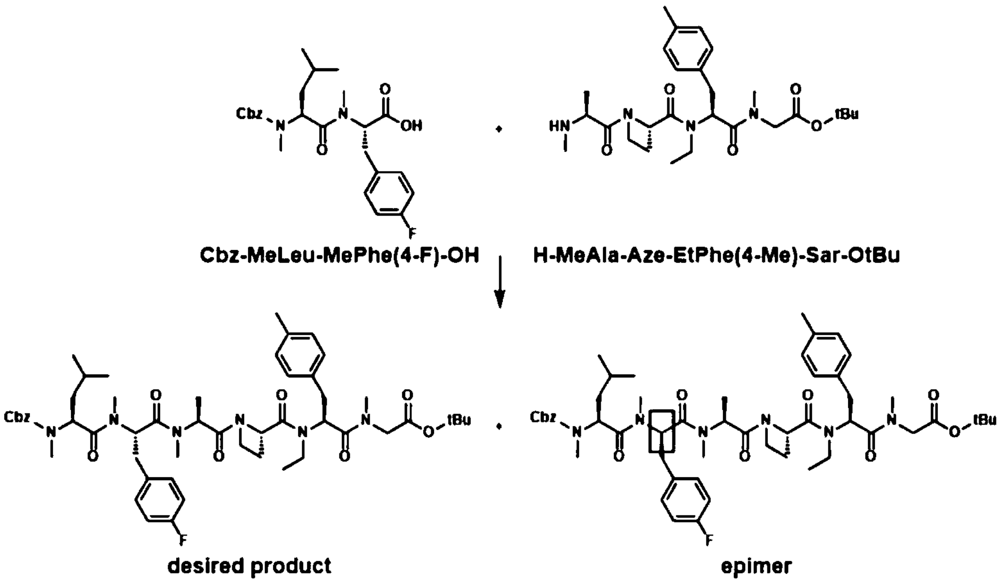


Under each condition shown in Table 6, to a test tube with a stirrer, additive-1 (0.139 mmol, 2.0 equiv.), OxymaPure (9.90 mg, 0.070 mmol, 1.0 equiv.), a solution of Cbz-MeLeu-MePhe(4-F)-OH in 2-MeTHF (concentration 129.0 mg/mL, 0.300 mL, 38.7 mg, 0.084 mmol, 1.2 equiv.), a solution of H-MeAla-Aze-EtPhe(4-Me)-Sar-OtBu in 2-MeTHF (concentration 142.9 mg/mL, 0.245 mL, 35.0 mg, 0.070 mmol, 1.0 equiv.), and DIPEA (85.0 μL, 0.487 mmol, 7.0 equiv.) were sequentially added. The reaction mixture was cooled to 10°C with stirring. To the reaction mixture, DPPC (28.8 μL, 0.139 mmol, 2.0 equiv.) was added, and the mixture was stirred for 3 hours. The reaction mixture was analyzed by HPLC, and the conversion rate, the diastereomer ratio, and the byproduct amount were evaluated.

Measurement method: HPLC Method A
Retention time: H-MeAla-Aze-EtPhe(4-Me)-Sar-OtBu: 8.5 min, desired product: 17.6 min, epimer: 17.9 min
Mass spectrometry: H-MeAla-Aze-EtPhe(4-Me)-Sar-OtBu: m/z 503.98 ($[M+H]^+$), desired product: m/z 944.14 ($[M+H]^+$), epimer: m/z 944.14 ($[M+H]^+$)

[Table 6]

| Reaction conditions | | Analysis results | | |
|---|---|---|---|---|
| Run | Additive-1 | Conversion rate (%) | Diastereomer ratio | Byproduct amount |
| 1 | HOPO | >99 | 99.7 / 0.3 | negligible |
| 2 | HOSu | 13.1 | 98.0 / 2.0 | - |
| 3 | HONB | 11.1 | 100.0 / 0.0 | - |
| 4 | HOPht | 61.7 | 88.8 / 11.2 | many |
| 5 | HOOBt | >99 | 99.6 / 0.4 | negligible |
| 6 | Oxyma-B | >99 | 99.9 / 0.1 | negligible |
| 7 | 4-nitrophenol | 78.4 | 98.8 / 1.2 | - |
| 8 | pentafluorophenol | >99 | 99.0 / 1.0 | negligible |

(continued)

| Reaction conditions | | Analysis results | | |
|---|---|---|---|---|
| Run | Additive-1 | Conversion rate (%) | Diastereomer ratio | Byproduct amount |
| 9 | 4,6-dimethoxy-1,3,5-triazin-2-ol | 96.6 | 98.1 / 1.9 | many |
| 10 | 2-hydroxypyridine | 93.5 | 98.4 / 1.6 | many |
| 11 | 2-hydroxy-5-nitropyridine | 99.4 | 97.8 / 2.2 | many |
| 12 | trimethylsilanol | 91.8 | 98.7 / 1.3 | many |

As shown in Table 6, HOOBt, Oxyma-B, and pentafluorophenol gave the results similar to that of HOPO.

(Example 10) Evaluation of additive-2

[Formula 12]

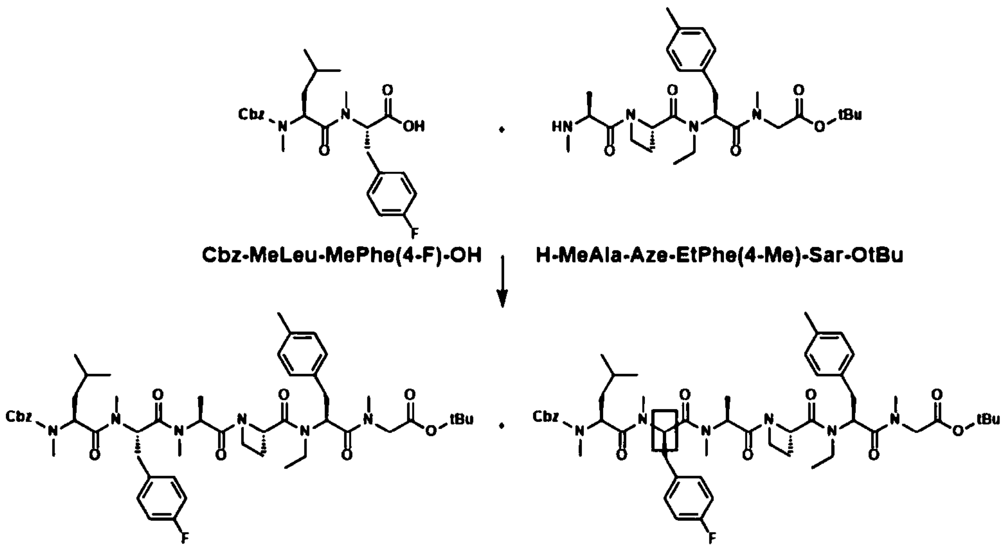

Cbz-MeLeu-MePhe(4-F)-OH

H-MeAla-Aze-EtPhe(4-Me)-Sar-OtBu

desired product

epimer

Under each condition shown in Table 7, to a test tube with a stirrer, HOPO (15.5 mg, 0.139 mmol, 2.0 equiv.), additive-2 (0.070 mmol, 1.0 equiv.), a solution of Cbz-MeLeu-MePhe(4-F)-OH in 2-MeTHF (concentration 129.0 mg/mL, 0.300 mL, 38.7 mg, 0.084 mmol, 1.2 equiv.), a solution of H-MeAla-Aze-EtPhe(4-Me)-Sar-OtBu in 2-MeTHF (concentration 142.9 mg/mL, 0.245 mL, 35.0 mg, 0.070 mmol, 1.0 equiv.), and DIPEA (85.0 μL, 0.487 mmol, 7.0 equiv.) were sequentially added. The reaction mixture was cooled to 10°C with stirring. To the reaction mixture, DPPC (28.8 μL, 0.139 mmol, 2.0 equiv.) was added, and the mixture was stirred for 3 hours. The reaction mixture was analyzed by HPLC, and the conversion rate, the diastereomer ratio, and the byproduct amount were evaluated.

Measurement method: HPLC Method A
Retention time: H-MeAla-Aze-EtPhe(4-Me)-Sar-OtBu: 8.4 min, desired product: 17.5 min, epimer: 17.8 min
Mass spectrometry: H-MeAla-Aze-EtPhe(4-Me)-Sar-OtBu: m/z 503.98 ([M+H]$^+$), desired product: m/z 944.14 ([M+H]$^+$), epimer: m/z 944.14 ([M+H]$^+$)

[Table 7]

| Reaction conditions | | Analysis results | | |
|---|---|---|---|---|
| Run | Additive-2 | Conversion rate (%) | Diastereomer ratio | Byproduct amount |
| 1 | OxymaPure | 99.7 | 99.7 / 0.3 | negligible |
| 2 | HOAt | 99.5 | 99.2 / 0.8 | negligible |
| 3 | HOOBt | 98.7 | 98.6 / 1.4 | negligible |
| 4 | Oxyma-B | 98.3 | 99.9 / 0.1 | negligible |
| 5 | K-Oxyma | 99.7 | 99.4 / 0.6 | negligible |

As shown in Table 7, HOAt, Oxyma-B, and K-Oxyma gave the results similar to that of OxymaPure.

(Example 11) Evaluation of condensing agent other than DPPC and DEPC

[Formula 13]

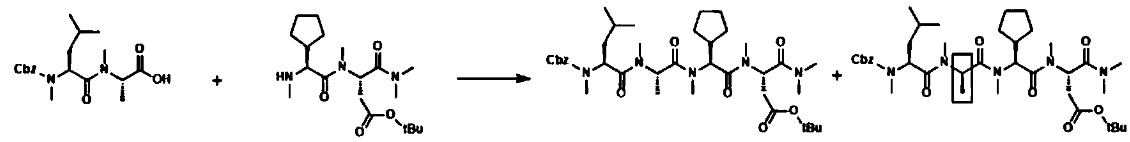

Cbz-MeLeu-MeAla-OH H-MeGly(cPent)-MeAsp(OtBu)-$NMe_2$ desired product epimer

Under each condition shown in Table 8, to a test tube with a stirrer, Cbz-MeLeu-MeAla-OH (27.1 mg, 0.074 mmol, 1.1 equiv.), HOPO (15.0 mg, 0.135 mmol, 2.0 equiv.), OxymaPure (9.6 mg, 0.068 mmol, 1.0 equiv.), 2-MeTHF (0.152 mL), a solution of H-MeGly(cPent)-MeAsp(OtBu)-$NMe_2$ in 2-MeTHF (concentration 170.0 mg/mL, 0.147 mL, 25.0 mg, 0.068 mmol, 1.0 equiv.), and DIPEA (81.5 $\mu$L, 0.474 mmol, 7.0 equiv.) were sequentially added. The reaction mixture was cooled to 10°C with stirring. To the reaction mixture, a condensing agent (0.135 mmol, 2.0 equiv.) was added, and the mixture was stirred for 5 hours. The reaction mixture was analyzed by HPLC, and the conversion rate, the diastereomer ratio, and the byproduct amount were evaluated.

Measurement method: HPLC Method A
Retention time: H-MeGly(cPent)-MeAsp(OtBu)-$NMe_2$: 5.5 min, desired product: 16.1 min, epimer: 16.3 min
Mass spectrometry: H-MeGly(cPent)-MeAsp(OtBu)-$NMe_2$: m/z 370.31 ($[M+H]^+$), desired product: m/z 738.54 ($[M+Na]^+$), epimer: m/z 738.41 ($[M+Na]^+$)

[Table 8]

| Reaction conditions | | Analysis results | | |
|---|---|---|---|---|
| Run | Condensing agent | Conversion rate (%) | Diastereomer ratio | Byproduct amount |
| 1 | DPPC | 78.2 | 99.6 / 0.4 | negligible |
| 2 | DPPA | 80.1 | 99.5 / 0.5 | negligible |
| 3 | FDPP | 81.1 | 99.7 / 0.3 | negligible |
| 4 | Ph2P(O)Cl | 86.5 | 99.6 / 0.4 | negligible |
| 5 | TCFH | 84.8 | 99.0 / 1.0 | many |
| 6 | MNBA | 1.5 | - | - |
| 7 | CDI | 14.0 | - | - |
| 8[*1] | T3P | 59.3 | 99.4 / 0.6 | - |

(continued)

| Reaction conditions | | Analysis results | | |
|---|---|---|---|---|
| Run | Condensing agent | Conversion rate (%) | Diastereomer ratio | Byproduct amount |
| 9*1 | p-TsCl | 14.1 | - | - |
| 10 | DIC | 14.7 | - | - |
| 11 | EDC·HCl | 9.3 | - | - |
| *1 OxymaPure 2.0 equiv. | | | | |

As shown in Table 8, DPPA, FDPP, Ph2P(O)Cl gave the results similar to that of DPPC. Other condensing agents gave significantly lower results in any of the conversion rate, the diastereomer ratio, and the byproduct amount.

(Example 12) Evaluation of base

[Formula 14]

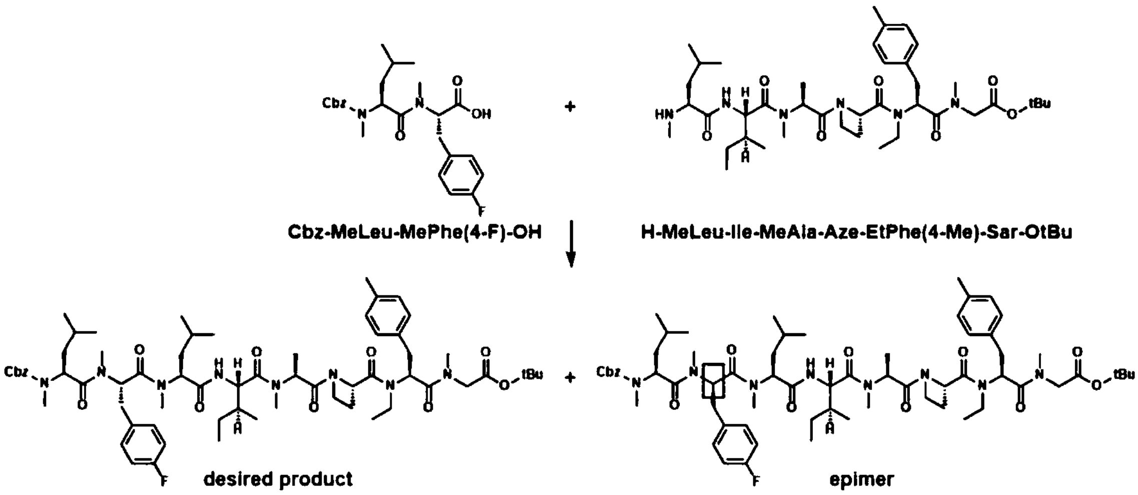

Cbz-MeLeu-MePhe(4-F)-OH

H-MeLeu-Ile-MeAla-Aze-EtPhe(4-Me)-Sar-OtBu

desired product

epimer

Under each condition shown in Table 9, to a test tube with a stirrer, HOPO (13.5 mg, 0.121 mmol, 2.0 equiv.), OxymaPure (8.6 mg, 0.061 mmol, 1.0 equiv.), a solution of Cbz-MeLeu-MePhe(4-F)-OH in 2-MeTHF (concentration 129.0 mg/mL, 0.260 mL, 33.5 mg, 0.073 mmol, 1.2 equiv.), a solution of H-MeLeu-Ile-MeAla-Aze-EtPhe(4-Me)-Sar-OtBu in 2-MeTHF (concentration 150.0 mg/mL, 0.300 mL, 45.0 mg, 0.061 mmol, 1.0 equiv.), and a base (0.424 mmol, 7.0 equiv.) were sequentially added. The reaction mixture was cooled to 10°C with stirring. To the reaction mixture, DPPC (25.0 μL, 0.121 mmol, 2.0 equiv.) was added, and the mixture was stirred for 10 hours. The reaction mixture was analyzed by HPLC, and the conversion rate, the diastereomer ratio, and the byproduct amount were evaluated.

Measurement method: HPLC Method A
Retention time: H-MeLeu-Ile-MeAla-Aze-EtPhe(4-Me)-Sar-OtBu: 10.3 min, desired product: 18.9 min, epimer: 19.1 min
Mass spectrometry: H-MeLeu-Ile-MeAla-Aze-EtPhe(4-Me)-Sar-OtBu: m/z 744.07 ($[M+H]^+$), desired product: m/z 1206.23 ($[M+Na]^+$), epimer: m/z 1206.35 ($[M+Na]^+$)

[Table 9]

| Reaction conditions | | Analysis results | | |
|---|---|---|---|---|
| Run | Base | Conversion rate (%) | Diastereomer ratio | Byproduct amount |
| 1 | DIPEA | 98.3 | 99.8 / 0.2 | negligible |

(continued)

| Reaction conditions | | Analysis results | | |
|---|---|---|---|---|
| Run | Base | Conversion rate (%) | Diastereomer ratio | Byproduct amount |
| 2 | TEA | 99.3 | 99.9 / 0.1 | negligible |
| 3 | 1-methylpiperidine | 99.3 | 99.9 / 0.1 | negligible |
| 4 | NMM | 79.1 | 99.7 / 0.3 | negligible |
| 5 | 2,6-lutidine | 20.8 | - | - |
| 6 | pyridine | 10.0 | - | - |

As shown in Table 9, TEA and 1-methylpiperidine gave comparable results to that of DIPEA.

(Example 13) Condensation reaction of Cbz-Gly-MePhe-OH and H-MePhe-MeAsp(OtBu)-$NMe_2$

[Formula 15]

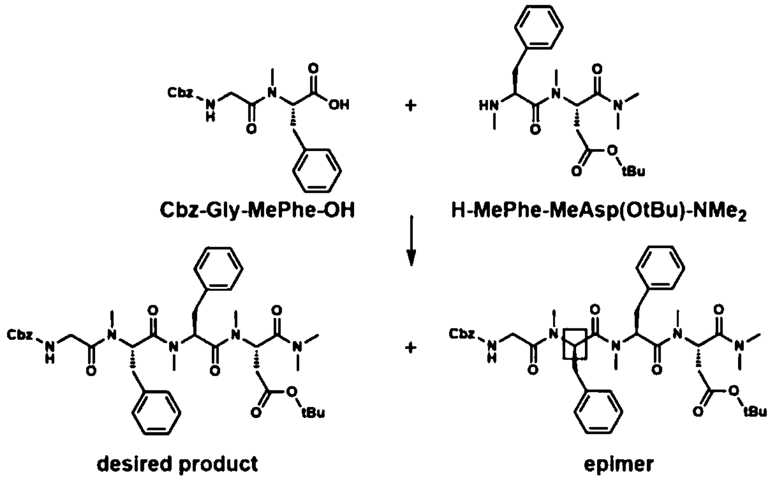


Experiment with DPPC, HOPO, and OxymaPure

To a test tube with a stirrer, OxymaPure (10.5 mg, 0.074 mmol), HOPO (16.4 mg, 0.148 mmol), H-MePhe-MeAsp(OtBu)-$NMe_2$ (30.0 mg, 0.074 mmol), 2-MeTHF (0.300 mL), a solution of Cbz-Gly-MePhe-OH in 2-MeTHF/DMI (concentration 182.6 mg/mL, 0.170 mL, 31.0 mg, 0.088 mmol), and $Cy_2NMe$ (110.0 μL, 0.518 mmol) were sequentially added. The reaction mixture was cooled to -5°C with stirring. To the reaction mixture, DPPC (30.6 μL, 0.148 mmol) was added, and the temperature was raised to 10°C and the mixture was stirred for 12 hours. The reaction mixture was analyzed by HPLC, and the reaction conversion rate and the diastereomer ratio were evaluated.

Comparative Experiment with HATU

To a test tube with a stirrer, H-MePhe-MeAsp(OtBu)-$NMe_2$ (30.0 mg, 0.074 mmol), MeCN (0.300 mL), a solution of Cbz-Gly-MePhe-OH in 2-MeTHF/DMI (concentration 182.6 mg/mL, 0.170 mL, 31.0 mg, 0.088 mmol), and DIPEA (25.8 μL, 0.148 mmol) were sequentially added. The reaction mixture was cooled to 10°C with stirring, HATU (56.3 mg, 0.148 mmol) was added, and the mixture was stirred for 12 hours. The reaction mixture was analyzed by HPLC, and the reaction conversion rate and the diastereomer ratio were evaluated.

Measurement method: HPLC Method A
Retention time: H-MePhe-MeAsp(OtBu)-$NMe_2$: 7.2 min, desired product: 15.6 min, epimer: 16.1 min
Mass spectrometry: H-MePhe-MeAsp(OtBu)-$NMe_2$: m/z 392.77 ($[M+H]^+$), desired product: m/z 700.00 ($[M-NMe_2]^+$), epimer: m/z 699.95 ($[M-NMe_2]^+$)

[Table 10]

| Reaction conditions | | Analysis results | |
|---|---|---|---|
| | Condensation condition | Reaction conversion rate (%) | Diastereomer ratio |
| Condition A | DPPC, HOPO, OxymaPure | 99.5 | 99.7 / 0.3 |
| Condition B | HATU | 96.8 | 70.3 / 29.7 |

As shown in Table 10, it was found that condition A with DPPC, HOPO, and OxymaPure gave a higher diastereomer ratio compared to condition B with HATU as the condensing agent.

(Example 14) Condensation reaction of Cbz-MeAla-MeLeu-OH and H-MeLeu-Ile-MeAla-Aze-EtPhe(4-Me)-Sar-OtBu

[Formula 16]

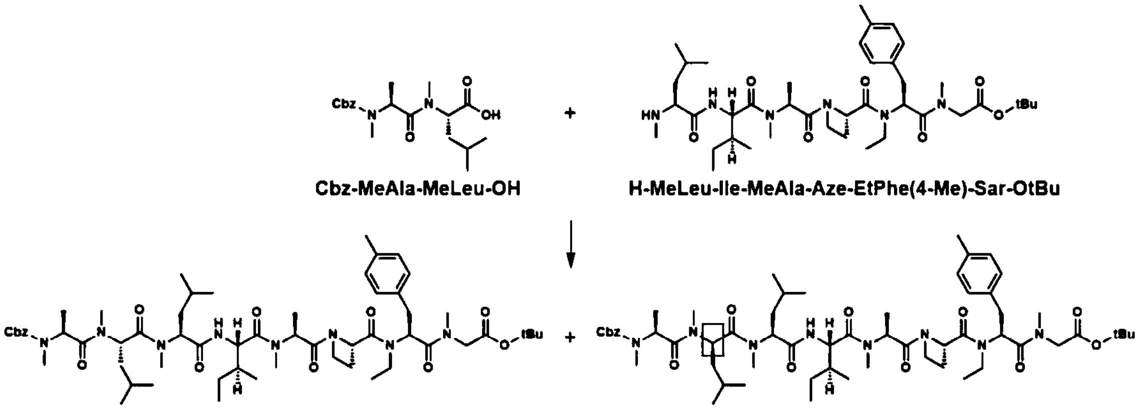

Cbz-MeAla-MeLeu-OH

H-MeLeu-Ile-MeAla-Aze-EtPhe(4-Me)-Sar-OtBu

desired product

epimer

Experiment with DPPC, HOPO, and OxymaPure

To a test tube with a stirrer, OxymaPure (8.6 mg, 0.061 mmol), HOPO (13.5 mg, 0.121 mmol), a solution of Cbz-MeAla-MeLeu-OH in 2-MeTHF (concentration 170.0 mg/mL, 0.160 mL, 27.0 mg, 0.073 mmol), a solution of H-MeLeu-Ile-MeAla-Aze-EtPhe(4-Me)-Sar-OtBu in 2-MeTHF (concentration 150.0 mg/mL, 0.300 mL, 45.0 mg, 0.061 mmol), and $Cy_2NMe$ (90.0 μL, 0.424 mmol) were sequentially added. The reaction mixture was cooled to -5°C with stirring. To the reaction mixture, DPPC (25.0 μL, 0.121 mmol) was added, and the temperature was raised to 10°C and the mixture was stirred for 11 hours. The reaction mixture was analyzed by HPLC, and the reaction conversion rate and the diastereomer ratio were evaluated.

Comparative Experiment with HATU

To a test tube with a stirrer, a solution of Cbz-MeAla-MeLeu-OH in 2-MeTHF (concentration 170.0 mg/mL, 0.160 mL, 27.0 mg, 0.073 mmol), a solution of H-MeLeu-Ile-MeAla-Aze-EtPhe(4-Me)-Sar-OtBu in MeCN (concentration 170.0 mg/mL, 0.270 mL, 45.9 mg, 0.061 mmol), and DIPEA (21.1 μL, 0.121 mmol) were sequentially added. The reaction mixture was cooled to 10°C with stirring, HATU (46.1 mg, 0.121 mmol) was added, and the mixture was stirred for 5 hours. The reaction mixture was analyzed by HPLC, and the reaction conversion rate and the diastereomer ratio were evaluated.

Measurement method: HPLC Method A
Retention time: H-MeLeu-Ile-MeAla-Aze-EtPhe(4-Me)-Sar-OtBu: 11.1 min, desired product: 18.7 min, epimer: 19.0 min
Mass spectrometry: H-MeLeu-Ile-MeAla-Aze-EtPhe(4-Me)-Sar-OtBu: m/z 744.18 ($[M+H]^+$), desired product: m/z 945.34 ($[M\text{-}Sar\text{-}OtBu]^+$), epimer: m/z 945.28 ($[M\text{-}Sar\text{-}OtBu]^+$)

[Table 11]

| Reaction conditions | | Analysis results | |
|---|---|---|---|
| | Condensation condition | Reaction conversion rate (%) | Diastereomer ratio |
| Condition A | DPPC, HOPO, OxymaPure | 97.0 | 100.0 / 0.0 |
| Condition B | HATU | 98.7 | 62.7 / 37.3 |

As shown in Table 11, it was found that condition A with DPPC, HOPO, and OxymaPure gave a higher diastereomer ratio compared to condition B with HATU as the condensing agent.

(Example 15) Condensation reaction of Cbz-MeAla-MeLeu-OH and H-MeGly(nPr)-Ile-Pro-OtBu

[Formula 17]

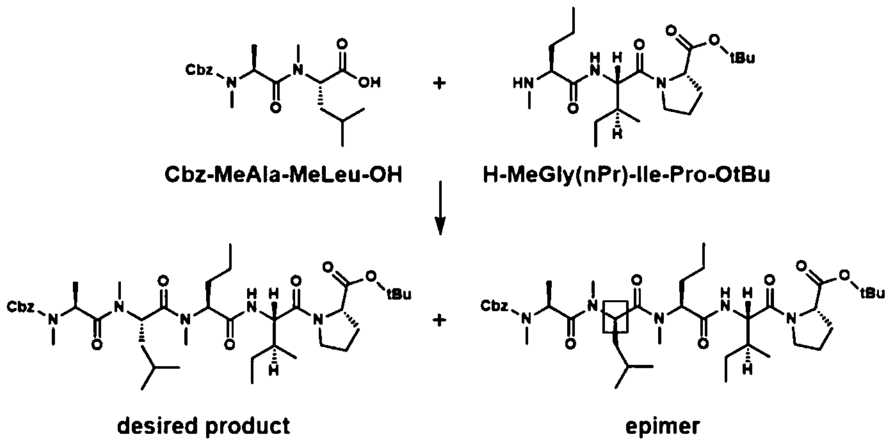

Cbz-MeAla-MeLeu-OH

H-MeGly(nPr)-Ile-Pro-OtBu

desired product

epimer

Experiment with DPPC, HOPO, and OxymaPure

To a test tube with a stirrer, H-MeGly(nPr)-Ile-Pro-OtBu (24.1 mg, 0.061 mmol), OxymaPure (8.6 mg, 0.061 mmol), HOPO (13.5 mg, 0.121 mmol), 2-MeTHF (0.200 mL), a solution of Cbz-MeAla-MeLeu-OH in 2-MeTHF (concentration 170.0 mg/mL, 0.160 mL, 27.0 mg, 0.073 mmol), and $Cy_2NMe$ (90.0 μL, 0.424 mmol) were sequentially added. The reaction mixture was cooled to -5°C with stirring. To the reaction mixture, DPPC (25.0 μL, 0.121 mmol) was added, and the temperature was raised to 10°C and the mixture was stirred for 10 hours. The reaction mixture was analyzed by HPLC, and the reaction conversion rate and the diastereomer ratio were evaluated.

Comparative Experiment with HATU

To a test tube with a stirrer, H-MeGly(nPr)-Ile-Pro-OtBu (24.1 mg, 0.073 mmol), MeCN (0.200 mL), a solution of Cbz-MeAla-MeLeu-OH in 2-MeTHF (concentration 170.0 mg/mL, 0.160 mL, 27.0 mg, 0.073 mmol), and DIPEA (21.1 μL, 0.121 mmol) were sequentially added. The reaction mixture was cooled to 10°C with stirring, HATU (46.1 mg, 0.121 mmol) was added, and the mixture was stirred for 10 hours. The reaction mixture was analyzed by HPLC, and the reaction conversion rate and the diastereomer ratio were evaluated.

Measurement method: HPLC Method A
Retention time: H-MeGly(nPr)-Ile-Pro-OtBu: 8.6 min, desired product: 18.0 min, epimer: 18.1 min
Mass spectrometry: H-MeGly(nPr)-Ile-Pro-OtBu: m/z 398.81 ($[M+H]^+$), desired product: m/z 745.09 ($[M+H]^+$), epimer: m/z 745.15 ($[M+H]^+$)

[Table 12]

| Reaction conditions | | Analysis results | |
|---|---|---|---|
| | **Condensation condition** | **Reaction conversion rate (%)** | **Diastereomer ratio** |
| **Condition A** | **DPPC, HOPO, OxymaPure** | **97.6** | **99.5 / 0.5** |
| **Condition B** | **HATU** | **99.3** | **67.5 / 32.5** |

As shown in Table 12, it was found that condition A with DPPC, HOPO, and OxymaPure gave a higher diastereomer ratio compared to condition B with HATU as the condensing agent.

(Example 16) Condensation reaction of Cbz-MeLeu-MePhe-OH and H-MeAla-Aze-EtPhe(4-Me)-Sar-OtBu

[Formula 18]

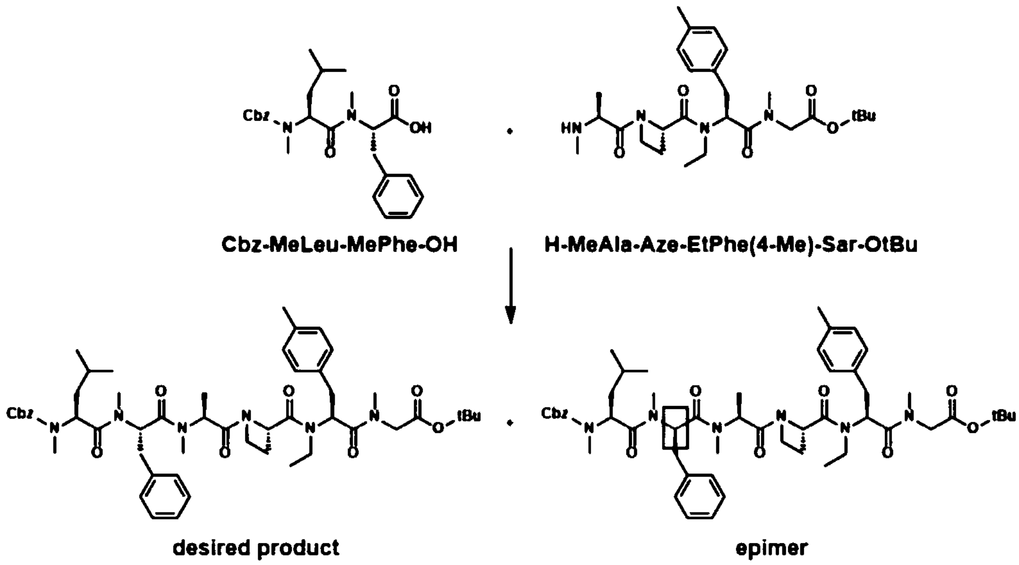

Cbz-MeLeu-MePhe-OH

H-MeAla-Aze-EtPhe(4-Me)-Sar-OtBu

desired product

epimer

Experiment with DPPC, HOPO, and OxymaPure

To a test tube with a stirrer, OxymaPure (8.5 mg, 0.060 mmol), HOPO (13.3 mg, 0.119 mmol), a solution of Cbz-MeLeu-MePhe-OH in 2-MeTHF (concentration 200.0 mg/mL, 0.160 mL, 32.0 mg, 0.072 mmol), a solution of H-MeAla-Aze-EtPhe(4-Me)-Sar-OtBu in 2-MeTHF (concentration 143.3 mg/mL, 0.210 mL, 30.0 mg, 0.060 mmol), and $Cy_2NMe$ (88.7 μL, 0.418 mmol) were sequentially added. The reaction mixture was cooled to -5°C with stirring. To the reaction mixture, DPPC (24.7 μL, 0.119 mmol) was added, and the temperature was raised to 10°C, and the mixture was stirred for 10 hours. The reaction mixture was analyzed by HPLC, and the reaction conversion rate and the diastereomer ratio were evaluated.

Comparative Experiment with HATU

To a test tube with a stirrer, a solution of Cbz-MeLeu-MePhe-OH in 2-MeTHF (concentration 200.0 mg/mL, 0.160 mL, 32.0 mg, 0.072 mmol), a solution of H-MeAla-Aze-EtPhe(4-Me)-Sar-OtBu in MeCN (concentration 200.0 mg/mL, 0.150 mL, 30.0 mg, 0.060 mmol), and DIPEA (20.8 μL, 0.119 mmol) were sequentially added. The reaction mixture was cooled to 10°C with stirring, HATU (45.4 mg, 0.119 mmol) was added, and the mixture was stirred for 5 hours. The reaction mixture was analyzed by HPLC, and the reaction conversion rate and the diastereomer ratio were evaluated.

Measurement method: HPLC Method A
Retention time: H-MeAla-Aze-EtPhe(4-Me)-Sar-OtBu: 8.0 min, desired product: 17.0 min, epimer: 17.3 min

Mass spectrometry: H-MeAla-Aze-EtPhe(4-Me)-Sar-OtBu: m/z 503.37 ([M+H]+), desired product: m/z 780.50 ([M-Sar-OtBu]+), epimer: m/z 780.50 ([M-Sar-OtBu]+)

[Table 13]

| Reaction conditions | | Analysis results | |
|---|---|---|---|
| | Condensation condition | Reaction conversion rate (%) | Diastereomer ratio |
| Condition A | DPPC, HOPO, OxymaPure | 99.9 | 99.7 / 0.3 |
| Condition B | HATU | 99.3 | 91.4 / 8.6 |

As shown in Table 13, it was found that condition A with DPPC, HOPO, and OxymaPure gave a higher diastereomer ratio compared to condition B with HATU as the condensing agent.

(Example 17) Condensation reaction of Cbz-MeLeu-D-MePhe-OH and H-MeAla-Aze-EtPhe(4-Me)-Sar-OtBu

[Formula 19]

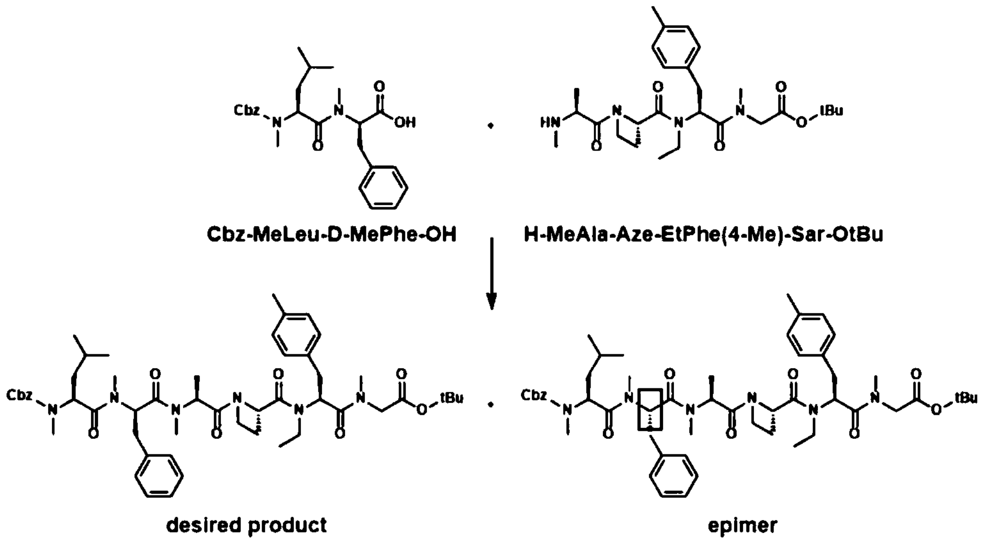


Experiment with DPPC, HOPO, and OxymaPure

To a test tube with a stirrer, OxymaPure (8.5 mg, 0.060 mmol), HOPO (13.3 mg, 0.119 mmol), a solution of Cbz-MeLeu-D-MePhe-OH in 2-MeTHF (concentration 200.0 mg/mL, 0.160 mL, 32.0 mg, 0.072 mmol, 1.2 equiv.), a solution of H-MeAla-Aze-EtPhe(4-Me)-Sar-OtBu in 2-MeTHF (concentration 143.3 mg/mL, 0.210 mL, 30.0 mg, 0.060 mmol, 1.0 equiv.), and $Cy_2NMe$ (88.7 μL, 0.418 mmol, 7.0 equiv.) were sequentially added. The reaction mixture was cooled to -5°C with stirring. To the reaction mixture, DPPC (24.7 μL, 0.119 mmol, 2.0 equiv.) was added, and the temperature was raised to 10°C, and the mixture was stirred for 10 hours. The reaction mixture was analyzed by HPLC, and the reaction conversion rate and the diastereomer ratio were evaluated.

Comparative Experiment with HATU

To a test tube with a stirrer, a solution of Cbz-MeLeu-D-MePhe-OH in 2-MeTHF (concentration 200.0 mg/mL, 0.160 mL, 32.0 mg, 0.072 mmol, 1.2 equiv.), a solution of H-MeAla-Aze-EtPhe(4-Me)-Sar-OtBu in MeCN (concentration 200.0 mg/mL, 0.150 mL, 30.0 mg, 0.060 mmol, 1.0 equiv.), and DIPEA (20.8 μL, 0.119 mmol) were sequentially added. The reaction mixture was cooled to 10°C with stirring, HATU (45.4 mg, 0.119 mmol) was added, and the mixture was stirred for 5 hours. The reaction mixture was analyzed by HPLC, and the reaction conversion rate and the diastereomer ratio were

evaluated.

Measurement method: HPLC Method A

Retention time: H-MeAla-Aze-EtPhe(4-Me)-Sar-OtBu: 8.0 min, desired product: 17.3 min, epimer: 17.0 min

Mass spectrometry: H-MeAla-Aze-EtPhe(4-Me)-Sar-OtBu: m/z 503.37 ([M+H]+), desired product: m/z 780.48 ([M-Sar-OtBu]+), epimer: m/z 780.57 ([M+H]+)

[Table 14]

| Reaction conditions | | Analysis results | |
|---|---|---|---|
| | **Condensation condition** | **Reaction conversion rate (%)** | **Diastereomer ratio** |
| **Condition A** | **DPPC, HOPO, OxymaPure** | **99.9** | **99.6 / 0.4** |
| **Condition B** | **HATU** | **99.5** | **96.1 / 3.9** |

As shown in Table 14, it was found that condition A with DPPC, HOPO, and OxymaPure gave a higher diastereomer ratio compared to condition B with HATU as the condensing agent.

(Example 18) Condensation reaction of Cbz-Leu-MePhe-OH and H-MeLeu-Ile-MeAla-Aze-EtPlie(4-Me)-Sar-OtBu

[Formula 20]

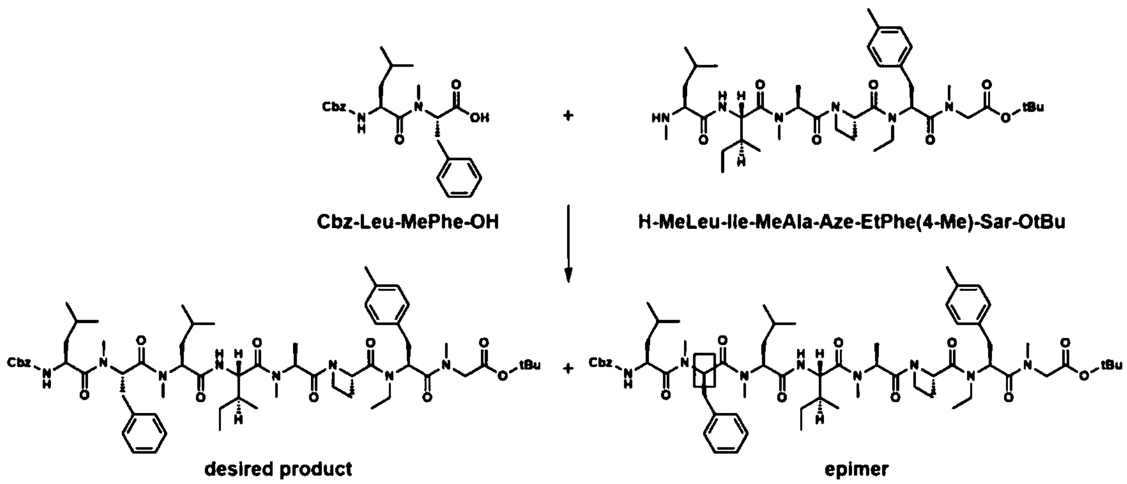


Experiment with DPPC, HOPO, and OxymaPure

To a test tube with a stirrer, OxymaPure (8.6 mg, 0.061 mmol), HOPO (13.5 mg, 0.121 mmol), Cbz-Leu-MePhe-OH (31.0 mg, 0.073 mmol), 2-MeTHF (0.200 mL), a solution of H-MeLeu-Ile-MeAla-Aze-EtPhe(4-Me)-Sar-OtBu in 2-MeTHF (concentration 150.0 mg/mL, 0.300 mL, 45.0 mg, 0.061 mmol), and $Cy_2NMe$ (90.0 μL, 0.424 mmol) were sequentially added. The reaction mixture was cooled to -5°C with stirring. To the reaction mixture, DPPC (25.0 μL, 0.121 mmol) was added, and the temperature was raised to 10°C, and the mixture was stirred for 10 hours. The reaction mixture was analyzed by HPLC, and the reaction conversion rate and the diastereomer ratio were evaluated.

Comparative Experiment with HATU

To a test tube with a stirrer, Cbz-Leu-MePhe-OH (31.0 mg, 0.073 mmol), MeCN (0.200 mL), a solution of H-MeLeu-Ile-MeAla-Aze-EtPhe(4-Me)-Sar-OtBu in MeCN (concentration 170.0 mg/mL, 0.270 mL, 45.9 mg, 0.061 mmol), and DIPEA (21.1 μL, 0.121 mmol) were sequentially added. The reaction mixture was cooled to 10°C with stirring, HATU (46.1 mg, 0.121 mmol) was added and the mixture was stirred for 5 hours. The reaction mixture was analyzed by HPLC,

and the reaction conversion rate and the diastereomer ratio were evaluated.

Measurement method: HPLC Method A
Retention time: H-MeLeu-Ile-MeAla-Aze-EtPhe(4-Me)-Sar-OtBu: 9.9 min, desired product: 17.9 min, epimer: 18.6 min
Mass spectrometry: H-MeLeu-Ile-MeAla-Aze-EtPhe(4-Me)-Sar-OtBu: m/z 744.24 ($[M+H]^+$), desired product: m/z 1007.53 ($[M\text{-Sar-OtBu}]^+$), epimer: m/z 1007.24 ($[M\text{-Sar-OtBu}]^+$)

[Table 15]

| Reaction conditions | | Analysis results | |
|---|---|---|---|
| | Condensation condition | Reaction conversion rate (%) | Diastereomer ratio |
| Condition A | DPPC, HOPO, OxymaPure | 94.5 | 99.2 / 0.8 |
| Condition B | HATU | 100.0 | 38.9 / 61.1 |

As shown in Table 15, it was found that condition A with DPPC, HOPO, and OxymaPure gave a higher diastereomer ratio compared to condition B with HATU as the condensing agent.

(Example 19) Condensation reaction of Cbz-Leu-MePhe-OH and H-MeLeu-Ile-MeAla-Aze-EtPhe(4-Me)-Sar-OtBu

[Formula 21]

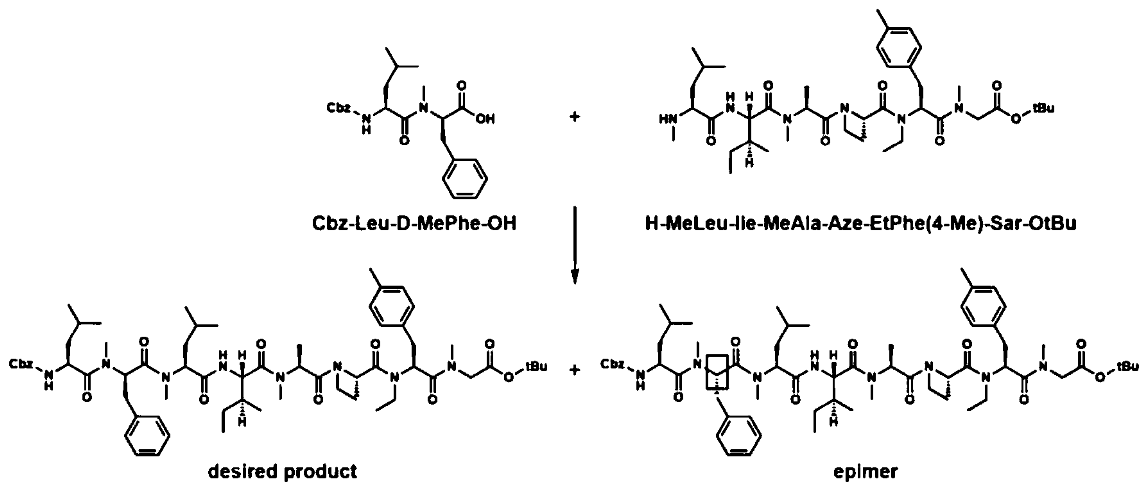


Experiment with DPPC, HOPO, and OxymaPure

To a test tube with a stirrer, OxymaPure (8.6 mg, 0.061 mmol), HOPO (13.5 mg, 0.121 mmol), Cbz-Leu-D-MePhe-OH (31.0 mg, 0.073 mmol), 2-MeTHF (0.200 mL), a solution of H-MeLeu-Ile-MeAla-Aze-EtPhe(4-Me)-Sar-OtBu in 2-MeTHF (concentration 150.0 mg/mL, 0.300 mL, 45.0 mg, 0.061 mmol), and $Cy_2NMe$ (90.0 μL, 0.424 mmol) were sequentially added. The reaction mixture was cooled to -5°C with stirring. To the reaction mixture, DPPC (25.0 μL, 0.121 mmol) was added, and the temperature was raised to 10°C, and the mixture was stirred for 5 hours. The reaction mixture was analyzed by HPLC, and the reaction conversion rate and the diastereomer ratio were evaluated.

Comparative Experiment with HATU

To a test tube with a stirrer, Cbz-Leu-D-MePhe-OH (31.0 mg, 0.073 mmol), MeCN (0.200 mL), a solution of H-MeLeu-Ile-MeAla-Aze-EtPhe(4-Me)-Sar-OtBu in MeCN (concentration 170.0 mg/mL, 0.270 mL, 45.9 mg, 0.061 mmol), and DIPEA (21.1 μL, 0.121 mmol) were sequentially added. The reaction mixture was cooled to 10°C with stirring, HATU (46.1 mg, 0.121 mmol) was added, and the mixture was stirred for 4 hours. The reaction mixture was analyzed by HPLC,

and the reaction conversion rate and the diastereomer ratio were evaluated.

Measurement method: HPLC Method A
Retention time: H-MeLeu-Ile-MeAla-Aze-EtPhe(4-Me)-Sar-OtBu: 9.9 min, desired product: 18.6 min, epimer: 17.9 min
Mass spectrometry: H-MeLeu-Ile-MeAla-Aze-EtPhe(4-Me)-Sar-OtBu: m/z 744.24 ([M+H]$^+$), desired product: m/z 1007.24 ([M-Sar-OtBu]$^+$), epimer: m/z 1007.35 ([M-Sar-OtBu]$^+$)

[Table 16]

| Reaction conditions | | Analysis results | |
|---|---|---|---|
| | **Condensation condition** | **Reaction conversion rate (%)** | **Diastereomer ratio** |
| **Condition A** | **DPPC, HOPO, OxymaPure** | **98.7** | **99.8 / 0.2** |
| **Condition B** | **HATU** | **100.0** | **99.5 / 0.5** |

As shown in Table 16, it was found that condition A with DPPC, HOPO, and OxymaPure gave a higher diastereomer ratio compared to condition B with HATU as the condensing agent.

(Example 20) Condensation reaction of Cbz-D-Leu-MePhe-OH and H-MeLeu-Ile-MeAla-Aze-EtPlie(4-Me)-Sar-OtBu

[Formula 22]

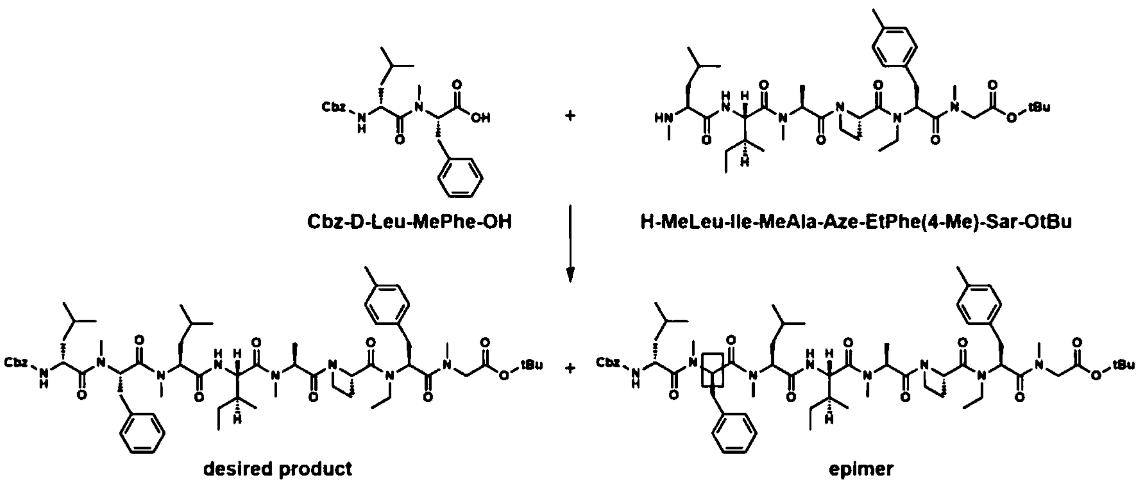


Experiment with DPPC, HOPO, and OxymaPure

To a test tube with a stirrer, OxymaPure (8.6 mg, 0.061 mmol), HOPO (13.5 mg, 0.121 mmol), Cbz-D-Leu-MePhe-OH (31.0 mg, 0.073 mmol), 2-MeTHF (0.200 mL), a solution of H-MeLeu-Ile-MeAla-Aze-EtPhe(4-Me)-Sar-OtBu in 2-MeTHF (concentration 150.0 mg/mL, 0.300 mL, 45.0 mg, 0.061 mmol), and $Cy_2NMe$ (90.0 μL, 0.424 mmol) were sequentially added. The reaction mixture was cooled to -5°C with stirring. To the reaction mixture, DPPC (25.0 μL, 0.121 mmol) was added, and the temperature was raised to 10°C, and the mixture was stirred for 5 hours. The reaction mixture was analyzed by HPLC, and the reaction conversion rate and the diastereomer ratio were evaluated.

Comparative Experiment with HATU

To a test tube with a stirrer, Cbz-D-Leu-MePhe-OH (31.0 mg, 0.073 mmol), MeCN (0.200 mL), a solution of H-MeLeu-Ile-MeAla-Aze-EtPhe(4-Me)-Sar-OtBu in MeCN (concentration 170.0 mg/mL, 0.270 mL, 45.9 mg, 0.061 mmol), and DIPEA (21.1 μL, 0.121 mmol) were sequentially added. The reaction mixture was cooled to 10°C with stirring, HATU

(46.1 mg, 0.121 mmol) was added, and the mixture was stirred for 4.5 hours. The reaction mixture was analyzed by HPLC, and the reaction conversion rate and the diastereomer ratio were evaluated.

Measurement method: HPLC Method A
Retention time: H-MeLeu-Ile-MeAla-Aze-EtPhe(4-Me)-Sar-OtBu: 9.9 min, desired product: 18.1 min, epimer: 18.2 min
Mass spectrometry: H-MeLeu-Ile-MeAla-Aze-EtPhe(4-Me)-Sar-OtBu: m/z 744.24 ([M+H]$^+$), desired product: m/z 1007.30 ([M-Sar-OtBu]$^+$), epimer: m/z 1007.30 ([M-Sar-OtBu]$^+$)

[Table 17]

| Reaction conditions | | Analysis results | |
|---|---|---|---|
| | Condensation condition | Reaction conversion rate (%) | Diastereomer ratio |
| Condition A | DPPC, HOPO, OxymaPure | 98.2 | 100.0 / 0.0 |
| Condition B | HATU | 99.8 | 94.6 / 5.4 |

As shown in Table 17, it was found that condition A with DPPC, HOPO, and OxymaPure gave a higher diastereomer ratio compared to condition B with HATU as the condensing agent.

(Example 21) Condensation reaction of Cbz-MeLeu-MeSer(OtBu)-OH and H-MeLeu-Ile-MeAla-Aze-EtPhe(4-Me)-Sar-OtBu

[Formula 23]

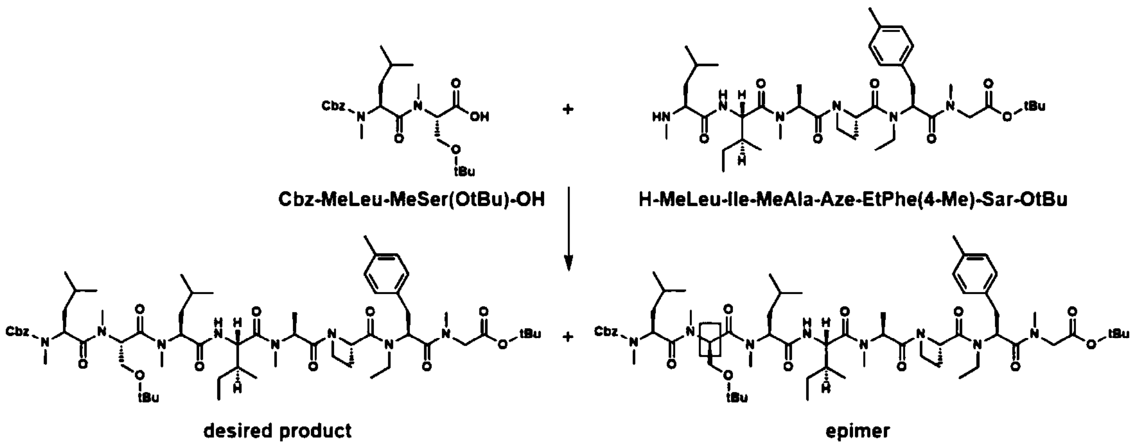


Experiment with DPPC, HOPO, and OxymaPure

To a test tube with a stirrer, OxymaPure (11.0 mg, 0.077 mmol), HOPO (17.0 mg, 0.153 mmol), a solution of Cbz-MeLeu-MeSer(OtBu)-OH in 2-MeTHF (concentration 166.5 mg/mL, 0.240 mL, 40.0 mg, 0.092 mmol), a solution of H-MeLeu-Ile-MeAla-Aze-EtPhe(4-Me)-Sar-OtBu in 2-MeTHF (concentration 150.0 mg/mL, 0.380 mL, 57.0 mg, 0.077 mmol), and $Cy_2NMe$ (114.0 μL, 0.537 mmol) were sequentially added. The reaction mixture was cooled to -5°C with stirring. To the reaction mixture, DPPC (31.7 μL, 0.153 mmol) was added, and the temperature was raised to 10°C, and the mixture was stirred for 10 hours. The reaction mixture was analyzed by HPLC, and the reaction conversion rate and the diastereomer ratio were evaluated.

Comparative Experiment with HATU

To a test tube with a stirrer, a solution of Cbz-MeLeu-MeSer(OtBu)-OH in 2-MeTHF (concentration 166.0 mg/mL, 0.240 mL, 40.0 mg, 0.092 mmol), a solution of H-MeLeu-Ile-MeAla-Aze-EtPhe(4-Me)-Sar-OtBu in MeCN (concentration

170.0 mg/mL, 0.340 mL, 57.8 mg, 0.077 mmol), and DIPEA (26.8 μL, 0.153 mmol) were sequentially added. The reaction mixture was cooled to 10°C with stirring, HATU (58.4 mg, 0.153 mmol) was added, and the mixture was stirred for 5 hours. The reaction mixture was analyzed by HPLC, and the reaction conversion rate and the diastereomer ratio were evaluated.

Measurement method: HPLC Method A
Retention time: H-MeLeu-Ile-MeAla-Aze-EtPhe(4-Me)-Sar-OtBu: 10.0 min, desired product: 18.9 min, epimer: 19.4 min
Mass spectrometry: H-MeLeu-Ile-MeAla-Aze-EtPhe(4-Me)-Sar-OtBu: m/z 744.01 ($[M+H]^+$), desired product: m/z 1017.67 ($[M\text{-Sar-OtBu}]^+$), epimer: m/z 1017.27 ($[M\text{-Sar-OtBu}]^+$)

[Table 18]

| Reaction conditions | | Analysis results | |
|---|---|---|---|
| | **Condensation condition** | **Reaction conversion rate (%)** | **Diastereomer ratio** |
| **Condition A** | **DPPC, HOPO, OxymaPure** | **99.1** | **98.8 / 1.2** |
| **Condition B** | **HATU** | **99.4** | **62.9 / 37.1** |

As shown in Table 18, it was found that condition A with DPPC, HOPO, and OxymaPure gave a higher diastereomer ratio compared to condition B with HATU as the condensing agent.

(Example 22) Condensation reaction of Cbz-MeLeu-MeSer(OtBu)-OH and H-MeGly(cPent)-MeAsp(OtBu)-$NMe_2$

[Formula 24]

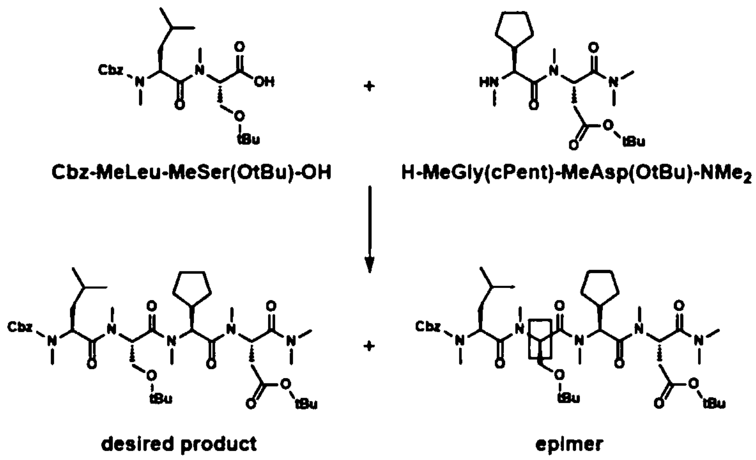

Cbz-MeLeu-MeSer(OtBu)-OH + H-MeGly(cPent)-MeAsp(OtBu)-$NMe_2$

desired product + epimer

Experiment with DPPC, HOPO, and OxymaPure

To a test tube with a stirrer, OxymaPure (11.0 mg, 0.077 mmol), HOPO (17.0 mg, 0.153 mmol), a solution of Cbz-MeLeu-MeSer(OtBu)-OH in 2-MeTHF (concentration 166.0 mg/mL, 0.240 mL, 40.0 mg, 0.092 mmol), a solution of H-MeGly(cPent)-MeAsp(OtBu)-$NMe_2$ in 2-MeTHF (concentration 129.7 mg/mL, 0.215 mL, 27.9 mg, 0.077 mmol), and $Cy_2NMe$ (114.0 μL, 0.536 mmol) were sequentially added. The reaction mixture was cooled to -5°C with stirring. To the reaction mixture, DPPC (31.7 μL, 0.153 mmol) was added, and the temperature was raised to 10°C, and the mixture was stirred for 24 hours. The reaction mixture was analyzed by HPLC, and the reaction conversion rate and the diastereomer ratio were evaluated.

Comparative Experiment with HATU

To a test tube with a stirrer, a solution of Cbz-MeLeu-MeSer(OtBu)-OH in 2-MeTHF (concentration 166.0 mg/mL, 0.240 mL, 40.0 mg, 0.092 mmol), a solution of H-MeGly(cPent)-MeAsp(OtBu)-$NMe_2$ in MeCN (concentration 75.8 mg/mL, 0.360 mL, 27.3 mg, 0.077 mmol), and DIPEA (26.8 μL, 0.153 mmol) were sequentially added. The reaction mixture was cooled to 10°C with stirring, HATU (58.4 mg, 0.153 mmol) was added, and the mixture was stirred for 2 hours. The reaction mixture was analyzed by HPLC, and the reaction conversion rate and the diastereomer ratio were evaluated.

Measurement method: HPLC Method A
Retention time: H-MeGly(cPent)-MeAsp(OtBu)-$NMe_2$: 5.7 min, desired product: 17.9 min, epimer: 18.3 min
Mass spectrometry: H-MeGly(cPent)-MeAsp(OtBu)-$NMe_2$: m/z 370.71 ($[M+H]^+$), desired product: m/z 744.07 ($[M-NMe_2]^+$), epimer: m/z 743.95 ($[M-NMe_2]^+$)

[Table 19]

| Reaction conditions | | Analysis results | |
|---|---|---|---|
| | **Condensation condition** | **Reaction conversion rate (%)** | **Diastereomer ratio** |
| **Condition A** | **DPPC, HOPO, OxymaPure** | **98.1** | **97.4 / 2.6** |
| **Condition B** | **HATU** | **94.9** | **38.2 / 61.8** |

As shown in Table 19, it was found that condition A with DPPC, HOPO, and OxymaPure gave a higher diastereomer ratio compared to condition B with HATU as the condensing agent.

(Example 23) Condensation reaction of Cbz-Aze-EtPhe(4-Me)-OH and H-MeAla-Aze-EtPhe(4-Me)-Sar-OtBu

[Formula 25]

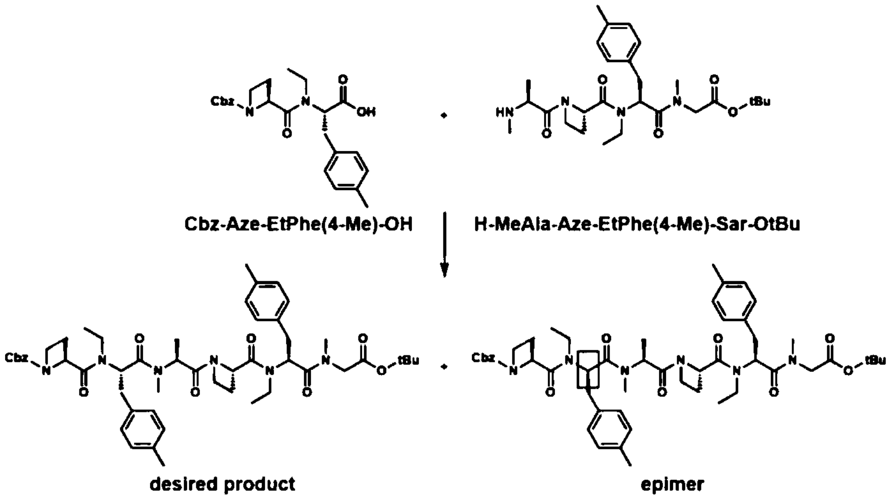


Experiment with DPPC, HOPO, and OxymaPure

To a test tube with a stirrer, OxymaPure (8.6 mg, 0.061 mmol), HOPO (13.5 mg, 0.121 mmol), Cbz-Aze-EtPhe(4-Me)-OH (31.0 mg, 0.073 mmol), 2-MeTHF (0.200 mL), a solution of H-MeAla-Aze-EtPhe(4-Me)-Sar-OtBu in 2-MeTHF (concentration 143.3 mg/mL, 0.210 mL, 30.1 mg, 0.061 mmol), and $Cy_2NMe$ (90.0 μL, 0.425 mmol) were sequentially added. The reaction mixture was cooled to -5°C with stirring. To the reaction mixture, DPPC (25.0 μL, 0.121 mmol) was added, and the temperature was raised to 10°C, and the mixture was stirred for 24 hours. The reaction mixture was analyzed by HPLC, and the reaction conversion rate and the diastereomer ratio were evaluated.

Comparative Experiment with HATU

To a test tube with a stirrer, Cbz-Aze-EtPhe(4-Me)-OH (31.0 mg, 0.073 mmol), MeCN (0.200 mL), a solution of H-MeAla-Aze-EtPhe(4-Me)-Sar-OtBu in MeCN (concentration 200.0 mg/mL, 0.150 mL, 30.0 mg, 0.060 mmol), and DIPEA (21.1 μL, 0.121 mmol) were sequentially added. The reaction mixture was cooled to 10°C with stirring, HATU (46.1 mg, 0.121 mmol) was added and the mixture was stirred for 10 hours. The reaction mixture was analyzed by HPLC, and the reaction conversion rate and the diastereomer ratio were evaluated.

Measurement method: HPLC Method A
Retention time: H-MeAla-Aze-EtPhe(4-Me)-Sar-OtBu: 8.1 min, desired product: 15.8 min, epimer: 15.9 min
Mass spectrometry: H-MeAla-Aze-EtPhe(4-Me)-Sar-OtBu: m/z 503.81 ($[M+H]^+$), desired product: m/z 766.01 ($[M\text{-}Sar\text{-}OtBu]^+$), epimer: m/z 765.95 ($[M\text{-}Sar\text{-}OtBu]^+$)

[Table 20]

| Reaction conditions | | Analysis results | |
|---|---|---|---|
| | **Condensation condition** | **Reaction conversion rate (%)** | **Diastereomer ratio** |
| **Condition A** | **DPPC, HOPO, OxymaPure** | **96.2** | **99.0 / 1.0** |
| **Condition B** | **HATU** | **98.4** | **84.9 / 15.1** |

As shown in Table 20, it was found that condition A with DPPC, HOPO, and OxymaPure gave a higher diastereomer ratio compared to condition B with HATU as the condensing agent.

(Example 24) Condensation reaction of Fmoc-MeAlGly-MeAlGly-OH and H-MeGly(nPr)-Ile-Pro-OtBu

[Formula 26]

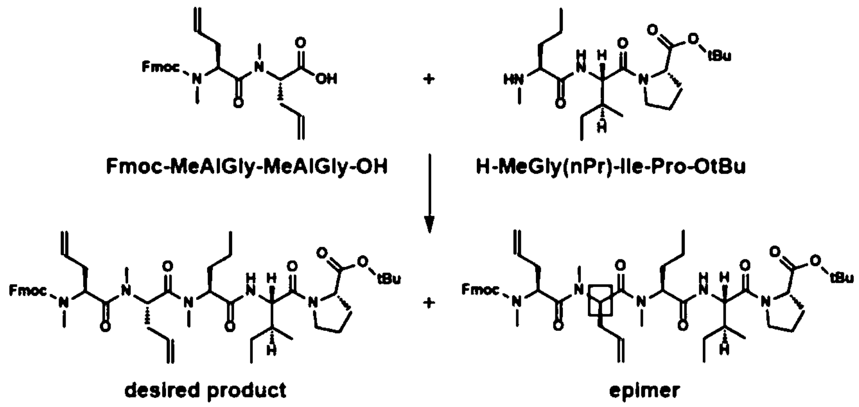

Fmoc-MeAlGly-MeAlGly-OH

H-MeGly(nPr)-Ile-Pro-OtBu

desired product

epimer

Experiment with DPPC, HOPO, and OxymaPure

To a test tube with a stirrer, H-MeGly(nPr)-Ile-Pro-OtBu (24.1 mg, 0.061 mmol), Fmoc-MeAlGly-MeAlGly-OH (33.6 mg, 0.073 mmol), OxymaPure (8.6 mg, 0.061 mmol), HOPO (13.5 mg, 0.121 mmol), 2-MeTHF (0.400 mL), and DIPEA (73.9 μL, 0.424 mmol) were sequentially added. The reaction mixture was cooled to -5°C with stirring. To the reaction mixture, DPPC (25.1 μL, 0.121 mmol) was added, and the temperature was raised to 10°C, and the mixture was stirred for 10 hours. The reaction mixture was analyzed by HPLC, and the reaction conversion rate and the diastereomer ratio were evaluated.

Comparative Experiment with HATU

To a test tube with a stirrer, H-MeGly(nPr)-Ile-Pro-OtBu (24.1 mg, 0.061 mmol), Fmoc-MeAlGly-MeAlGly-OH (33.6 mg, 0.073 mmol), MeCN (0.400 mL), and DIPEA (21.1 μL, 0.121 mmol) were sequentially added. The reaction mixture was cooled to 10°C with stirring, HATU (46.1 mg, 0.121 mmol) was added, and the mixture was stirred for 10 hours. The reaction mixture was analyzed by HPLC, and the reaction conversion rate and the diastereomer ratio were evaluated.

Measurement method: HPLC Method A
Retention time: H-MeGly(nPr)-Ile-Pro-OtBu: 8.7 min, desired product: 19.3 min, epimer: 19.4 min
Mass spectrometry: H-MeGly(nPr)-Ile-Pro-OtBu: m/z 398.81 ($[M+H]^+$), desired product: m/z 865.19 ($[M+Na]^+$), epimer: m/z 865.08 ($[M+Na]^+$)

[Table 21]

| Reaction conditions | | Analysis results | |
|---|---|---|---|
| | Condensation condition | Reaction conversion rate (%) | Diastereomer ratio |
| Condition A | DPPC, HOPO, OxymaPure | 99.7 | 99.7 / 0.3 |
| Condition B | HATU | 99.8 | 80.8 / 19.2 |

As shown in Table 21, it was found that condition A with DPPC, HOPO, and OxymaPure gave a higher diastereomer ratio compared to condition B with HATU as the condensing agent.

(Example 25) Study of solvent in condensation reaction of Cbz-MeLeu-MeIle-OH and H-MeAla-Aze-EtPhe(4-Me)-Sar-OtBu

[Formula 27]

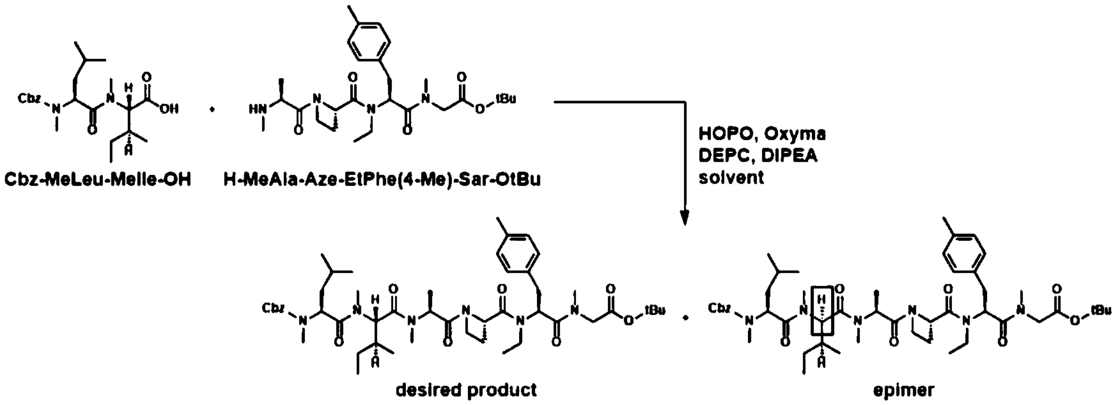


Cbz-MeLeu-MeIle-OH H-MeAla-Aze-EtPhe(4-Me)-Sar-OtBu

desired product epimer

To a test tube with a stirrer, Cbz-MeLeu-MeIle-OH (38.2 mg, 0.092 mmol), H-MeAla-Aze-EtPhe(4-Me)-Sar-OtBu (38.6 mg, 0.077 mmol), OxymaPure (11.0 mg, 0.077 mmol), HOPO (17.1 mg, 0.154 mmol), a solvent (0.500 mL), and DIPEA (93.6 μL, 0.538 mmol) were sequentially added. The reaction mixture was cooled to 10°C with stirring. To the reaction mixture, DEPC (22.2 μL, 0.154 mmol) was added, and the mixture was stirred for 24 hours. The reaction mixture was analyzed by HPLC, and the reaction conversion rate and the diastereomer ratio were evaluated.

Measurement method: HPLC Method A
Retention time: H-MeAla-Aze-EtPhe(4-Me)-Sar-OtBu: 8.3 min, desired product: 17.6 min, epimer: 18.1 min
Mass spectrometry: H-MeAla-Aze-EtPhe(4-Me)-Sar-OtBu m/z: 503.98 ($[M+H]^+$), desired product: m/z 914.21 ($[M+Na]^+$), epimer: m/z 914.27 ($[M+Na]^+$)

[Table 22]

| Reaction conditions | | Analysis results | |
|---|---|---|---|
| Run | Solvent | Reaction conversion rate (%) | Diastereomer ratio |
| 1 | α,α,α-trifluorotoluene | 63.7 | 91.9 / 8.1 |
| 2 | Toluene | 64.4 | 91.5 / 8.5 |
| 3 | IPAC | 61.0 | 90.5 / 9.5 |
| 4 | CPME | 49.0 | 87.0 / 13.0 |
| 5 | MTHP | 49.4 | 90.0 / 10.0 |
| 6 | 2-MeTHF | 57.4 | 90.3 / 9.7 |
| 7 | THF | 53.7 | 88.1 / 11.9 |
| 8 | MeCN | 41.6 | 92.9 / 7.1 |
| 9 | DMI | 33.0 | 83.1 / 16.9 |
| 10 | DMA | 30.9 | 86.1 / 13.9 |
| 11 | DCM | 42.9 | 90.5 / 9.5 |

As shown in Table 22, it was found that α,α,α-trifluorotoluene and toluene gave a particularly high reaction conversion rate and diastereomer ratio.

(Example 26) Study of organic base in condensation reaction of Cbz-MeLeu-MeIle-OH and H-MeAla-Aze-EtPhe(4-Me)-Sar-OtBu

[Formula 28]

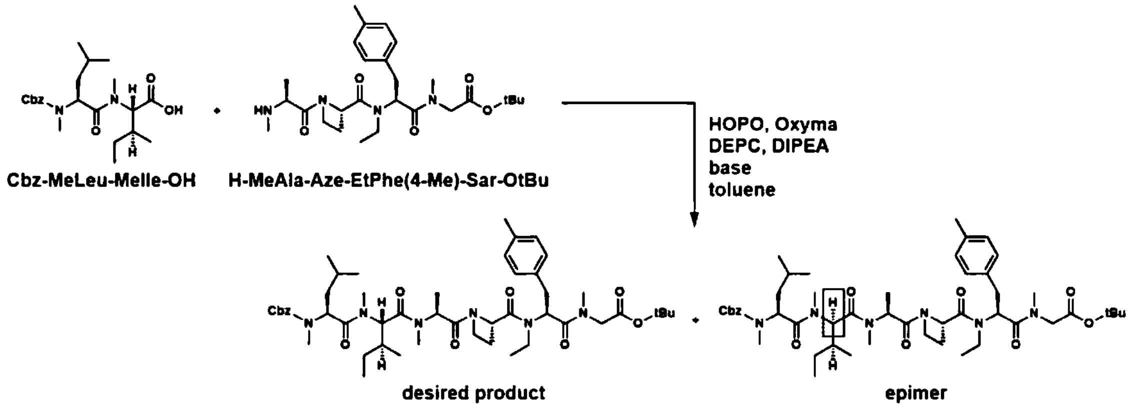


To a test tube with a stirrer, Cbz-MeLeu-MeIle-OH (38.2 mg, 0.092 mmol), H-MeAla-Aze-EtPhe(4-Me)-Sar-OtBu (38.6 mg, 0.077 mmol), OxymaPure (11.0 mg, 0.077 mmol), HOPO (17.1 mg, 0.154 mmol), toluene (0.500 mL), and DIPEA (93.6 μL, 0.538 mmol) were sequentially added. The reaction mixture was cooled to 10°C with stirring. To the reaction mixture, DEPC (22.2 μL, 0.154 mmol) was added, and the mixture was stirred for 3 hours. To the reaction mixture, a base (0.538 mmol) was added, and the mixture was stirred continuously for an additional 21 hours. The reaction mixture was analyzed by HPLC, and the reaction conversion rate and the diastereomer ratio were evaluated.

Measurement method: HPLC Method A
Retention time: H-MeAla-Aze-EtPhe(4-Me)-Sar-OtBu: 8.3 min, desired product: 17.6 min, epimer: 18.1 min
Mass spectrometry: H-MeAla-Aze-EtPhe(4-Me)-Sar-OtBu: m/z 503.81 ([M+H]$^+$), desired product: m/z 914.21 ([M+Na]$^+$), epimer: m/z 913.99 ([M+Na]$^+$)

[Table 23]

| Reaction conditions | | Analysis results | |
|---|---|---|---|
| Run | Organic base | Reaction conversion rate (%) | Diastereomer ratio |
| 1 | none | 61.7 | 91.9 / 8.1 |
| 2 | NMI | 53.3 | 92.0 / 8.0 |
| 3 | DIPEA | 63.5 | 91.7 / 8.3 |
| 4 | $Cy_2NMe$ | 72.3 | 92.4 / 7.6 |
| 5 | DBU | 72.9 | 54.8 / 45.2 |
| 6 | DBN | 74.8 | 50.4 / 49.6 |
| 7 | TMG | 23.0 | 91.1 / 8.9 |
| 8 | MTBD | 74.7 | 84.1 / 15.9 |
| 9 | BTMG | 74.1 | 92.6 / 7.4 |

As shown in Table 23, it was found that $Cy_2NMe$ and BTMG gave a high reaction conversion rate and diastereomer ratio.

(Example 27) Study of inorganic base in condensation reaction of Cbz-MeLeu-MeIle-OH and H-MeAla-Aze-EtPhe(4-Me)-Sar-OtBu

[Formula 29]

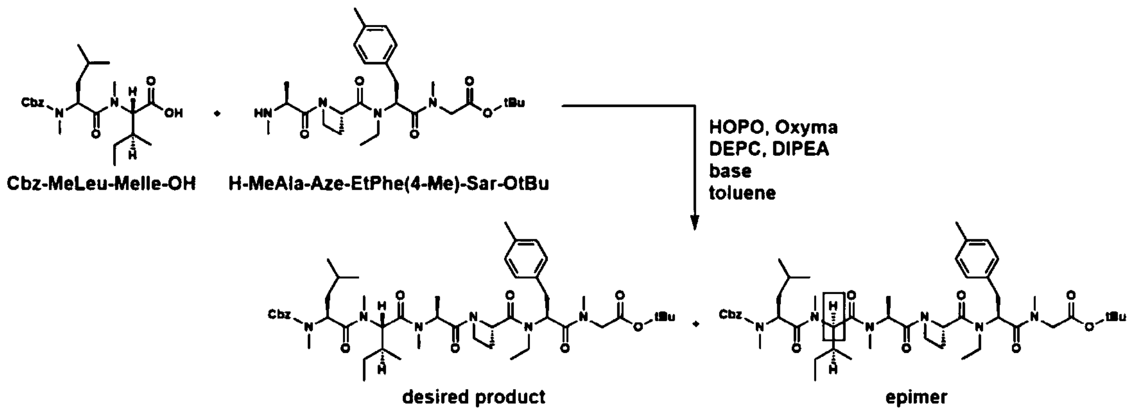


To a test tube with a stirrer, OxymaPure (11.0 mg, 0.077 mmol), HOPO (17.1 mg, 0.154 mmol), a solution of Cbz-MeLeu-MeIle-OH in toluene (concentration 164.0 mg/mL, 0.240 mL, 39.4 mg, 0.096 mmol), a solution of H-MeAla-Aze-EtPhe(4-Me)-Sar-OtBu in toluene (concentration 169.0 mg/mL, 0.230 mL, 38.9 mg, 0.077 mmol), and $Cy_2NMe$ (114.0 μL, 0.538 mmol) were sequentially added. The reaction mixture was cooled to 10°C with stirring. To the reaction mixture, DEPC (22.2 μL, 0.154 mmol) was added, and the mixture was stirred for 5 hours. To the reaction mixture, an inorganic base (0.538 mmol) was added, and the mixture was stirred continuously for an additional 19 hours. The reaction mixture was analyzed by HPLC, and the reaction conversion rate and the diastereomer ratio were evaluated.

Measurement method: HPLC Method A
Retention time: H-MeAla-Aze-EtPhe(4-Me)-Sar-OtBu: 8.3 min, desired product: 17.6 min, epimer: 18.1 min
Mass spectrometry: H-MeAla-Aze-EtPhe(4-Me)-Sar-OtBu: m/z 503.98 ($[M+H]^+$), desired product: m/z 914.21 ($[M+Na]^+$), epimer: m/z 914.27 ($[M+Na]^+$)

[Table 24]

| Reaction conditions | | Analysis results | |
|---|---|---|---|
| Run | Inorganic base | Reaction conversion rate (%) | Diastereomer ratio |
| 1 | none | 77.7 | 95.9 / 4.1 |
| 2 | $K_2CO_3$ fine powder | 88.8 | 96.1 / 3.9 |
| 3 | $K_3PO_4$ fine powder | 93.8 | 96.2 / 3.8 |

As shown in Table 24, it was found that $K_3PO_4$ and $K_2CO_3$ gave a high reaction conversion rate and diastereomer ratio.

(Example 28) Study of equivalent ratio of HOPO and Oxyma Pure in condensation reaction of Cbz-MeLeu-MeIle-OH and H-MeAla-Aze-EtPhe(4-Me)-Sar-OtBu

[Formula 30]

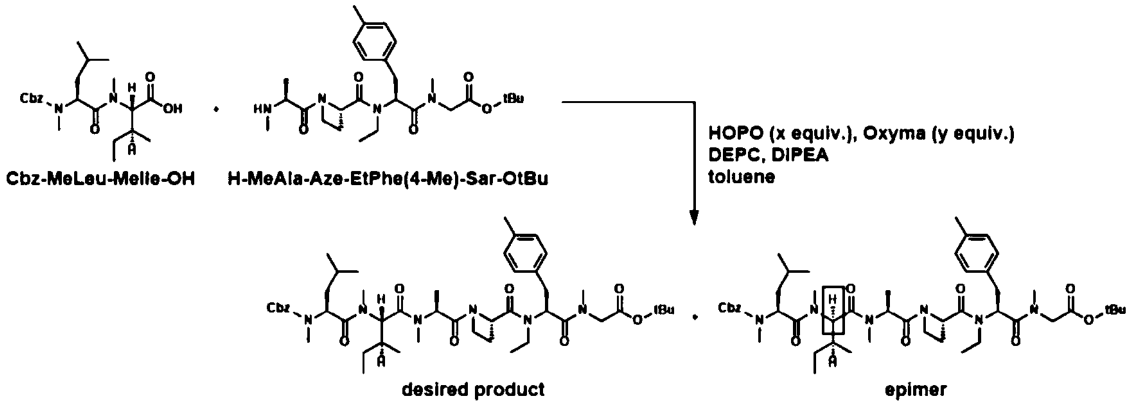


To a test tube with a stirrer, Cbz-MeLeu-MeIle-OH (38.2 mg, 0.092 mmol), H-MeAla-Aze-EtPhe(4-Me)-Sar-OtBu (38.6 mg, 0.077 mmol), HOPO (x mmol), OxymaPure (y mmol), toluene (0.500 mL), and DIPEA (93.6 μL, 0.538 mmol) were sequentially added. The reaction mixture was cooled to 10°C with stirring. To the reaction mixture, DEPC (22.2 μL, 0.154 mmol) was added, and the mixture was stirred for 5 hours. To the reaction mixture, DIPEA (93.6 μL, 0.538 mmol) was added, and the mixture was stirred continuously for an additional 19 hours. The reaction mixture was analyzed by HPLC, and the reaction conversion rate and the diastereomer ratio were evaluated.

Measurement method: HPLC Method A
Retention time: H-MeAla-Aze-EtPhe(4-Me)-Sar-OtBu: 8.3 min, desired product: 17.6 min, epimer: 18.1 min
Mass spectrometry: H-MeAla-Aze-EtPhe(4-Me)-Sar-OtBu: m/z 503.98 ($[M+H]^+$), desired product: m/z 914.21 ($[M+Na]^+$), epimer: m/z 914.27 ($[M+Na]^+$)

[Table 25]

| Reaction conditions | | | Analysis results | |
|---|---|---|---|---|
| Run | HOPO (equiv.) | OxymaPure (equiv.) | Reaction conversion rate (%) | Diastereomer ratio |
| 1 | 2.0 | 1.0 | 73.8 | 92.1 / 7.9 |
| 2 | 2.0 | 2.0 | 76.8 | 92.3 / 7.7 |
| 3 | 2.0 | 3.0 | 78.8 | 92.0 / 8.0 |
| 4 | 3.0 | 3.0 | 63.7 | 93.1 / 6.9 |
| 5 | 3.0 | 1.0 | 60.5 | 91.6 / 8.4 |

As shown in Table 25, the effect of the equivalent ratio of HOPO and OxymaPure on the diastereomer ratio is small.

(Example 29) Study of hydroxypyridine derivative in condensation reaction of Cbz-MeLeu-MeIle-OH and H-MeAla-Aze-EtPhe(4-Me)-Sar-OtBu

[Formula 31]

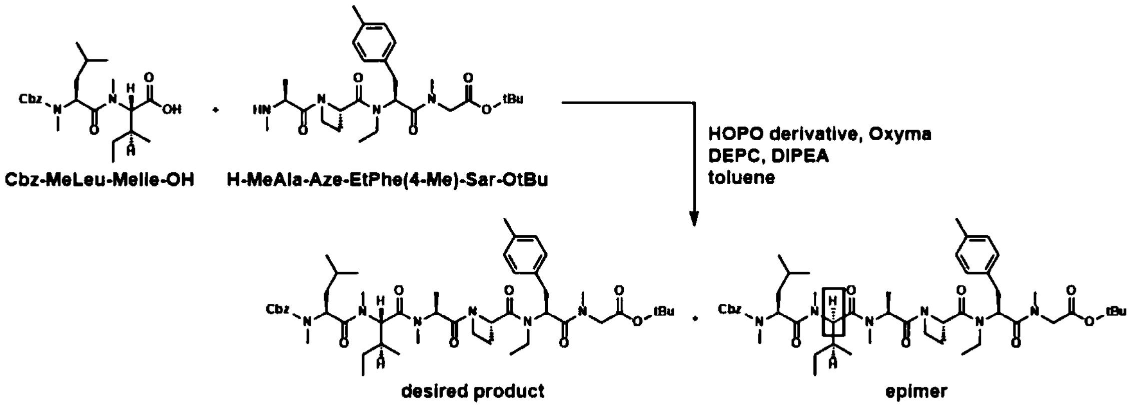


Cbz-MeLeu-MeIle-OH H-MeAla-Aze-EtPhe(4-Me)-Sar-OtBu

desired product epimer

[Formula 32]

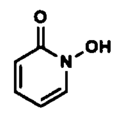
HOPO

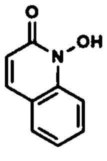
A

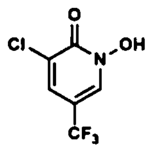
3-Cl-5-$CF_3$-HOPO

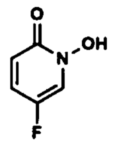
5-F-HOPO

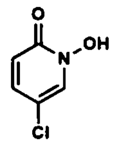
5-Cl-HOPO

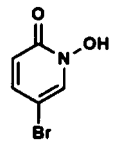
5-Br-HOPO

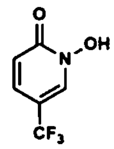
5-$CF_3$-HOPO

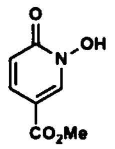
5-$CO_2$Me-HOPO

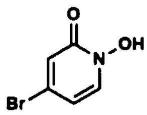
4-Br-HOPO

To a test tube with a stirrer, Cbz-MeLeu-MeIle-OH (38.2 mg, 0.092 mmol), H-MeAla-Aze-EtPhe(4-Me)-Sar-OtBu (38.6 mg, 0.077 mmol), OxymaPure (11.0 mg, 0.077 mmol), a HOPO derivative (0.154 mmol), toluene (0.500 mL), and DIPEA (187.0 μL, 1.080 mmol) were sequentially added. The reaction mixture was cooled to 10°C with stirring. To the reaction mixture, DEPC (22.2 μL, 0.154 mmol) was added, and the mixture was stirred for 24 hours. The reaction mixture was analyzed by HPLC, and the reaction conversion rate and the diastereomer ratio were evaluated.

Measurement method: HPLC Method A
Retention time: H-MeAla-Aze-EtPhe(4-Me)-Sar-OtBu: 8.3 min, desired product: 17.6 min, epimer: 18.1 min
Mass spectrometry: H-MeAla-Aze-EtPhe(4-Me)-Sar-OtBu: m/z 503.98 ($[M+H]^+$), desired product: m/z 914.21 ($[M+Na]^+$), epimer: m/z 914.27 ($[M+Na]^+$)

[Table 26]

| Reaction conditions | | Analysis results | |
|---|---|---|---|
| Run | Hydroxypyridine derivative | Reaction conversion rate (%) | Diastereomer ratio |
| 1 | HOPO | 67.3 | 92.2 / 7.8 |
| 2 | A | 3.8 | NA |
| 3 | 3-Cl-5-CF3-HOPO | 55.9 | 65.5 / 34.5 |
| 4 | 5-F-HOPO | 87.6 | 91.3 / 8.7 |
| 5 | 5-Cl-HOPO | 90.7 | 91.4 / 8.6 |
| 6 | 5-Br-HOPO | 90.3 | 93.4 / 6.6 |

(continued)

| Reaction conditions | | Analysis results | |
|---|---|---|---|
| Run | Hydroxypyridine derivative | Reaction conversion rate (%) | Diastereomer ratio |
| 7 | 5-$CF_3$-HOPO | 91.5 | 89.1 / 10.9 |
| 8 | 5-$CO_2$Me-HOPO | 88.7 | 94.4 / 5.6 |
| 9 | 4-Br-HOPO | 79.2 | 91.6 / 8.4 |

As shown in Table 26, it was found that the conditions of adding 5-Br-HOPO and 5-$CO_2$Me-HOPO gave a high reaction conversion rate and diastereomer ratio. Since the reaction conversion rate in the conditions of Run 2 was significantly low, the diastereomer ratio is not described.

(Raw material synthesis 1) Synthesis of Cbz-MeLeu-MePhe-OH

[Formula 33]

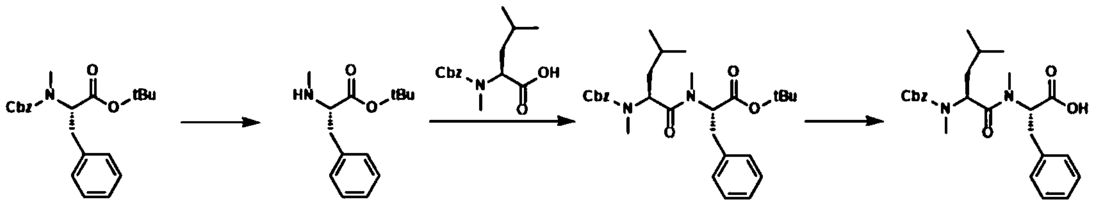

Cbz-MePhe-OtBu H-MePhe-OtBu Cbz-MeLeu-MePhe-OtBu Cbz-MeLeu-MePhe-OH

Cbz-MePhe-OtBu (6.50 g, 17.59 mmol) was dissolved in 2-MeTHF (50 mL) and added to a pressurized reaction vessel. 5% Pd/C (3.76 g, 0.88 mmol on Pd metal basis) was added to the pressurized reaction vessel, and nitrogen replacement and hydrogen replacement were performed, and the mixture was stirred under hydrogen pressure condition (3 to 4 atm) for 6 hours. The reaction mixture was filtered and Pd/C was washed with 2-MeTHF. The filtrate and the washing solution were combined and concentrated under reduced pressure condition to obtain H-MePhe-OtBu (4.13 g, 17.55 mmol). To a flask with a stirrer, Cbz-MeLeu-OH (4.62 g, 16.54 mmol) was added. To the flask, a solution of H-MePhe-OtBu in MeCN (concentration 0.10 g/mL, 37.0 mL, 3.70 g, 15.72 mmol) and NMM (5.2 mL, 47.20 mmol) were sequentially added. HATU (8.97 g, 23.58 mmol) was added to the reaction mixture, and the mixture was stirred continuously at room temperature. The stirring was continued for 1 hour, and the completion of the reaction was confirmed by HPLC analysis. To the reaction mixture, CPME (50.0 mL), 5% aqueous $K_2CO_3$ solution (50.0 mL), and NMI (1.25 mL, 15.72 mmol) were added, and the mixture was stirred continuously for 1.5 hours. All the contents of the flask were transferred to a separatory funnel and the aqueous layer was removed. The organic layer was washed 3 times with 2.5% aqueous ammonia solution (50 mL x 3), 2 times with 5% aqueous $NaHSO_4$ solution (50 mL x 2), and 1 time with 5% aqueous $Na_2CO_3$ solution (40 mL x 1). The obtained organic layer was dried over anhydrous Na2SO4. The desiccant was filtered off, and then the filtrate was concentrated under reduced pressure condition to obtain Cbz-MeLeu-Phe-OtBu (6.90 g, 13.89 mmol). To a flask with a stirrer, Cbz-MeLeu-MePhe-OtBu (6.90 g, 13.89 mmol), 2-MeTHF (70 mL), and HMDS (15.0 mL, 69.50 mmol) were added, and then TMSOTf (10.1 mL, 55.60 mmol) was added dropwise. The mixture was stirred continuously for 2 hours, and then HMDS (2.9 mL) and TMSOTf (2.5 mL) were added to the reaction mixture. The mixture was stirred continuously for additional 1.5 hours, and then HMDS (6.0 mL) and TMSOTf (5.0 mL) were added to the reaction mixture. The conversion rate was confirmed to reach 94% by HPLC analysis. The flask was cooled, and 5% aqueous $NaHCO_3$ solution (70 mL) was added dropwise to the reaction mixture while the reaction mixture was kept at 33°C or lower. All the contents of the flask were transferred to a separatory funnel, and 5% aqueous $NaHCO_3$ solution (30 mL) and 2-MeTHF (30 mL) were used for rinsing. The organic and aqueous layers were subjected to HPLC analysis, and then the organic layer was removed. To the resulting aqueous layer, 2-MeTHF (100 mL) was added, and 85% $H_3PO_4$ (7.5 mL) was gradually added. The aqueous layer was removed, and the resulting organic layer was washed with 5% aqueous NaCl solution (50 mL) and concentrated under reduced pressure condition. The resulting concentrate was purified by silica gel column chromatography to obtain Cbz-MeLeu-MePhe-OH (5.00 g, 11.35 mmol).

Yield: 72% (over 2 steps starting from H-MePhe-OtBu)

Purity: 100%
Measurement method: HPLC Method A, Retention time: 13.9 min
Mass spectrometry: m/z 441.78 ([M+H]$^+$)

(Raw material synthesis 2) Synthesis of Cbz-MeLeu-MePhe(4-F)-OH

[Formula 34]

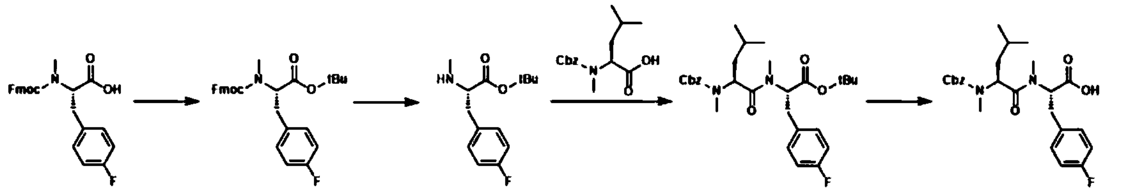

Fmoc-MePhe(4-F)-OH Fmoc-MePhe(4-F)-OtBu H-MePhe(-F)-OtBu Cbz-MeLeu-MePhe(4-F)-OtBu Cbz-MeLeu-MePhe(4-F)-OH

To a flask with a stirrer, Fmoc-MePhe(4-F)-OH (4.98 g, 11.87 mmol), cyclohexane (15 mL), and DCM (40 mL) were added. To the flask, tert-butyl 2,2,2-trichloroacetimidate (4.3 mL, 23.80 mmol) was added. A boron trifluoride diethyl ether complex (0.15 mL, 1.19 mmol) was added dropwise while the flask was cooled in an ice bath, and then the mixture was stirred continuously at room temperature for 4.5 hours. The completion of the reaction was confirmed by HPLC analysis, the reaction mixture was filtered, and the filtered solid was washed with cyclohexane. The filtrate and the wash solution were combined and washed 5 times with 10% aqueous citric acid solution (40 mL x 5). The resulting organic layer was washed twice with 5% aqueous $Na_2CO_3$ solution (40 mL x 2). The resulting organic layer was dried over anhydrous $Na_2SO_4$, the desiccant was filtered off, and then the filtrate was concentrated under reduced pressure condition to obtain Fmoc-MeLeu-MePhe(4-F)-OtBu (5.30 g, 11.10 mmol). To a flask with a stirrer, Fmoc-MeLeu-MePhe(4-F)-OtBu (5.30 g, 11.10 mmol) and MeCN (40 mL) were added. To the flask, DBU (1.7 mL, 11.10 mmol) was added and the mixture was stirred for 30 minutes. The completion of the reaction was confirmed by HPLC analysis, and TEA (6.2 mL, 44.60 mmol) and water (2.0 mL, 111.00 mmol) were added to the reaction mixture. The flask was cooled in an ice bath while sodium bisulfite (2.90 g, 27.90 mmol) was added, and the mixture was stirred at room temperature for 2 hours. To the reaction mixture, MTBE (100 mL) and 10% aqueous ammonia solution (50 mL) were added, and all the contents of the flask were transferred to a separatory funnel. The aqueous layer was removed, and the resulting organic layer was washed 3 times with 10% aqueous ammonia solution (50 mL x 3) and once with 5% aqueous NaCl solution (50 mL x 1). The resulting organic layer was concentrated under reduced pressure condition to obtain H-MePhe(4-F)-OtBu. To a flask with a stirrer, H-MePhe(4-F)-OtBu, MeCN (30 mL), Cbz-MeLeu-OH (3.41 g, 12.30 mmol), and NMM (3.7 mL, 33.40 mmol) were added. The flask was cooled in an ice bath while HATU (5.53 g, 14.50 mmol) was added, and the mixture was stirred continuously at room temperature for 2 hours. The completion of the reaction was confirmed by HPLC analysis, CPME (50 mL), 5% aqueous $K_2CO_3$ solution (30 mL), and NMI (0.88 mL, 11.10 mmol) were added to the reaction mixture, and the mixture was stirred continuously for 1.5 hours. All the contents of the flask were transferred to a separatory funnel and the aqueous layer was removed. The resulting organic layer was washed 3 times with 2.5% aqueous ammonia solution (50 mL x 3), 2 times with 5% aqueous $NaHSO_4$ solution (50 mL x 2), 1 time with 5% aqueous $Na_2CO_3$ solution (50 mL x 1), and 1 time with 5% aqueous NaCl solution (50 mL x 1). The obtained organic layer was dried over anhydrous Na2SO4. The desiccant was filtered off, and then the filtrate was concentrated under reduced pressure condition to obtain Cbz-MeLeu-MePhe(4-F)-OtBu (5.37 g, 10.40 mmol). To a flask with a stirrer, Cbz-MeLeu-MePhe(4-F)-OtBu (5.37 g, 10.40 mmol), 2-MeTHF (50 mL), and HMDS (10.9 mL, 52.20 mmol) were added, and then TMSOTf (7.5 mL, 41.70 mmol) was added dropwise. The mixture was stirred continuously for 2 hours, and then HMDS (5.0 mL) and TMSOTf (3.0 mL) were added to the reaction mixture. The mixture was stirred continuously for additional 4 hours, and the conversion rate was confirmed to reach 97% by HPLC analysis. The flask was cooled, and 5% aqueous $NaHCO_3$ solution (70 mL) was added dropwise to the reaction mixture while the reaction mixture was kept at 25°C or lower. All the contents of the flask were transferred to a separatory funnel, and 5% aqueous $NaHCO_3$ solution (30 mL) and 2-MeTHF (20 mL) were used for rinsing. The organic and aqueous layers were subjected to HPLC analysis, and then the organic layer was removed. To the resulting aqueous layer, 2-MeTHF (100 mL) was added, and 85% $H_3PO_4$ (10 mL) was gradually added. The aqueous layer was removed, and the resulting organic layer was washed with 5% aqueous NaCl solution (30 mL) and concentrated under reduced pressure condition. The resulting concentrate was purified by silica gel column chromatography to obtain Cbz-MeLeu-MePhe(4-F)-OH (4.00 g, 8.72 mmol).

Yield: 73% (over 4 steps starting from Fmoc-MePhe(4-F)-OH)
Purity: 98.3%
Measurement method: HPLC Method A, Retention time: 14.04 min
Mass spectrometry: m/z 459.80 ([M+H]$^+$)

(Raw material synthesis 3) Synthesis of Cbz-MeLeu-MeAla-OH

[Formula 35]

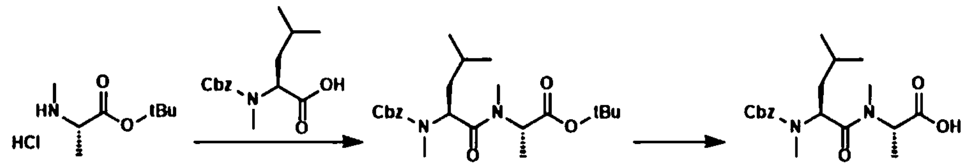

H-MeAla-OtBu-HCl Cbz-MeLeu-MeAla-OtBu Cbz-MeLeu-MeAla-OH

To a flask with a stirrer, Cbz-MeLeu-OH (10.50 g, 37.59 mmol), H-MeAla-OtBu·HCl (7.00 g, 35.77 mmol), 2-MeTHF (70 mL), MeCN (50 mL), and NMM (11.8 mL, 107 mmol) were added. The flask was cooled, HATU (20.40 g, 53.65 mmol) was added to the reaction mixture while the reaction mixture was kept at 30°C or lower, and the mixture was stirred continuously at room temperature. The stirring was continued for 3 hours, and the completion of the reaction was confirmed by HPLC analysis. To the reaction mixture, IPAC (50 mL), 5% aqueous $Na_2CO_3$ solution (70 mL), and NMI (2.84 mL, 35.81 mmol) were added, and the mixture was stirred continuously for 30 minutes. All the contents of the flask were transferred to a separatory funnel and the aqueous layer was removed. The organic layer was washed 3 times with 2.5% aqueous ammonia solution (50 mL x 3), 2 times with 5% aqueous $NaHSO_4$ solution (50 mL x 2), 1 time with 5% aqueous $Na_2CO_3$ solution (50 mL), and 1 time with 10% aqueous NaCl solution (50 mL). The obtained organic layer was dried over anhydrous $Na_2SO_4$, the desiccant was filtered off, and then the filtrate was concentrated under reduced pressure condition to obtain Cbz-MeLeu-MeAla-OtBu (14.35 g, 34.12 mmol). To the obtained concentrate, 2-MeTHF (140 mL) and HMDS (49.9 mL, 238.9 mmol) were added, and TMSOTf (37.7 mL, 204.7 mmol) was added dropwise. The stirring was continued for 3.5 hours, and the completion of the reaction was confirmed by HPLC analysis. The flask was cooled, and 5% aqueous $Na_2CO_3$ solution (70 mL) was added dropwise to the reaction mixture while the reaction mixture was kept at 30°C or lower. All the contents of the flask were transferred to a separatory funnel, 2-MeTHF (30 mL) and 5% aqueous $Na_2CO_3$ solution (30 mL) were added and the aqueous layer was removed. 2-MeTHF (100 mL) was added, and then 85% $H_3PO_4$ (15 mL) was gradually added. The aqueous layer was removed, and the organic layer was washed once with 10% aqueous NaCl solution (50 mL) and concentrated under reduced pressure condition. The resulting concentrate was purified by silica gel column chromatography to obtain Cbz-MeLeu-MeAla-OH (9.74 g).

Yield: 75% (over 2 steps)
Purity: 98.2%
Measurement method: HPLC Method A, Retention time: 11.3 min
Mass spectrometry: m/z 365.08 ([M+H]$^+$)

(Analytical standard synthesis 2) Synthesis of Cbz-MeLeu-D-MeAla-OH

[Formula 36]

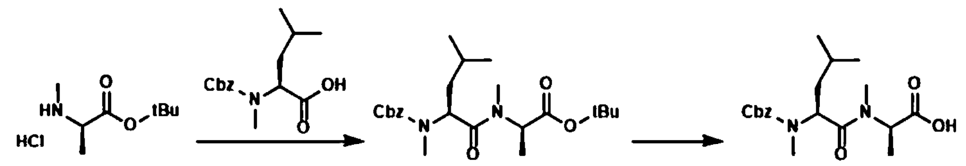

H-D-MeAla-OtBu-HCl Cbz-MeLeu-D-MeAla-OtBu Cbz-MeLeu-D-MeAla-OH

To a flask with a stirrer, Cbz-MeLeu-OH (2.25 g, 8.05 mmol), H-D-MeAla-OtBu·HCl (1.50 g, 7.67 mmol), MeCN (20 mL), and NMM (2.53 mL, 23.0 mmol) were added. The flask was cooled, HATU (4.37 g, 11.5 mmol) was added to the reaction mixture while the reaction mixture was kept at 30°C or lower, and the mixture was stirred continuously at room temperature. The stirring was continued for 6 hours, and the completion of the reaction was confirmed by HPLC analysis. To the reaction mixture, IPAC (20 mL), 5% aqueous $Na_2CO_3$ solution (20 mL), and NMI (0.608 mL, 7.67 mmol) were added, and the mixture was stirred continuously for 30 minutes. All the contents of the flask were transferred to a separatory funnel and the aqueous layer was removed. The organic layer was washed 3 times with 2.5% aqueous ammonia solution (15 mL x 3), 2 times with 5% aqueous $NaHSO_4$ solution (15 mL x 2), 1 time with 5% aqueous $Na_2CO_3$ solution (15 mL), and 1 time with 10% aqueous NaCl solution (15 mL). The obtained organic layer was dried over anhydrous Na2SO4. The desiccant was filtered off, and then the filtrate was concentrated under reduced pressure condition to obtain Cbz-MeLeu-D-MeAla-OtBu (3.18 g, 7.56 mmol). To the obtained concentrate, 2-MeTHF (30 mL) and HMDS (11.1 mL, 52.9 mmol) were added, and TMSOTf (8.36 mL, 45.4 mmol) was added dropwise. The stirring was continued for 4 hours, and the completion of the reaction was confirmed by HPLC analysis. The flask was cooled, and 5% aqueous $Na_2CO_3$ solution (15 mL) was added dropwise to the reaction mixture while the reaction mixture was kept at 30°C or lower. All the contents of the flask were transferred to a separatory funnel, 2-MeTHF (10 mL) and 5% aqueous $Na_2CO_3$ solution (10 mL) were added and the aqueous layer was removed. 2-MeTHF (20 mL) was added, and then 85% $H_3PO_4$ (3.0 mL) was gradually added. The aqueous layer was removed, and the organic layer was washed once with 10% aqueous NaCl solution (15 mL) and concentrated under reduced pressure condition. The resulting concentrate was purified by silica gel column chromatography to obtain Cbz-MeLeu-D-MeAla-OH (2.54 g).

Yield: 91% (over 2 steps)
Purity: 99.7%
Measurement method: HPLC Method A, Retention time: 11.4 min
Mass spectrometry: m/z 365.20 ($[M+H]^+$)

(Raw material synthesis 4) Synthesis of H-MeAla-Aze-EtPhe(4-Me)-Sar-OtBu

[Formula 37]

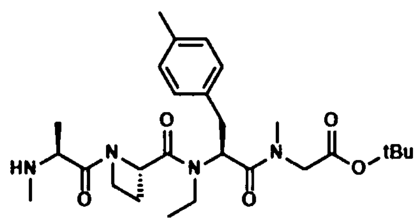

**H-MeAla-Aze-EtPhe(4-Me)-Sar-OtBu**

The compound was synthesized in accordance with the method described in International Publication No. WO2023/127869.

(Raw material synthesis 5) Synthesis of H-MeLeu-Ile-MeAla-Aze-EtPhe(4-Me)-Sar-OtBu

[Formula 38]

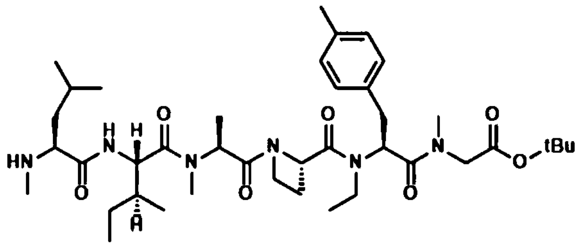

H-MeLeu-Ile-MeAla-Aze-EtPhe(4-Me)-Sar-OtBu

The compound was synthesized by desalting an L-tartrate salt of the title compound obtained by the method described in International Publication No. WO 2023/127869.

(Raw material synthesis 6) Synthesis of H-MeGly(cPent)-MeAsp(OtBu)-$NMe_2$

[Formula 39]

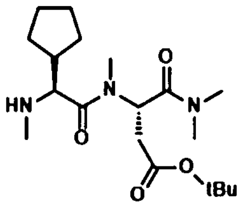

H-MeGly(cPent)-MeAsp(OtBu)-$NMe_2$

The compound was synthesized by desalting a hydrochloride of the title compound obtained by the method described in International Publication No. WO 2023/127869.

(Raw material synthesis 7) Synthesis of Cbz-Gly-MePhe-OH

[Formula 40]

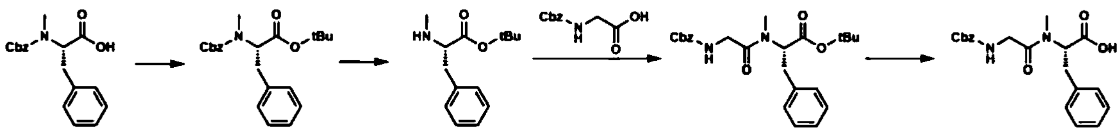

Cbz-MePhe-OH Cbz-MePhe-OtBu H-MePhe-OtBu Cbz-Gly-MePhe-OtBu Cbz-Gly-MePhe-OH

To a flask with a stirrer, Cbz-MePhe-OH (10.00 g, 31.90 mmol), cyclohexane (80 mL), and DCM (30 mL) were added. To the flask, tert-butyl 2,2,2-trichloroacetimidate (11.4 mL, 63.70 mmol) was added. A boron trifluoride diethyl ether complex (0.4 mL, 3.19 mmol) was added dropwise while the flask was cooled in an ice bath, and then the mixture was stirred continuously at room temperature for 2 hours. To the flask, tert-butyl 2,2,2-trichloroacetimidate (3.0 mL, 16.76 mmol) was added, and the mixture was stirred continuously for an additional 30 minutes. The reaction mixture was filtered, and the filtered solid was washed with cyclohexane. The filtrate and the wash solution were combined and washed 5 times with 10% aqueous citric acid solution (50 mL × 5). The resulting organic layer was washed 2 times with 5% aqueous $Na_2CO_3$ solution (50 mL × 2). The resulting organic layer was concentrated under reduced pressure condition. The resulting concentrate was purified by silica gel column chromatography to obtain Cbz-MePhe-OtBu (9.84 g, 26.6 mmol). To a pressure-resistant reaction vessel with a stirrer, Cbz-MePhe-OtBu (9.50 g, 25.70 mmol), 2-MeTHF (70 mL), and 5% Pd/C (5.50 g, 1.29 mmol on Pd metal basis) were sequentially added. The nitrogen replacement and hydrogen replacement in the reaction vessel were performed, and the reaction mixture was stirred under a hydrogen atmosphere

(3 to 4 atm) for 5 hours. The reaction mixture was filtered and Pd/C was washed with 2-MeTHF. The filtrate and the washing solution were combined and concentrated under reduced pressure condition to obtain H-MePhe-OtBu (6.02 g, 25.60 mmol). To a flask with a stirrer, H-MePhe-OtBu (1.33 g, 5.52 mmol), MeCN (16.0 mL), Cbz-Gly-OH (1.29 g, 6.08 mmol) and NMM (2.4 mL, 21.80 mmol) were sequentially added. HATU (4.20 g, 11.05 mmol) was added to the reaction mixture while the flask was cooled in an ice bath, and the mixture was stirred continuously at room temperature for 2 hours. The completion of the reaction was confirmed by HPLC analysis, CPME (20 mL), 5% aqueous $Na_2CO_3$ solution (15 mL), and NMI (0.45 mL, 5.65 mmol) were sequentially added to the reaction mixture, and the mixture was stirred continuously for 2 hours. All the contents of the flask were transferred to a separatory funnel and the aqueous layer was removed. The organic layer was washed 3 times with 2.5% aqueous ammonia solution (25 mL × 3), 2 times with 5% aqueous $NaHSO_4$ solution (25 mL × 2), and 1 time with 5% aqueous $K_2CO_3$ solution (25 mL × 1). The resulting organic layer was dried over anhydrous $Na_2SO_4$. The desiccant was filtered off, and then the filtrate was concentrated under reduced pressure condition to obtain Cbz-MeLeu-MePhe-OtBu (2.36 g, 5.52 mmol). To a flask with a stirrer, Cbz-Gly-MePhe-OtBu (2.36 g, 5.52 mmol), 2-MeTHF (25.0 mL), and HMDS (6.1 mL, 29.10 mmol) were added, and then TMSOTf (4.3 mL, 23.76 mmol) was added dropwise. The mixture was stirred continuously for 1.5 hours. During this time, 2-MeTHF (10.0 mL) was added to the reaction mixture to improve fluidity. After confirming the completion of the reaction by HPLC analysis, the flask was cooled, and a 5% aqueous $NaHCO_3$ solution (25 mL) was added dropwise to the reaction mixture while the reaction mixture was kept at 25°C or lower. All the contents of the flask were transferred to a separatory funnel. 2-MeTHF (50 mL) was added to the separatory funnel, and 85% $H_3PO_4$ (3.0 mL) was gradually added. The aqueous layer was removed, and the resulting organic layer was washed with 5% aqueous NaCl solution (30 mL) and concentrated under reduced pressure condition. The resulting concentrate was purified by silica gel column chromatography to obtain Cbz-Gly-MePhe-OH (1.77 g, 4.78 mmol).

Purity: 100.0%
Measurement method: HPLC Method A, Retention time: 11.3 min
Mass spectrometry: m/z 371.62 ($[M+H]^+$)

(Raw material synthesis 8) Synthesis of Cbz-MeAla-MeLeu-OH

[Formula 41]

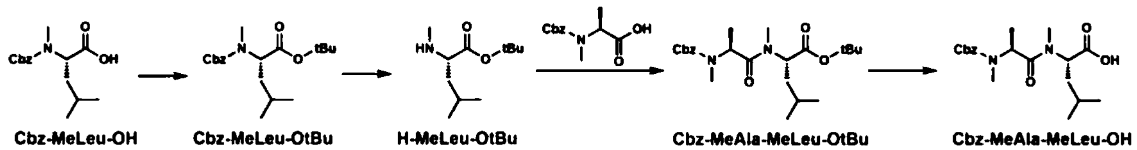

To a flask with a stirrer, Cbz-MePhe-OH (2.00 g, 7.20 mmol), cyclohexane (16 mL), and DCM (6 mL) were added. To the flask, tert-butyl 2,2,2-trichloroacetimidate (2.6 mL, 14.53 mmol) was added. A boron trifluoride diethyl ether complex (90 μL, 0.72 mmol) was added dropwise while the flask was cooled in an ice bath, and then the mixture was stirred continuously at room temperature for 1 hour. The completion of the reaction was confirmed by HPLC analysis, the reaction mixture was filtered, and the filtered solid was washed with cyclohexane. The filtrate and the wash solution were combined and washed 5 times with 10% aqueous citric acid solution (16 mL × 5). The resulting organic layer was washed 2 times with 5% aqueous $Na_2CO_3$ solution (16 mL × 2). The resulting organic layer was concentrated under reduced pressure conditions to obtain Cbz-MeLeu-OtBu (2.38 g, 7.10 mmol). To a flask with a stirrer, Cbz-MePhe-OtBu (2.38 g, 7.10 mmol), 2-MeTHF (24 mL), and 5% Pd/C (1.51 g, 0.36 mmol on Pd metal basis) were sequentially added. The nitrogen replacement and hydrogen replacement in the flask were performed, and the reaction mixture was stirred under a hydrogen atmosphere (1 atm) for 1 hour. The reaction mixture was filtered and Pd/C was washed with 2-MeTHF. The filtrate and the washing solution were combined and concentrated under reduced pressure condition to obtain H-MeLeu-OtBu (1.39 g, 6.90 mmol). To a flask with a stirrer, H-MeLeu-OtBu (1.39 g, 6.90 mmol), MeCN (14.0 mL), Cbz-MeAla-OH (1.80 g, 7.60 mmol) and NMM (1.5 mL, 13.64 mmol) were sequentially added. COMU (3.55 g, 8.29 mmol) was added to the reaction mixture while the flask was cooled in an ice bath, and the mixture was stirred continuously at room temperature for 3 hours. The completion of the reaction was confirmed by HPLC analysis, 2-MeTHF (42 mL), 5% aqueous $K_2CO_3$ solution (21 mL), and NMI (0.55 mL, 6.90 mmol) were sequentially added to the reaction mixture, and the mixture was stirred continuously for 1 hour. All the contents of the flask were transferred to a separatory funnel and the aqueous layer was removed. The organic layer was washed 1 time with 5% aqueous $K_2CO_3$ solution (35 mL × 1), 3 times with 2.5% aqueous ammonia solution (35

mL × 3), 2 times with 5% aqueous $NaHSO_4$ solution (35 mL × 2), 1 time with 5% aqueous $K_2CO_3$ solution (35 mL × 1), and 1 time with 5% aqueous NaCl solution (35 mL × 1). The resulting organic layer was dried over anhydrous $Na_2SO_4$. The desiccant was filtered off, and then the filtrate was concentrated under reduced pressure condition to obtain Cbz-MeAla-MeLeu-OtBu (2.70 g, 6.42 mmol). To a flask with a stirrer, Cbz-MeAla-MeLeu-OtBu (2.70 g, 6.42 mmol), 2-MeTHF (27.0 mL), and HMDS (5.8 mL, 27.67 mmol) were added, and then TMSOTf (3.6 mL, 19.89 mmol) was added dropwise. The mixture was stirred continuously for 3 hours, and then HMDS (2.9 mL, 13.84 mmol) and TMSOTf (1.8 mL, 9.95 mmol) were added to the reaction mixture. The mixture was stirred continuously for an additional 1.5 hours, and then HMDS (1.4 mL, 6.68 mmol) and TMSOTf (0.95 mL, 5.25 mmol) were added to the reaction mixture. After confirming that the conversion rate reached 93% by HPLC analysis, the flask was cooled and a 5% aqueous $NaHCO_3$ solution (32 mL) was added dropwise while the reaction mixture was kept at 13°C or lower. All the contents of the flask were transferred to a separatory funnel and the organic layer was removed. To the resulting aqueous layer, 2-MeTHF (65 mL) was added, and 85% $H_3PO_4$ (4.8 mL) was gradually added. The aqueous layer was removed, and the resulting organic layer was washed with 5% aqueous NaCl solution (32 mL) and concentrated under reduced pressure condition. The resulting concentrate was purified by silica gel column chromatography to obtain Cbz-MeAla-MeLeu-OH (1.73 g, 4.74 mmol).

Purity: 99.3%
Measurement method: HPLC Method A, Retention time: 12.6 min
Mass spectrometry: m/z 365.64 ($[M+H]^+$)

(Raw material synthesis 9) Synthesis of Cbz-MeLeu-MePhe-OH

[Formula 42]

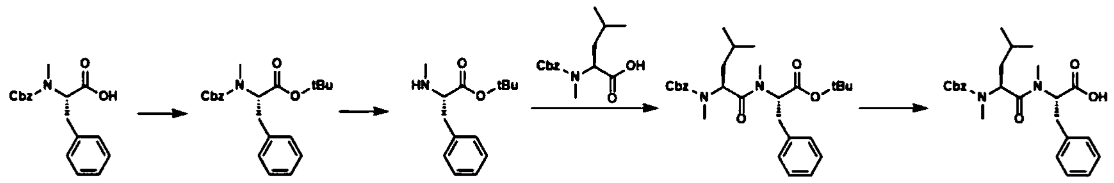

Cbz-MePhe-OH Cbz-MePhe-OtBu H-MePhe-OtBu Cbz-MeLeu-MePhe-OtBu Cbz-MeLeu-MePhe-OH

To a flask with a stirrer, Cbz-MePhe-OH (4.50 g, 14.00 mmol), cyclohexane (36 mL), and DCM (14 mL) were added. To the flask, tert-butyl 2,2,2-trichloroacetimidate (5.0 mL, 27.94 mmol) was added. A boron trifluoride diethyl ether complex (0.18 mL, 1.43 mmol) was added dropwise while the flask was cooled in an ice bath, and then the mixture was stirred continuously at room temperature for 1.5 hours. The completion of the reaction was confirmed by HPLC analysis, the reaction mixture was filtered, and the filtered solid was washed with cyclohexane. The filtrate and the wash solution were combined and washed 5 times with 10% aqueous citric acid solution (36 mL × 5). The resulting organic layer was washed 2 times with 5% aqueous $Na_2CO_3$ solution (36 mL × 2). The resulting organic layer was concentrated under reduced pressure conditions to obtain Cbz-MePhe-OtBu (5.18 g, 14.00 mmol). To a flask with a stirrer, Cbz-MePhe-OtBu (5.18 g, 14.00 mmol), 2-MeTHF (52 mL), and 5% Pd/C (2.98 g, 0.70 mmol on Pd metal basis) were sequentially added. The nitrogen replacement and hydrogen replacement in the flask were performed, and the reaction mixture was stirred under a hydrogen atmosphere (1 atm) for 2 hours. The reaction mixture was filtered and Pd/C was washed with 2-MeTHF. The filtrate and the washing solution were combined and concentrated under reduced pressure condition to obtain H-MePhe-OtBu (3.20 g, 13.60 mmol). To a flask with a stirrer, H-MePhe-OtBu (3.20 g, 13.60 mmol), MeCN (32.0 mL), Cbz-MeLeu-OH (4.19 g, 15.00 mmol) and NMM (4.5 mL, 40.93 mmol) were sequentially added. COMU (7.57 g, 17.68 mmol) was added to the reaction mixture while the flask was cooled in an ice bath, and the mixture was stirred continuously at room temperature for 4 hours. The completion of the reaction was confirmed by HPLC analysis, 2-MeTHF (96 mL), 5% aqueous $K_2CO_3$ solution (48 mL), and NMI (1.1 mL, 13.80 mmol) were sequentially added to the reaction mixture, and the mixture was stirred continuously for 30 minutes. All the contents of the flask were transferred to a separatory funnel and the aqueous layer was removed. The organic layer was washed 1 time with 5% aqueous $K_2CO_3$ solution (80 mL × 1), 3 times with 2.5% aqueous ammonia solution (80 mL × 3), 2 times with 5% aqueous $NaHSO_4$ solution (80 mL × 2), 1 time with 5% aqueous $K_2CO_3$ solution (80 mL × 1), and 1 time with 5% aqueous NaCl solution (80 mL × 1). The resulting organic layer was dried over anhydrous $Na_2SO_4$. The desiccant was filtered off, and then the filtrate was concentrated under reduced pressure condition to obtain Cbz-MeLeu-MePhe-OtBu (7.14 g, 14.40 mmol). To a flask with a stirrer, Cbz-MeLeu-MePhe-OtBu (6.75 g, 13.60 mmol), 2-MeTHF (68.0 mL), and HMDS (20.0 mL, 95.42 mmol) were added, and then TMSOTf (15.0 mL, 82.88 mmol) was added dropwise. The mixture was stirred continuously for 5.5 hours, and then the conversion rate

was confirmed to reach 95% by HPLC analysis. The flask was cooled, and 5% aqueous $NaHCO_3$ solution (68 mL) was added dropwise to the reaction mixture while the reaction mixture was kept at 20°C or lower. All the contents of the flask were transferred to a separatory funnel and the organic layer was removed. To the resulting aqueous layer, 2-MeTHF (100 mL) was added, and 85% $H_3PO_4$ (10.0 mL) was gradually added. The aqueous layer was removed, and the resulting organic layer was washed with 5% aqueous NaCl solution (68 mL) and concentrated under reduced pressure condition. The resulting concentrate was purified by silica gel column chromatography to obtain Cbz-MeLeu-MePhe-OH (5.08 g, 11.50 mmol).

Purity: 99.2%
Measurement method: HPLC Method A, Retention time: 13.4 min
Mass spectrometry: m/z 441.33 ($[M+H]^+$)

(Raw material synthesis 10) Synthesis of Cbz-MeLeu-D-MePhe-OH

[Formula 43]

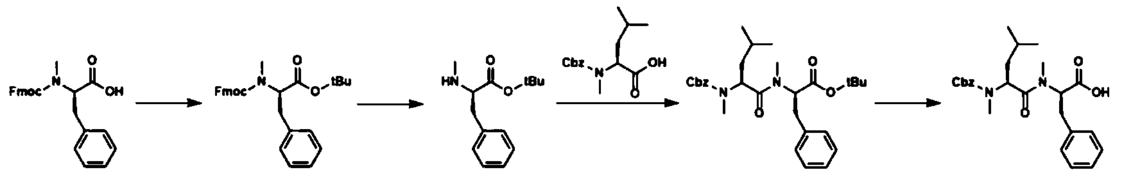

Fmoc-D-MePhe-OH Fmoc-D-MePhe-OtBu H-D-MePhe-OtBu Cbz-MeLeu-D-MePhe-OtBu Cbz-MeLeu-D-MePhe-OH

To a flask with a stirrer, Fmoc-D-MePhe-OH (2.01 g, 4.98 mmol), cyclohexane (16 mL), and DCM (22 mL) were added. To the flask, tert-butyl 2,2,2-trichloroacetimidate (0.9 mL, 5.03 mmol) was added. A boron trifluoride diethyl ether complex (0.06 mL, 0.478 mmol) was added dropwise while the flask was cooled in an ice bath, and then the mixture was stirred continuously at room temperature for 1 hour. To the flask, tert-butyl 2,2,2-trichloroacetimidate (0.9 mL, 5.03 mmol) was added, and the mixture was stirred continuously for 2 hours. To the flask, tert-butyl 2,2,2-trichloroacetimidate (0.9 mL, 5.03 mmol) was added, and the mixture was stirred continuously for 1 hour. The completion of the reaction was confirmed by HPLC analysis, the reaction mixture was filtered, and the filtered solid was washed with cyclohexane. The filtrate and the wash solution were combined and washed 5 times with 10% aqueous citric acid solution (14 mL × 5). The resulting organic layer was washed 2 times with 5% aqueous $Na_2CO_3$ solution (14 mL × 2). The resulting organic layer was dried over anhydrous $Na_2SO_4$. The desiccant was filtered off, and then the filtrate was concentrated under reduced pressure condition to obtain Fmoc-D-MePhe-OtBu (2.79 g, 6.09 mmol). To a flask with a stirrer, Fmoc-D-MePhe-OtBu (2.28 g, 4.98 mmol) and MeCN (18 mL) were added. To the flask, DBU (0.74 mL, 4.95 mmol) was added and the mixture was stirred for 1 hour. The completion of the reaction was confirmed by HPLC analysis, and TEA (2.8 mL, 20.09 mmol) and water (1.0 mL, 55.49 mmol) were added to the reaction mixture. Sodium bisulfite (1.30 g, 12.49 mmol) was added while the flask was cooled in an ice bath, and the mixture was stirred at room temperature for 1 hour. To the reaction mixture, MTBE (60 mL) and 10% aqueous ammonia solution (30 mL) were added, and all the contents of the flask were transferred to a separatory funnel. The aqueous layer was removed, and the resulting organic layer was washed 3 times with 10% aqueous ammonia solution (30 mL × 3) and 1 time with 5% aqueous NaCl solution (15 mL × 1). The resulting organic layer was concentrated under reduced pressure condition to obtain H-D-MePhe-OtBu (0.98 g). To a flask with a stirrer, H-D-MePhe-OtBu (0.98 g), MeCN (12 mL), Cbz-MeLeu-OH (1.29 g, 4.60 mmol) and NMM (1.4 mL, 12.73 mmol) were added. COMU (2.33 g, 5.44 mmol) was added to the reaction mixture while the flask was cooled in an ice bath, and the reaction mixture was stirred continuously at room temperature for 2 hours. The completion of the reaction was confirmed by HPLC analysis, 2-MeTHF (30 mL), 5% aqueous $K_2CO_3$ solution (15 mL), and NMI (0.33 mL, 4.14 mmol) were added to the reaction mixture, and the mixture was stirred continuously for 30 minutes. All the contents of the flask were transferred to a separatory funnel and the aqueous layer was removed. The resulting organic layer was washed 1 time with 5% aqueous $K_2CO_3$ solution (25 mL × 1), 3 times with 2.5% aqueous ammonia solution (25 mL × 3), 2 times with 5% aqueous $NaHSO_4$ solution (25 mL × 2), 1 time with 5% aqueous $K_2CO_3$ solution (25 mL × 1), and 1 time with 5% aqueous NaCl solution (50 mL × 1). The resulting organic layer was dried over anhydrous $Na_2SO_4$. The desiccant was filtered off, and then the filtrate was concentrated under reduced pressure condition to obtain Cbz-MeLeu-D-MePhe-OtBu (2.05 g, 4.12 mmol). To a flask with a stirrer, Cbz-MeLeu-D-MePhe-OtBu (2.05 g, 4.12 mmol), 2-MeTHF (20 mL), and HMDS (6.0 mL, 28.63 mmol) were added, and then TMSOTf (4.6 mL, 25.42 mmol) was added dropwise. The mixture was stirred continuously for 4.5 hours, and then the

completion of the reaction was confirmed by HPLC analysis. The flask was cooled, and 5% aqueous $NaHCO_3$ solution (20 mL) was added dropwise to the reaction mixture while the reaction mixture was kept at 18°C or lower. All the contents of the flask were transferred to a separatory funnel and the organic layer was removed. To the resulting aqueous layer, 2-MeTHF (30 mL) was added, and 85% $H_3PO_4$ (3.0 mL) was gradually added. The aqueous layer was removed, and the resulting organic layer was washed with 5% aqueous NaCl solution (20 mL) and concentrated under reduced pressure condition. The resulting concentrate was purified by silica gel column chromatography to obtain Cbz-MeLeu-D-MePhe-OH (1.36 g, 3.09 mmol).

Purity: 99.2%
Measurement method: HPLC Method A, Retention time: 13.6 min
Mass spectrometry: m/z 441.28 ($[M+H]^+$)

(Raw material synthesis 11) Synthesis of Cbz-Leu-MePhe-OH

[Formula 44]

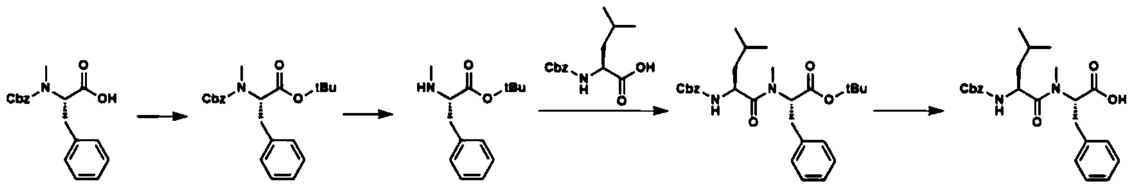

Cbz-MePhe-OH Cbz-MePhe-OtBu H-MePhe-OtBu Cbz-Leu-MePhe-OtBu Cbz-Leu-MePhe-OH

To a flask with a stirrer, Cbz-MePhe-OH (4.98 g, 16.00 mmol), cyclohexane (40 mL), and DCM (15 mL) were added. To the flask, tert-butyl 2,2,2-trichloroacetimidate (5.8 mL, 32.41 mmol) was added. A boron trifluoride diethyl ether complex (0.20 mL, 1.59 mmol) was added dropwise while the flask was cooled in an ice bath, and then the mixture was stirred continuously at room temperature for 2.5 hours. To the reaction mixture, tert-butyl 2,2,2-trichloroacetimidate (1.4 mL, 7.82 mmol) and a boron trifluoride diethyl ether complex (0.20 mL, 1.59 mmol) were added, and the mixture was stirred continuously for 1 hour. The completion of the reaction was confirmed by HPLC analysis, the reaction mixture was filtered, and the filtered solid was washed with cyclohexane. The filtrate and the wash solution were combined and washed 5 times with 10% aqueous citric acid solution (40 mL × 5). The resulting organic layer was washed 2 times with 5% aqueous $Na_2CO_3$ solution (40 mL × 2). The resulting organic layer was concentrated under reduced pressure conditions to obtain Cbz-MePhe-OtBu (5.02 g, 13.59 mmol). To a flask with a stirrer, Cbz-MePhe-OtBu (5.02 g, 13.59 mmol), 2-MeTHF (50 mL), and 5% Pd/C (2.88 g, 0.68 mmol on Pd metal basis) were sequentially added. The nitrogen replacement and hydrogen replacement in the flask were performed, and the reaction mixture was stirred under a hydrogen atmosphere (1 atm) for 1.5 hours. The reaction mixture was filtered and Pd/C was washed with 2-MeTHF. The filtrate and the washing solution were combined and concentrated under reduced pressure condition to obtain H-MePhe-OtBu (3.19 g, 13.55 mmol). To a flask with a stirrer, H-MePhe-OtBu (2.01 g, 8.50 mmol), MeCN (20.0 mL), Cbz-Leu-OH (2.48 g, 9.35 mmol) and NMM (1.9 mL, 17.28 mmol) were sequentially added. COMU (4.37 g, 10.20 mmol) was added to the reaction mixture while the flask was cooled in an ice bath, and the mixture was stirred continuously at room temperature for 3 hours. The completion of the reaction was confirmed by HPLC analysis, 2-MeTHF (30.0 mL), 5% aqueous $K_2CO_3$ solution (20.0 mL), and NMI (0.67 mL, 8.41 mmol) were sequentially added to the reaction mixture, and the mixture was stirred continuously for 45 minutes. All the contents of the flask were transferred to a separatory funnel and the aqueous layer was removed. The organic layer was washed 3 times with 2.5% aqueous ammonia solution (25 mL × 3), 2 times with 5% aqueous $NaHSO_4$ solution (25 mL × 2), and 1 time with 5% aqueous $Na_2CO_3$ solution (25 mL × 1). The resulting organic layer was dried over anhydrous $Na_2SO_4$. The desiccant was filtered off, and then the filtrate was concentrated under reduced pressure condition to obtain Cbz-Leu-MePhe-OtBu (4.13 g, 8.56 mmol). To a flask with a stirrer, Cbz-Leu-MePhe-OtBu (4.13 g, 8.56 mmol), 2-MeTHF (41.0 mL), and HMDS (13.0 mL, 62.02 mmol) were added, and then TMSOTf (9.5 mL, 52.49 mmol) was added dropwise. The mixture was stirred continuously for 2 hours, and then the conversion rate was confirmed to reach 94% by HPLC analysis. The flask was cooled, and 5% aqueous $NaHCO_3$ solution (40 mL) was added dropwise to the reaction mixture while the reaction mixture was kept at 23°C or lower. All the contents of the flask were transferred to a separatory funnel and the organic layer was removed. To the resulting aqueous layer, 2-MeTHF (50 mL) was added, and 85% $H_3PO_4$ (4.5 mL) was gradually added. The aqueous layer was removed, and the resulting organic layer was washed with 5% aqueous NaCl solution (30 mL) and concentrated under reduced pressure condition. The resulting concentrate was purified by silica gel column chromatography to obtain Cbz-Leu-MePhe-OH (2.49 g, 5.84 mmol).

Purity: 98.7%
Measurement method: HPLC Method A, Retention time: 12.7 min
Mass spectrometry: m/z 427.71 ($[M+H]^+$)

(Raw material synthesis 12) Synthesis of Cbz-D-Leu-MePhe-OH

[Formula 45]

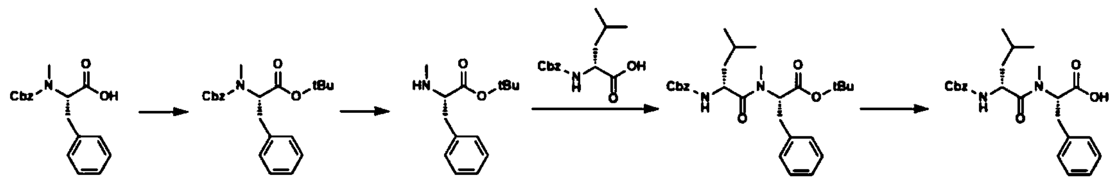

Cbz-MePhe-OH Cbz-MePhe-OtBu H-MePhe-OtBu Cbz-D-Leu-MePhe-OtBu Cbz-D-Leu-MePhe-OH

To a flask with a stirrer, Cbz-MePhe-OH (4.98 g, 16.00 mmol), cyclohexane (40 mL), and DCM (15 mL) were added. To the flask, tert-butyl 2,2,2-trichloroacetimidate (5.8 mL, 32.41 mmol) was added. A boron trifluoride diethyl ether complex (0.20 mL, 1.59 mmol) was added dropwise while the flask was cooled in an ice bath, and then the mixture was stirred continuously at room temperature for 2.5 hours. To the reaction mixture, tert-butyl 2,2,2-trichloroacetimidate (1.4 mL, 7.82 mmol) and a boron trifluoride diethyl ether complex (0.20 mL, 1.59 mmol) were added, and the mixture was stirred continuously for 1 hour. The completion of the reaction was confirmed by HPLC analysis, the reaction mixture was filtered, and the filtered solid was washed with cyclohexane. The filtrate and the wash solution were combined and washed 5 times with 10% aqueous citric acid solution (40 mL × 5). The resulting organic layer was washed 2 times with 5% aqueous $Na_2CO_3$ solution (40 mL × 2). The resulting organic layer was concentrated under reduced pressure conditions to obtain Cbz-MePhe-OtBu (5.02 g, 13.59 mmol). To a flask with a stirrer, Cbz-MePhe-OtBu (5.02 g, 13.59 mmol), 2-MeTHF (50 mL), and 5% Pd/C (2.88 g, 0.68 mmol on Pd metal basis) were sequentially added. The nitrogen replacement and hydrogen replacement in the flask were performed, and the reaction mixture was stirred under a hydrogen atmosphere (1 atm) for 1.5 hours. The reaction mixture was filtered and Pd/C was washed with 2-MeTHF. The filtrate and the washing solution were combined and concentrated under reduced pressure condition to obtain H-MePhe-OtBu (3.19 g, 13.55 mmol). To a flask with a stirrer, H-MePhe-OtBu (1.16 g, 4.93 mmol), MeCN (12.0 mL), Cbz-D-Leu-OH (1.44 g, 5.42 mmol) and NMM (1.1 mL, 10.01 mmol) were sequentially added. COMU (2.53 g, 5.91 mmol) was added to the reaction mixture while the flask was cooled in an ice bath, and the mixture was stirred continuously at room temperature for 2 hours. The completion of the reaction was confirmed by HPLC analysis, 2-MeTHF (20.0 mL), 5% aqueous $K_2CO_3$ solution (20.0 mL), and NMI (0.39 mL, 4.89 mmol) were sequentially added to the reaction mixture, and the mixture was stirred continuously for 30 minutes. All the contents of the flask were transferred to a separatory funnel and the aqueous layer was removed. The organic layer was washed 1 time with 5% aqueous $Na_2CO_3$ solution (15 mL × 1), 3 times with 2.5% aqueous ammonia solution (15 mL × 3), 2 times with 5% aqueous $NaHSO_4$ solution (15 mL × 2), and 1 time with 5% aqueous $Na_2CO_3$ solution (15 mL × 1). The resulting organic layer was dried over anhydrous $Na_2SO_4$. The desiccant was filtered off, and then the filtrate was concentrated under reduced pressure condition to obtain Cbz-D-Leu-MePhe-OtBu (2.47 g, 5.12 mmol). To a flask with a stirrer, Cbz-D-Leu-MePhe-OtBu (2.47 g, 5.12 mmol), 2-MeTHF (25.0 mL), and HMDS (7.5 mL, 35.78 mmol) were added, and then TMSOTf (5.6 mL, 30.94 mmol) was added dropwise. The mixture was stirred continuously for 3 hours, and then the conversion rate was confirmed to reach 97% by HPLC analysis. The flask was cooled, and 5% aqueous $NaHCO_3$ solution (30 mL) was added dropwise to the reaction mixture while the reaction mixture was kept at 24°C or lower. All the contents of the flask were transferred to a separatory funnel and the organic layer was removed. To the resulting aqueous layer, 2-MeTHF (30 mL) was added, and 85% $H_3PO_4$ (2.5 mL) was gradually added. The aqueous layer was removed, and the resulting organic layer was washed with 5% aqueous NaCl solution (20 mL) and concentrated under reduced pressure condition. The resulting concentrate was purified by silica gel column chromatography to obtain Cbz-D-Leu-MePhe-OH (1.44 g, 3.38 mmol).

Purity: 99.1%
Measurement method: HPLC Method A, Retention time: 12.9 min
Mass spectrometry: m/z 427.71 ($[M+H]^+$)

(Raw material synthesis 13) Synthesis of Cbz-Leu-D-MePhe-OH

[Formula 46]

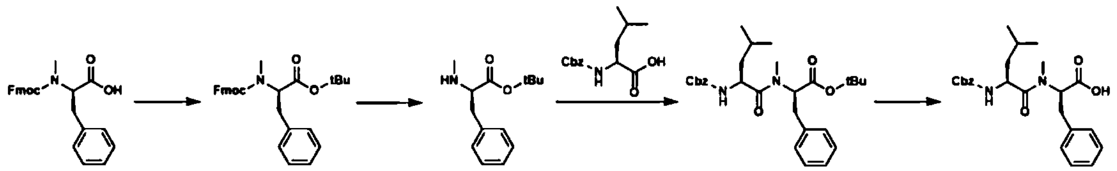

Fmoc-D-MePhe-OH Fmoc-D-MePhe-OtBu H-D-MePhe-OtBu Cbz-Leu-D-MePhe-OtBu Cbz-Leu-D-MePhe-OH

To a flask with a stirrer, Fmoc-D-MePhe-OH (2.00 g, 4.98 mmol), cyclohexane (16 mL) and DCM (6 mL) were added. To the flask, tert-butyl 2,2,2-trichloroacetimidate (2.7 mL, 15.09 mmol) was added. A boron trifluoride diethyl ether complex (0.06 mL, 0.478 mmol) was added dropwise while the flask was cooled in an ice bath, and then the mixture was stirred continuously at room temperature for 1 hour. The completion of the reaction was confirmed by HPLC analysis, the reaction mixture was filtered, and the filtered solid was washed with cyclohexane. The filtrate and the wash solution were combined and washed 5 times with 10% aqueous citric acid solution (14 mL × 5). The resulting organic layer was washed 2 times with 5% aqueous $Na_2CO_3$ solution (14 mL × 2). The resulting organic layer was dried over anhydrous $Na_2SO_4$. The desiccant was filtered off, and then the filtrate was concentrated under reduced pressure condition to obtain Fmoc-D-MePhe-OtBu (2.62 g, 5.72 mmol). To a flask with a stirrer, Fmoc-D-MePhe-OtBu (2.28 g, 4.98 mmol) and MeCN (18 mL) were added. To the flask, DBU (0.74 mL, 4.95 mmol) was added and the mixture was stirred for 1 hour. The completion of the reaction was confirmed by HPLC analysis, and TEA (2.8 mL, 20.09 mmol) and water (1.0 mL, 55.49 mmol) were added to the reaction mixture. Sodium bisulfite (1.30 g, 12.49 mmol) was added while the flask was cooled in an ice bath, and the mixture was stirred at room temperature for 1 hour. To the reaction mixture, MTBE (60 mL) and 10% aqueous ammonia solution (30 mL) were added, and all the contents of the flask were transferred to a separatory funnel. The aqueous layer was removed, and the resulting organic layer was washed 3 times with 10% aqueous ammonia solution (30 mL × 3) and 1 time with 5% aqueous NaCl solution (15 mL × 1). The resulting organic layer was concentrated under reduced pressure condition to obtain H-D-MePhe-OtBu (1.08 g, 4.59 mmol). To a flask with a stirrer, H-D-MePhe-OtBu (1.08 g, 4.59 mmol), MeCN (14 mL), Cbz-Leu-OH (1.45 g, 5.48 mmol) and NMM (1.6 mL, 14.6 mmol) were added. COMU (2.78 g, 6.49 mmol) was added to the reaction mixture while the flask was cooled in an ice bath, and the mixture was stirred continuously at room temperature for 1 hour. The completion of the reaction was confirmed by HPLC analysis, 2-MeTHF (30 mL), 5% aqueous $K_2CO_3$ solution (15 mL), and NMI (0.39 mL, 4.89 mmol) were added to the reaction mixture, and the mixture was stirred continuously for 30 minutes. All the contents of the flask were transferred to a separatory funnel and the aqueous layer was removed. The resulting organic layer was washed 1 time with 5% aqueous $K_2CO_3$ solution (25 mL × 1), 3 times with 2.5% aqueous ammonia solution (25 mL × 3), 2 times with 5% aqueous $NaHSO_4$ solution (25 mL × 2), 1 time with 5% aqueous $K_2CO_3$ solution (25 mL × 1), and 1 time with 5% aqueous NaCl solution (50 mL × 1). The resulting organic layer was dried over anhydrous $Na_2SO_4$. The desiccant was filtered off, and then the filtrate was concentrated under reduced pressure condition to obtain Cbz-Leu-D-MePhe-OtBu (2.35 g, 4.87 mmol). To a flask with a stirrer, Cbz-Leu-D-MePhe-OtBu (2.35 g, 4.87 mmol), 2-MeTHF (24 mL), and HMDS (7.1 mL, 33.87 mmol) were added, and then TMSOTf (5.4 mL, 29.84 mmol) was added dropwise. The mixture was stirred continuously for 3 hours, and then the completion of the reaction was confirmed by HPLC analysis. The flask was cooled, and 5% aqueous $NaHCO_3$ solution (24 mL) was added dropwise to the reaction mixture while the reaction mixture was kept at 25°C or lower. All the contents of the flask were transferred to a separatory funnel and the organic layer was removed. To the resulting aqueous layer, 2-MeTHF (36 mL) was added, and 85% $H_3PO_4$ (3.5 mL) was gradually added. The aqueous layer was removed, and the resulting organic layer was washed with 5% aqueous NaCl solution (24 mL) and concentrated under reduced pressure condition. The resulting concentrate was purified by silica gel column chromatography to obtain Cbz-Leu-D-MePhe-OH (1.40 g, 3.28 mmol).

Purity: 99.8%
Measurement method: HPLC Method A, Retention time: 12.9 min
Mass spectrometry: m/z 427.71 ($[M+H]^+$)

(Raw material synthesis 14) Synthesis of Cbz-MeLeu-MeSer(OtBu)-OH

[Formula 47]

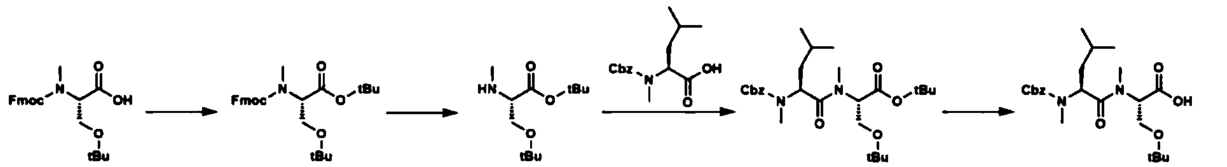

Fmoc-MeSer(OtBu)-OH Fmoc-MeSer(OtBu)-OtBu H-MeSer(OtBu)-OtBu Cbz-MeLeu-MeSer(OtBu)-OtBu Cbz-MeLeu-MeSer(OtBu)-OH

To a flask with a stirrer, Fmoc-MeSer(OtBu)-OH (1.80 g, 4.53 mmol), cyclohexane (14.4 mL), and DCM (5.4 mL) were added. To the flask, tert-butyl 2,2,2-trichloroacetimidate (1.6 mL, 8.94 mmol) was added. A boron trifluoride diethyl ether complex (57 μmL, 0.454 mmol) was added dropwise while the flask was cooled in an ice bath, and then the mixture was stirred continuously at room temperature for 3 hours. The completion of the reaction was confirmed by HPLC analysis, the reaction mixture was filtered, and the filtered solid was washed with cyclohexane. The filtrate and the wash solution were combined and washed 5 times with 10% aqueous citric acid solution (15 mL × 5). The resulting organic layer was washed 2 times with 5% aqueous $Na_2CO_3$ solution (15 mL × 2). The resulting organic layer was dried over anhydrous $Na_2SO_4$. The desiccant was filtered off, and then the filtrate was concentrated under reduced pressure condition to obtain Fmoc-MeSer(OtBu)-OtBu (2.05 g, 4.52 mmol). To a flask with a stirrer, Fmoc-MeSer(OtBu)-OtBu (2.05 g, 4.52 mmol) and MeCN (16 mL) were added. To the flask, DBU (0.68 mL, 4.55 mmol) was added and the mixture was stirred for 30 minutes. The completion of the reaction was confirmed by HPLC analysis, and TEA (2.5 mL, 17.94 mmol) and water (0.81 mL, 44.95 mmol) were added to the reaction mixture. Sodium bisulfite (1.19 g, 11.44 mmol) was added while the flask was cooled in an ice bath, and the mixture was stirred at room temperature for 1 hour. To the reaction mixture, MTBE (50 mL) and 10% aqueous ammonia solution (25 mL) were added, and all the contents of the flask were transferred to a separatory funnel. The aqueous layer was removed, and the resulting organic layer was washed 3 times with 10% aqueous ammonia solution (25 mL × 3) and 1 time with 5% aqueous NaCl solution (25 mL × 1). The resulting organic layer was concentrated under reduced pressure condition to obtain H-MeSer(OtBu)-OtBu. To the flask, 2-MeTHF (12.0 mL), Cbz-MeLeu-OH (1.39 g, 4.97 mmol), and NMM (1.5 mL, 13.64 mmol) were added. COMU (2.52 g, 5.88 mmol) was added to the reaction mixture while the flask was cooled in an ice bath, and the mixture was stirred continuously at room temperature for 2 hours. The completion of the reaction was confirmed by HPLC analysis, 2-MeTHF (30 mL), 5% aqueous $K_2CO_3$ solution (15 mL), and NMI (0.36 mL, 4.52 mmol) were sequentially added to the reaction mixture, and the mixture was stirred continuously for 30 minutes. All the contents of the flask were transferred to a separatory funnel and the aqueous layer was removed. The organic layer was washed 1 time with 5% aqueous $K_2CO_3$ solution (25 mL × 1), 3 times with 2.5% aqueous ammonia solution (25 mL × 3), 2 times with 5% aqueous $NaHSO_4$ solution (25 mL × 2), 1 time with 5% aqueous $K_2CO_3$ solution (25 mL × 1), and 1 time with 5% aqueous NaCl solution (25 mL × 1). The resulting organic layer was dried over anhydrous $Na_2SO_4$. The desiccant was filtered off, and then the filtrate was concentrated under reduced pressure condition to obtain Cbz-MeLeu-MeSer(OtBu)-OtBu (1.98 g, 4.02 mmol). To a flask with a stirrer, Cbz-MeLeu-MeSer(OtBu)-OtBu (1.98 g, 4.02 mmol), 2-MeTHF (20 mL), and HMDS (6.0 mL, 28.63 mmol) were added, and then TMSOTf (4.4 mL, 24.31 mmol) was added dropwise. The mixture was stirred continuously for 2.5 hours, and then the completion of the reaction was confirmed by HPLC analysis. The flask was cooled, and 5% aqueous $NaHCO_3$ solution (20 mL) was added dropwise to the reaction mixture while the reaction mixture was kept at 24°C or lower. All the contents of the flask were transferred to a separatory funnel and the organic layer was removed. To the resulting aqueous layer, 2-MeTHF (30 mL) was added, and 85% $H_3PO_4$ (2.25 mL) was gradually added. The aqueous layer was removed, and the resulting organic layer was washed with 5% aqueous NaCl solution (20 mL) and concentrated under reduced pressure condition. The resulting concentrate was purified by silica gel column chromatography to obtain Cbz-MeLeu-MeSer(OtBu)-OH (0.69 g, 1.59 mmol).

Purity: 99.7%
Measurement method: HPLC Method A, Retention time: 14.0 min
Mass spectrometry: m/z 437.74 ($[M+H]^+$)

(Raw material synthesis 15) Synthesis of Cbz-Aze-EtPhe(4-Me)-OH

[Formula 48]

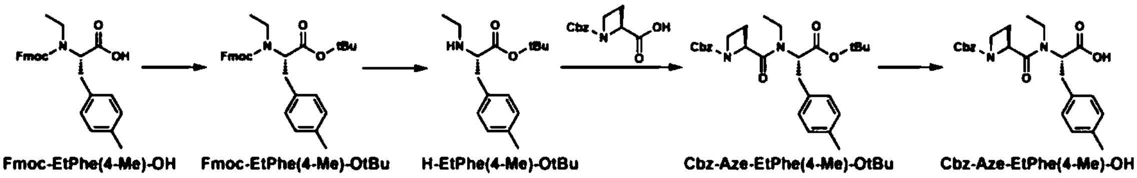


To a flask with a stirrer, Fmoc-EtPhe(4-Me)-OH (5.02 g, 11.60 mmol), cyclohexane (40 mL), and DCM (15 mL) were added. To the flask, tert-butyl 2,2,2-trichloroacetimidate (4.2 mL, 23.47 mmol) was added. A boron trifluoride diethyl ether complex (0.15 mL, 1.19 mmol) was added dropwise while the flask was cooled in an ice bath, and then the mixture was stirred continuously at room temperature for 1 hour. The completion of the reaction was confirmed by HPLC analysis, the reaction mixture was filtered, and the filtered solid was washed with cyclohexane. The filtrate and the wash solution were combined and washed 5 times with 10% aqueous citric acid solution (40 mL × 5). The resulting organic layer was washed 2 times with 5% aqueous $Na_2CO_3$ solution (40 mL × 2). The resulting organic layer was dried over anhydrous $Na_2SO_4$. The desiccant was filtered off, and then the filtrate was concentrated under reduced pressure condition to obtain Fmoc-EtPhe(4-Me)-OtBu (6.13 g, 12.60 mmol). To a flask with a stirrer, Fmoc-EtPhe(4-Me)-OtBu (5.65 g, 11.60 mmol) and MeCN (45 mL) were added. To the flask, DBU (1.80 mL, 12.04 mmol) was added and the mixture was stirred for 30 minutes. The completion of the reaction was confirmed by HPLC analysis, and TEA (6.5 mL, 46.64 mmol) and water (2.1 mL, 116.54 mmol) were added to the reaction mixture. Sodium bisulfite (3.03 g, 29.12 mmol) was added while the flask was cooled in an ice bath, and the mixture was stirred at room temperature for 1 hour. To the reaction mixture, MTBE (140 mL) and 10% aqueous ammonia solution (85 mL) were added, and all the contents of the flask were transferred to a separatory funnel. The aqueous layer was removed, and the resulting organic layer was washed 3 times with 10% aqueous ammonia solution (85 mL × 3) and 1 time with 5% aqueous NaCl solution (85 mL × 1). The resulting organic layer was concentrated under reduced pressure condition to obtain H-EtPhe(4-Me)-OtBu (3.06 g, 11.60 mmol). To a flask with a stirrer, H-EtPhe(4-Me)-OtBu (3.06 g, 11.60 mmol), 2-MeTHF (28 mL), Cbz-Aze-OH (3.28 g, 13.90 mmol), and DIPEA (16.0 mL, 91.85 mmol) were added. To the reaction mixture, a T3P solution (50% in 2-MeTHF, 21.0 mL, 11.1 g, 34.9 mmol) was added while the flask was cooled in an ice bath, and the mixture was stirred continuously at room temperature for 2 hours. The completion of the reaction was confirmed by HPLC analysis, and 5% aqueous $Na_2CO_3$ solution (28 mL) was added to the reaction mixture. All the contents of the flask were transferred to a separatory funnel and the aqueous layer was removed. The resulting organic layer was washed 1 time with 4% aqueous $H_2SO_4$ solution (28 mL × 1), 1 time with 10% aqueous $KHSO_4$ solution (28 mL × 1), 1 time with 5% aqueous $Na_2CO_3$ solution (28 mL × 1), and 1 time with 5% aqueous NaCl solution (28 mL × 1). The resulting organic layer was dried over anhydrous $Na_2SO_4$. The desiccant was filtered off, and then the filtrate was concentrated under reduced pressure condition to obtain Cbz-Aze-EtPhe(4-Me)-OtBu (5.42 g, 11.30 mmol). To a flask with a stirrer, Cbz-Aze-EtPhe(4-Me)-OtBu (5.42 g, 11.30 mmol), 2-MeTHF (54 mL), and HMDS (9.5 mL, 45.32 mmol) were added, and then TMSOTf (6.5 mL, 35.91 mmol) was added dropwise. The mixture was stirred continuously for 4 hours, and then the completion of the reaction was confirmed by HPLC analysis. The flask was cooled, and 5% aqueous $NaHCO_3$ solution (65 mL) was added dropwise to the reaction mixture while the reaction mixture was kept at 24°C or lower. All the contents of the flask were transferred to a separatory funnel and the organic layer was removed. To the resulting aqueous layer, 2-MeTHF (108 mL) was added, and 85% $H_3PO_4$ (8.2 mL) was gradually added. The aqueous layer was removed, and the resulting organic layer was washed with 5% aqueous NaCl solution (65 mL) and concentrated under reduced pressure condition. The resulting concentrate was purified by silica gel column chromatography to obtain Cbz-Aze-EtPhe(4-Me)-OH (1.67 g, 3.93 mmol).

Purity: 99.0%
Measurement method: HPLC Method A, Retention time: 11.9 min
Mass spectrometry: m/z 425.72 ($[M+H]^+$)

(Raw material synthesis 16) Synthesis of Fmoc-MeAlGly-MeAlGly-OH

[Formula 49]

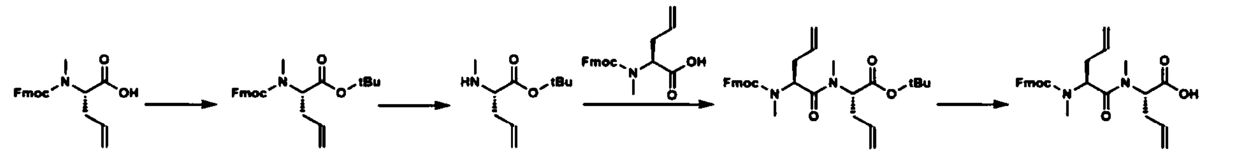

Fmoc-MeAlGly-OH    Fmoc-MeAlGly-OtBu    H-MeAlGly-OtBu    Fmoc-MeAlGly-MeAlGly-OtBu    Fmoc-MeAlGly-MeAlGly-OH

To a flask with a stirrer, Fmoc-MeAlGly-OH (1.50 g, 4.27 mmol), cyclohexane (12 mL), and DCM (4.5 mL) were added. To the flask, tert-butyl 2,2,2-trichloroacetimidate (1.5 mL, 8.38 mmol) was added. A boron trifluoride diethyl ether complex (54 μL, 0.43 mmol) was added dropwise while the flask was cooled in an ice bath, and then the mixture was stirred continuously at room temperature for 1.5 hours. The completion of the reaction was confirmed by HPLC analysis, the reaction mixture was filtered, and the filtered solid was washed with cyclohexane. The filtrate and the wash solution were combined and washed 5 times with 10% aqueous citric acid solution (12 mL × 5). The resulting organic layer was washed 2 times with 5% aqueous $Na_2CO_3$ solution (12 mL × 2). The resulting organic layer was dried over anhydrous $Na_2SO_4$. The desiccant was filtered off, and then the filtrate was concentrated under reduced pressure condition to obtain Fmoc-MeAlGly-OtBu (1.89 g, 4.64 mmol). To a flask with a stirrer, Fmoc-MeAlGly-OtBu (1.74 g, 4.27 mmol) and MeCN (14 mL) were added. To the flask, DBU (0.64 mL, 4.28 mmol) was added and the mixture was stirred for 30 minutes. The completion of the reaction was confirmed by HPLC analysis, and TEA (2.4 mL, 17.22 mmol) and water (0.77 mL, 42.73 mmol) were added to the reaction mixture. Sodium bisulfite (1.11 g, 10.67 mmol) was added while the flask was cooled in an ice bath, and the mixture was stirred at room temperature for 30 minutes. To the reaction mixture, MTBE (44 mL) and 10% aqueous ammonia solution (24 mL) were added, and all the contents of the flask were transferred to a separatory funnel. The aqueous layer was removed, and the resulting organic layer was washed 3 times with 10% aqueous ammonia solution (24 mL × 3) and 1 time with 5% aqueous NaCl solution (24 mL × 1). The resulting organic layer was concentrated under reduced pressure condition to obtain H-MeAlGly-OtBu (0.74 g). To the flask, MeCN (10.0 mL), Fmoc-MeAlGly-OH (1.65 g, 4.70 mmol), and NMM (1.4 mL, 12.73 mmol) were sequentially added. COMU (2.20 g, 5.14 mmol) was added to the reaction mixture while the flask was cooled in an ice bath, and the mixture was stirred continuously at room temperature for 2 hours. COMU (0.19 g, 0.44 mmol) was added to the reaction mixture, and the mixture was stirred continuously at room temperature for 30 minutes. COMU (0.36 g, 0.84 mmol) was added to the reaction mixture, and the mixture was stirred continuously at room temperature for an additional 30 minutes. The completion of the reaction was confirmed by HPLC analysis, 2-MeTHF (24 mL), 5% aqueous $K_2CO_3$ solution (12 mL), and NMI (0.34 mL, 4.27 mmol) were sequentially added to the reaction mixture, and the mixture was stirred continuously for 30 minutes. All the contents of the flask were transferred to a separatory funnel and the aqueous layer was removed. The organic layer was washed 4 times with 5% aqueous $K_2CO_3$ solution (20 mL × 4), 2 times with 5% aqueous $NaHSO_4$ solution (20 mL × 2), 1 time with 5% aqueous $K_2CO_3$ solution (20 mL × 1), and 1 time with 5% aqueous NaCl solution (20 mL × 1). The resulting organic layer was dried over anhydrous $Na_2SO_4$, the desiccant was filtered off, and then the filtrate was concentrated under reduced pressure condition. The resulting concentrate was purified by silica gel column chromatography to obtain Fmoc-MeAlGly-MeAlGly-OtBu (2.08 g, 4.01 mmol). To a flask with a stirrer, Fmoc-MeAlGly-MeAlGly-OtBu (2.08 g, 4.01 mmol), 2-MeTHF (20 mL), and HMDS (3.4 mL, 16.22 mmol) were added, and then TMSOTf (2.2 mL, 12.16 mmol) was added dropwise. The mixture was stirred continuously for 2 hours, and then HMDS (1.3 mL, 6.20 mmol) and TMSOTf (1.2 mL, 6.63 mmol) were added to the reaction mixture. 2-MeTHF (20 mL) was added to the reaction mixture and the precipitate was dissolved. The mixture was stirred continuously for an additional 1 hour, and then HMDS (2.1 mL, 10.02 mmol) and TMSOTf (1.9 mL, 10.50 mmol) were added to the reaction mixture. The mixture was stirred continuously for an additional 1 hour, and the completion of the reaction was confirmed by HPLC analysis. The flask was cooled, and 5% aqueous $NaHCO_3$ solution (20 mL) was added dropwise to the reaction mixture while the reaction mixture was kept at 13°C or lower. All the contents of the flask were transferred to a separatory funnel and the organic layer was removed. To the resulting aqueous layer, 2-MeTHF (30 mL) was added, and 85% $H_3PO_4$ (3.6 mL) was gradually added. The aqueous layer was removed, and the resulting organic layer was washed with 5% aqueous NaCl solution (20 mL) and concentrated under reduced pressure condition. The resulting concentrate was purified by silica gel column chromatography to obtain Fmoc-MeAlGly-MeAlGly-OH (1.41 g, 3.05 mmol).

Purity: 98.1%
Measurement method: HPLC Method A, Retention time: 14.6 min
Mass spectrometry: m/z 463.79 ($[M+H]^+$)

(Raw material synthesis 17) Synthesis of Cbz-MeLeu-Melle-OH

[Formula 50]

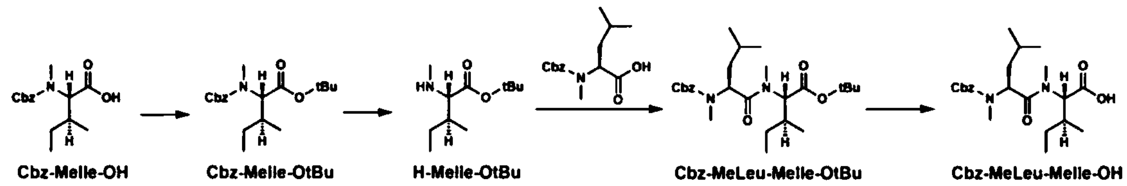

To a flask with a stirrer, Cbz-Melle-OH (4.50 g, 16.00 mmol), cyclohexane (36 mL), and DCM (14 mL) were added. To the flask, tert-butyl 2,2,2-trichloroacetimidate (5.8 mL, 32.41 mmol) was added. A boron trifluoride diethyl ether complex (0.20 mL, 1.59 mmol) was added dropwise while the flask was cooled in an ice bath, and then the mixture was stirred continuously at room temperature for 2 hours. The completion of the reaction was confirmed by HPLC analysis, the reaction mixture was filtered, and the filtered solid was washed with cyclohexane. The filtrate and the wash solution were combined and washed 5 times with 10% aqueous citric acid solution (36 mL × 5). The resulting organic layer was washed 2 times with 5% aqueous $Na_2CO_3$ solution (36 mL × 2). The resulting organic layer was concentrated under reduced pressure conditions to obtain Cbz-Melle-OtBu (5.11 g, 15.20 mmol). To a flask with a stirrer, Cbz-Melle-OtBu (5.11 g, 15.20 mmol), 2-MeTHF (51 mL), and 5% Pd/C (3.24 g, 0.76 mmol on Pd metal basis) were sequentially added. The nitrogen replacement and hydrogen replacement in the flask were performed, and the reaction mixture was stirred under a hydrogen atmosphere (1 atm) for 2 hours. The reaction mixture was filtered and Pd/C was washed with 2-MeTHF. The filtrate and the washing solution were combined and concentrated under reduced pressure condition to obtain H-Melle-OtBu (2.73 g, 13.57 mmol). To a flask with a stirrer, H-Melle-OtBu (2.73 g, 13.57 mmol), MeCN (27.0 mL), Cbz-MeLeu-OH (4.17 g, 14.90 mmol) and NMM (4.5 mL, 40.93 mmol) were sequentially added. COMU (7.55 g, 17.63 mmol) was added to the reaction mixture while the flask was cooled in an ice bath, and the mixture was stirred continuously at room temperature for 4 hours. COMU (0.58 g, 1.35 mmol) was added, and the mixture was stirred continuously at room temperature for 30 minutes. The completion of the reaction was confirmed by HPLC analysis, 2-MeTHF (80 mL), 5% aqueous $K_2CO_3$ solution (40 mL), and NMI (1.0 mL, 12.55 mmol) were sequentially added to the reaction mixture, and the mixture was stirred continuously for 30 minutes. All the contents of the flask were transferred to a separatory funnel and the aqueous layer was removed. The organic layer was washed 1 time with 5% aqueous $K_2CO_3$ solution (40 mL × 1), 3 times with 2.5% aqueous ammonia solution (70 mL × 3), 2 times with 5% aqueous $NaHSO_4$ solution (70 mL × 2), 1 time with 5% aqueous $K_2CO_3$ solution (70 mL × 1), and 1 time with 5% aqueous NaCl solution (70 mL × 1). The resulting organic layer was dried over anhydrous $Na_2SO_4$. The desiccant was filtered off, and then the filtrate was concentrated under reduced pressure condition to obtain Cbz-MeLeu-Melle-OtBu (5.99 g, 12.95 mmol). To a flask with a stirrer, Cbz-MeLeu-Melle-OtBu (5.99 g, 12.95 mmol), 2-MeTHF (60.0 mL), and HMDS (19.0 mL, 90.64 mmol) were added, and then TMSOTf (14.0 mL, 77.35 mmol) was added dropwise. The mixture was stirred continuously for 6 hours, and then HMDS (8.0 mL, 38.17 mmol) and TMSOTf (4.8 mL, 26.52 mmol) were added to the reaction mixture. The mixture was stirred continuously for 30 minutes, and then the conversion rate was confirmed to reach 94% by HPLC analysis. The flask was cooled, and 5% aqueous $NaHCO_3$ solution (60 mL) was added dropwise to the reaction mixture while the reaction mixture was kept at 24°C or lower. All the contents of the flask were transferred to a separatory funnel and the organic layer was removed. To the resulting aqueous layer, 2-MeTHF (90 mL) was added, and 85% $H_3PO_4$ (8.5 mL) was gradually added. The aqueous layer was removed, and the resulting organic layer was washed with 5% aqueous NaCl solution (60 mL) and concentrated under reduced pressure condition. The resulting concentrate was purified by silica gel column chromatography to obtain Cbz-MeLeu-Melle-OH (3.09 g, 7.59 mmol).

Purity: 97.7%
Measurement method: HPLC Method A, Retention time: 13.8 min
Mass spectrometry: m/z 407.70 ($[M+H]^+$)

(Raw material synthesis 18-1) Synthesis of HOPO having a substituent

[Formula 51]

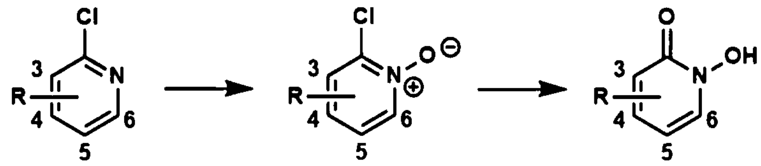

The synthesis was performed based on the conditions described in the following literatures.

Reference Literature 1: Bioorganic & Medicinal Chemistry 2009, 17, 6106-6122.
Reference Literature 2: Communications Chemistry 2023, 14, 5790.

(Raw material synthesis 18-2) Synthesis of 5-F-HOPO

[Formula 52]

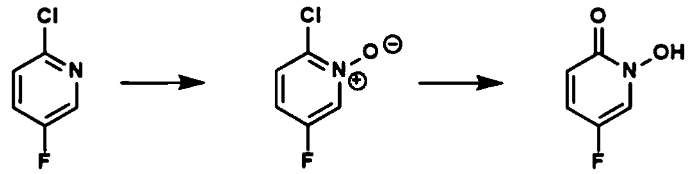

**5-F-HOPO**

To a flask with a stirrer, 2-chloro-5-fluoropyridine (0.77 g, 5.83 mmol), urea hydrogen peroxide (1.15 g, 12.24 mmol), and DCM (10 mL) were sequentially added, and the mixture was cooled to -5°C. To the reaction mixture, TFAA (1.63 mL, 11.66 mmol) was added dropwise while the flask was stirred, and the mixture was stirred at 25°C for 3.5 hours. The flask was cooled to 0°C, and a 10% aqueous $NaHSO_3$ solution (5 mL) was added. All the contents of the flask were transferred to a separatory funnel, and EtOAc (30 mL) and 10% $NaHSO_3$ (5 mL) were added. The aqueous layer was removed and the resulting organic layer was washed 2 times with saturated aqueous $NaHCO_3$ solution (15 mL × 1) and 1 time with 5% aqueous NaCl solution (10 mL × 1). The resulting organic layer was dried over anhydrous $Na_2SO_4$, the desiccant was filtered off, and then the filtrate was concentrated under reduced pressure condition to obtain 2-chloro-5-fluoropyridine N-oxide (360 mg). To a flask with a stirrer, 2-chloro-5-fluoropyridine N-oxide (360 mg, 2.44 mmol) and MeCN (2.0 mL) were added. To the reaction mixture, TFAA (4.0 mL) was added dropwise while the flask was stirred, and the mixture was stirred for 4.5 hours. The reaction mixture was concentrated under reduced pressure condition, and DCM (4 mL) and $NaHCO_3$ (1.4 g) were added. After the solid was filtered off, the filtrate was concentrated under reduced pressure condition. The resulting concentrate was purified by silica gel column chromatography to obtain 5-F-HOPO (50 mg, 0.39 mmol).
$^1$H-NMR (500 MHz, DMSO-$d_6$): δ (ppm) 8.26 (t, J = 3.7 Hz, 1H), 7.56-7.52 (m, 1H), 6.54 (dd, J = 10.0, 6.0 Hz, 1H)

(Raw material synthesis 18-3) Synthesis of 5-Cl-HOPO

[Formula 53]

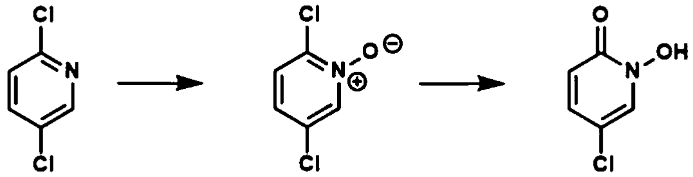

**5-Cl-HOPO**

To a flask with a stirrer, 2,5-dichloropyridine (0.86 g, 5.83 mmol), urea hydrogen peroxide (1.15 g, 12.24 mmol), and DCM (10 mL) were sequentially added, and the mixture was cooled to -5°C. To the reaction mixture, TFAA (1.63 mL,

11.66 mmol) was added dropwise while the flask was stirred, and the mixture was stirred at 25°C for 6 hours. The flask was cooled to 0°C, and a 10% aqueous $NaHSO_3$ solution (5 mL) was added. All the contents of the flask were transferred to a separatory funnel, and EtOAc (30 mL) and 10% $NaHSO_3$ (5 mL) were added. The aqueous layer was removed and the resulting organic layer was washed 2 times with saturated aqueous $NaHCO_3$ solution (15 mL × 1) and 1 time with 5% aqueous NaCl solution (10 mL × 1). The resulting organic layer was dried over anhydrous $Na_2SO_4$, the desiccant was filtered off, and then the filtrate was concentrated under reduced pressure condition to obtain 2,5-dichloropyridine N-oxide (760 mg). To a flask with a stirrer, 2,5-dichloropyridine N-oxide (455 mg, 2.75 mmol) and MeCN (2.0 mL) were added. To the reaction mixture, TFAA (4.0 mL, mmol) was added dropwise while the flask was stirred, and the mixture was stirred for 5 hours. The reaction mixture was concentrated under reduced pressure condition, and DCM (4 mL) and $NaHCO_3$ (1.4 g) were added. After the solid was filtered off, the filtrate was concentrated under reduced pressure condition. The resulting concentrate was purified by silica gel column chromatography to obtain 5-Cl-HOPO (100 mg, 0.69 mmol).
$^1$H-NMR (500 MHz, DMSO-$d_6$): δ (ppm) 8.26 (d, J = 2.9 Hz, 1H), 7.46 (dd, J = 9.7, 2.9 Hz, 1H), 6.55 (d, J = 9.2 Hz, 1H)

(Raw material synthesis 18-4) Synthesis of 5-$CF_3$-HOPO

[Formula 54]

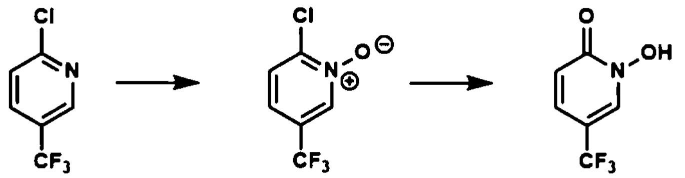

5-$CF_3$-HOPO

To a flask with a stirrer, 2-chloro-5-(trifluoromethyl)pyridine (1.06 g, 5.83 mmol), urea hydrogen peroxide (1.15 g, 12.24 mmol), and DCM (10 mL) were sequentially added, and the mixture was cooled to -5°C. To the reaction mixture, TFAA (1.63 mL, 11.66 mmol) was added dropwise while the flask was stirred, and the mixture was stirred at 25°C for 6 hours. The flask was cooled to 0°C, and a 10% aqueous $NaHSO_3$ solution (5 mL) was added. All the contents of the flask were transferred to a separatory funnel, and EtOAc (30 mL) and 10% $NaHSO_3$ (5 mL) were added. The aqueous layer was removed and the resulting organic layer was washed 2 times with saturated aqueous $NaHCO_3$ solution (15 mL × 1) and 1 time with 5% aqueous NaCl solution (10 mL × 1). The resulting organic layer was dried over anhydrous $Na_2SO_4$, the desiccant was filtered off, and then the filtrate was concentrated under reduced pressure condition to obtain 2-chloro-5-(trifluoromethyl)pyridine N-oxide (940 mg). To a flask with a stirrer, 2-chloro-5-(trifluoromethyl)pyridine N-oxide (542 mg, 2.75 mmol) and MeCN (2.0 mL) were added. To the reaction mixture, TFAA (4.0 mL, mmol) was added dropwise while the flask was stirred, and the mixture was stirred for 4.5 hours. The reaction mixture was concentrated under reduced pressure condition, and DCM (4 mL) and $NaHCO_3$ (1.4 g) were added. After the solid was filtered off, the filtrate was concentrated under reduced pressure condition. The resulting concentrate was purified by silica gel column chromatography to obtain 5-$CF_3$-HOPO (350 mg, 1.95 mmol).
$^1$H-NMR (500 MHz, DMSO-$d_6$): δ (ppm) 8.60 (d, J = 0.9 Hz, 1H), 7.66 (dd, J = 9.5, 2.6 Hz, 1H), 6.66 (d, J = 9.2 Hz, 1H)

(Raw material synthesis 18-5) Synthesis of 5-$CO_2$Me-HOPO

[Formula 55]

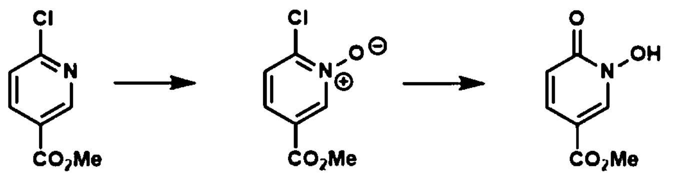

5-$CO_2$Me-HOPO

To a flask with a stirrer, methyl 6-chloronicotinate (1.01 g, 5.83 mmol), urea hydrogen peroxide (1.15 g, 12.24 mmol), and DCM (10 mL) were sequentially added, and the mixture was cooled to -5°C. To the reaction mixture, TFAA (1.63 mL, 11.66 mmol) was added dropwise while the flask was stirred, and the mixture was stirred at 25°C for 6 hours. The flask was cooled to 0°C, and a 10% aqueous $NaHSO_3$ solution (5 mL) was added. All the contents of the flask were transferred to a separatory funnel, and EtOAc (30 mL) and 10% $NaHSO_3$ (5 mL) were added. The aqueous layer was removed and the resulting organic layer was washed 2 times with saturated aqueous $NaHCO_3$ solution (15 mL × 1) and 1 time with 5% aqueous NaCl solution (10 mL × 1). The resulting organic layer was dried over anhydrous $Na_2SO_4$, the desiccant was filtered off, and then the filtrate was concentrated under reduced pressure condition to obtain methyl 6-chloronicotinate N-oxide (540 mg). To a flask with a stirrer, methyl 6-chloronicotinate N-oxide (521 mg, 2.75 mmol) and MeCN (2.0 mL) were added. To the reaction mixture, TFAA (4.0 mL, mmol) was added dropwise while the flask was stirred, and the mixture was stirred for 4.5 hours. The reaction mixture was concentrated under reduced pressure condition, and DCM (4 mL) and $NaHCO_3$ (1.4 g) were added. After the solid was filtered off, the filtrate was concentrated under reduced pressure condition. The resulting concentrate was purified by silica gel column chromatography to obtain 5-$CO_2$Me-HOPO (300 mg, 1.77 mmol).
$^1$H-NMR (500 MHz, DMSO-$d_6$): δ (ppm) 8.50 (d, J = 2.3 Hz, 1H), 7.79 (dd, J = 9.5, 2.6 Hz, 1H), 6.56 (d, J = 9.2 Hz, 1H), 3.78 (s, 3H)

(Raw material synthesis 19) Synthesis of H-MeGly(nPr)-Ile-Pro-OtBu

[Formula 56]

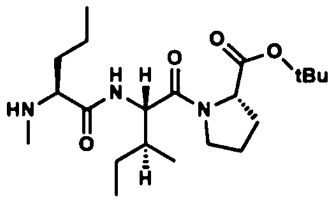

H-MeGly(nPr)-Ile-Pro-OtBu

Step 19-1

Synthesis of Compound 19-1: tert-butyl (2S)-1-[(2S,3S)-2-(benzyloxycarbonylamino)-3-methyl-pentanoyl]pyrrolidine-2-carboxylic acid

[Formula 57]

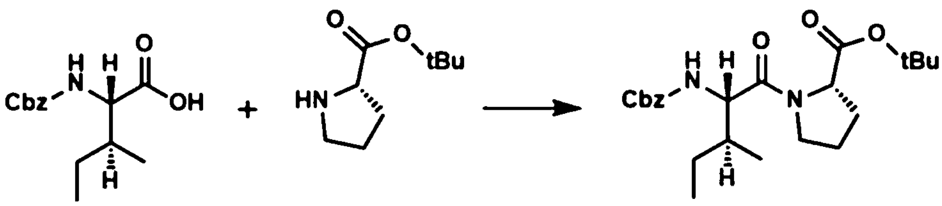

To a reactor subjected to nitrogen replacement, tert-butyl (2S)-pyrrolidine-2-carboxylic acid (18.8 g), (2S,3S)-2-(benzyloxycarbonylamino)-3-methyl-pentanoic acid (20.0 g), and DMF (140 mL) were added at room temperature, and the mixture was stirred. After confirming complete dissolution, the mixture was cooled to 0°C and DIPEA (52.7 mL) was added. To the mixed solution, 50 wt.% propylphosphonic acid anhydride, ethyl acetate solution (58.3 mL) was added at 0°C over 20 minutes, and the mixture was stirred at 0°C for 1.5 hours. Water (100 mL) and ethyl acetate (200 mL) were added to the mixed solution in this order. The mixture was separated into aqueous layer 1 and organic layer 1 by liquid-liquid separation, and the aqueous layer 1 was removed. The organic layer 1 was washed with 5% aqueous potassium hydrogen sulfate solution (100 mL), 5% aqueous sodium carbonate solution (100 mL), and 10% saline (100

mL). To the removed aqueous layer 1, water (100 mL) and ethyl acetate (200 mL) were added. The mixture was stirred, and then organic layer 2 was obtained by liquid-liquid separation. The organic layer 1 and the organic layer 2 were combined and concentrated under reduced pressure to obtain compound 19-1 (33.8 g).
LCMS (ESI) of compound 19-1: Retention time: 2.75 minutes, m/z = 419 [M+H]+

Step 19-2

Synthesis of Compound 19-2: tert-butyl (2S)-1-[(2S,3S)-2-amino-3-methyl-pentanoyl]pyrrolidine-2-carboxylic acid

[Formula 58]

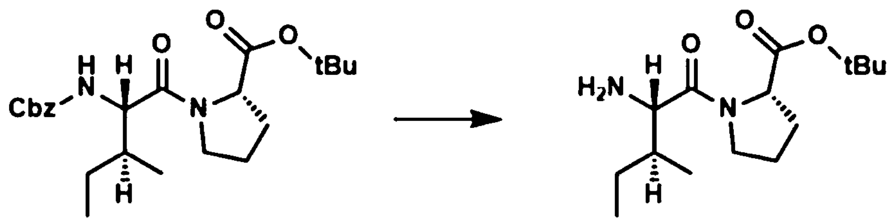

To a reactor subjected to nitrogen replacement, compound 19-1 (31.6 g) obtained in Step 19-1 and 2-MeTHF (221 mL) were added, and the mixture was cooled to 10°C. To the mixed solution, 5% Pd/C (6.32 g, 50% moistened with water) was added, and then triethylsilane (60.3 mL) was added over 20 minutes. The mixture was stirred at the internal temperature of 15°C for 6 hours, and then the mixture was stirred at room temperature for an additional 17 hours. The mixed solution was filtered and the filtrate was concentrated under reduced pressure to obtain compound 19-2. Compound 19-2 was used in Step 19-3 without further purification.
LCMS (ESI) of compound 19-2: Retention time: 1.14 minutes, m/z = 285 [M+H]+

Step 19-3

Synthesis of Compound 19-3: tert-butyl (2S)-1-[(2S,3S)-2-[[(2S)-2-[9H-fluoren-9-ylmethoxycarbonyl(methyl)amino] pentanoyl]amino]-3-methyl-pentanoyl]pyrrolidine-2-carboxylic acid

[Formula 59]

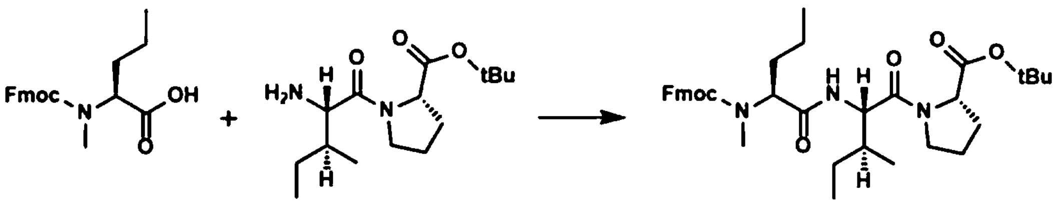

To compound 19-2 obtained in Step 19-2, acetonitrile (221 mL) was added at 5°C, and then (2S)-2-[9H-fluoren-9-ylmethoxycarbonyl(methyl)amino]pentanoic acid (28.0 g) and DIPEA (39.6 mL) were added. To the mixed solution, HATU (34.5 g) was slowly added at 2.5°C. The mixture was stirred at 2.5°C for 1 hour, and then stirred at room temperature for 2.5 hours. To the mixed solution, 5% aqueous sodium carbonate solution (189 mL) and water (150 mL) were added in this order. Toluene (80 mL) and 2-MeTHF (140 mL) were added and the mixture was stirred to undergo liquid-liquid separation. The organic layer was washed with 5% aqueous potassium hydrogen sulfate solution (190 mL × 2) and 10% saline (190 mL × 2). The resulting organic layer was concentrated under reduced pressure to obtain compound 19-3. Compound 19-3 was used in Step 19-4 without further purification.
LCMS (ESI) of compound 19-3: Retention time: 3.43 minutes, m/z = 620 [M+H]+

Step 19-4

Synthesis of Compound 19-4: tert-butyl (2S)-1-[(2S,3S)-3-methyl-2-[[(2S)-2-(methylamino)pentanoyl]amino]pentanoyl] pyrrolidine-2-acetic acid (synthesis of H-MeGly(nPr)-Ile-Pro-OtBu)

[Formula 60]

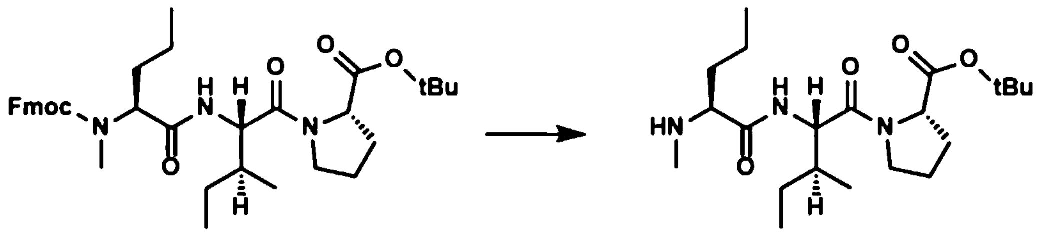

To a reactor subjected to nitrogen replacement, compound 19-3 (296 mg) synthesized by the same method as in Step 19-3 and toluene (2.07 mL) were added, and the mixture was stirred. To the mixed solution, DBU (0.072 mL) was added, and the mixture was stirred for 30 minutes. To the mixed solution, acetonitrile (1.00 mL) was added, and then the mixture was stirred for 30 minutes. DBU (0.072 mL) was added, and the mixture was stirred for an additional 30 minutes. 1 M hydrochloric acid (2.00 mL) and n-heptane (1.00 mL) were added, and the mixture was stirred, and then aqueous layer 1 and organic layer 1 were separated. The organic layer 1 was extracted with 1 M hydrochloric acid (1.00 mL) to obtain aqueous layer 2 containing compound 19-4. The aqueous layer 1 and the aqueous layer 2 were combined and extracted with 5% aqueous potassium carbonate solution (2.00 mL) and toluene (4.00 mL) to separate an organic layer containing compound 19-4. The resulting organic layer was washed with 10% saline (2.00 mL) and then concentrated under reduced pressure to obtain compound 19-4 (166 mg).
LCMS (ESI) of compound 19-4: Retention time: 1.36 minutes, m/z = 398 $[M+H]^+$

The analytical conditions by LCMS for compounds 19-1 to 19-4 were shown below.

Apparatus: Waters UPLC/SQD
Column: Ascentis Express RP 90A amide, 2.1 mm ID × 50 mm, 2.7 μm
Mobile Phase: 0.1% FA/water (A), 0.1% FA/MeCN (B)
Elution method: B) 5% (0 min) → 100% (4.5 min) → 100% (5.0 min) → 5% (5.01 min) → 5% (7 min)
Flow rate: 0.5 mL/min
Column temperature: 40°C
Detection wavelength: 210-400 nm (PDA)

Industrial Applicability

According to the present invention, a method for synthesizing a peptide compound with high diastereoselectivity and high yield even by fragment coupling can be provided.

## Claims

1. A method for producing a peptide compound or a salt thereof, comprising a step (linking step) of linking an amino group of a first amino acid or peptide and a carboxy group of a second amino acid or peptide with an amide bond, wherein a condensing agent and two or more additives are used in the linking step, and pKa of the additive is between 0 and 10.0.

2. The method according to claim 1, wherein the condensing agent is a phosphorus-based condensing agent, and the phosphorus-based condensing agent is represented by the following formula (1):

[Formula 1]

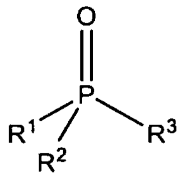

(1)

wherein

$R^1$ is $C_1$-$C_6$ alkoxy, $C_6$-$C_{10}$ aryloxy, $C_6$-$C_{10}$ aryl, or di-$C_1$-$C_6$ alkylamino;
$R^2$ is $C_1$-$C_6$ alkoxy, $C_6$-$C_{10}$ aryloxy, $C_6$-$C_{10}$ aryl, or di-$C_1$-$C_6$ alkylamino; and
$R^3$ is halogen, optionally substituted $C_6$-$C_{10}$ aryloxy, or azide.

**3.** The method according to claim 1 or 2, wherein the phosphorus-based condensing agent is at least one selected from the group consisting of diphenyl chlorophosphate (DPPC), diethyl chlorophosphate (DEPC), dimethyl chlorophosphate, diisopropyl chlorophosphate, dibenzyl chlorophosphate, diphenylphosphoryl azide (DPPA), pentafluorophenyl diphenyl phosphinate (FDPP), 2-chloro-2-oxo-1,3,2-disoxaphosphorane, dimethylphosphinic chloride, diethylphosphinic chloride, dicyclohexylphosphinic chloride, and diphenylphosphinic chloride ($Ph_2P(O)Cl$).

**4.** The method according to any one of claims 1 to 3, wherein the additives are two or more selected from the group consisting of 2-hydroxypyridine-N-oxide (HOPO), 1-hydroxy-7-azabenzotriazole (HOAt), 1-hydroxybenzotriazole (HOBt), 3,4-dihydro-3-hydroxy-4-oxo-benzotriazine (HOOBt), ethyl 1-hydroxy-1H-1,2,3-triazole-4-carboxylate (HOCt), 2,2,3,3,3-pentafluoro-1-propanol (PfpOH), ethyl 2-cyano-2-(hydroxyimino)acetate (Oxyma), 5-(hydroxyimino)-1,3-dimethylpyrimidine-2,4,6-(1H,3H,5H)-trion (Oxyma-B), ethyl (hydroxyimino)cyanoacetate potassium salt (K-Oxyma), 4,6-dimethoxy-1,3,5-triazin-2-ol, 2-hydroxypyridine, and 2-hydroxy-5-nitropyridine.

**5.** The method according to any one of claims 1 to 4, wherein the additives are Oxyma and HOPO.

**6.** The method according to any one of claims 1 to 5, wherein the one or more additives are used in 0.5 to 5.0 molar equivalents relative to the first amino acid or peptide.

**7.** The method according to any one of claims 1 to 6, wherein nitrogen located at $\beta$ position of the carboxy group of the second amino acid or peptide is represented by formula: $-NR^5-$, wherein $R^5$ is a hydrogen atom, linear $C_1$-$C_6$ alkyl, branched $C_3$-$C_6$ alkyl, or $C_3$-$C_8$ cycloalkyl.

**8.** The method according to any one of claims 1 to 7, wherein the amino group of the first amino acid or peptide is an amino group represented by formula: $-NR^6R^7$, wherein $R^6$ is a hydrogen atom, and $R^7$ is a hydrogen atom, linear $C_1$-$C_6$ alkyl, branched $C_3$-$C_6$ alkyl, or $C_3$-$C_8$ cycloalkyl.

**9.** The method according to any one of claims 1 to 8, wherein $\alpha$-carbon of the carboxy group of the second amino acid or peptide is represented by formula: $-CR^8R^9-$, wherein $R^8$ and $R^9$ are identical or different and each is a hydrogen atom, linear $C_1$-$C_4$ alkyl, optionally substituted branched $C_3$-$C_6$ alkyl, optionally substituted phenyl $C_1$-$C_2$ alkyl, or optionally substituted 5- to 6-membered heteroaryl $C_1$-$C_2$ alkyl.

**10.** The method according to any one of claims 1 to 9, wherein a base used in the linking step is at least one selected from the group consisting of dicyclohexylmethylamine, diisopropylethylamine, triethylamine, 2,6-lutidine, N-methylmorpholine, 4-N,N-dimethylaminopyridine, and 2,4,6-collidine.

**11.** The method according to any one of claims 1 to 10, wherein the linking step is performed by a liquid-phase synthesis.

**12.** The method according to any one of claims 1 to 11, wherein the peptide compound or a salt thereof produced contains 8 to 20 amino acid residues.

**13.** The method according to any one of claims 1 to 12, wherein the peptide compound produced contains at least one non-natural amino acid residues.

**14.** A peptide compound or a salt thereof produced by the method according to any one of claims 1 to 13.

**15.** A pharmaceutical composition comprising the peptide compound or a salt thereof produced by the method according to any one of claims 1 to 13.

## INTERNATIONAL SEARCH REPORT

International application No.
**PCT/JP2024/046067**

**A. CLASSIFICATION OF SUBJECT MATTER**

***C07K 1/02***(2006.01)i; ***A61K 38/02***(2006.01)i; ***A61K 38/08***(2019.01)i; ***A61K 38/10***(2006.01)i; ***C07K 1/10***(2006.01)i
FI: C07K1/02 ZNA; C07K1/10; A61K38/02; A61K38/08; A61K38/10

According to International Patent Classification (IPC) or to both national classification and IPC

**B. FIELDS SEARCHED**

Minimum documentation searched (classification system followed by classification symbols)

C07K1/02; A61K38/02; A61K38/08; A61K38/10; C07K1/10

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2023/117904 A1 (BACHEM HOLDING AG) 29 June 2023 (2023-06-29)<br>claims, p. 59, line 35 to p. 61, line 18, p. 68, lines 17-33, example 18 | 1, 4-15<br>2, 3 |
| A | ALBERICIO, F. et al. Choosing the Right Coupling Reagent for Peptides: A Twenty-Five-Year Journey. Org. Process Res. Dev.. 2018, vol. 22, pp. 760-772<br>entire text | 1-15 |
| A | HOFFMANN, F. et al. SYNTHESIS AND CHEMICAL CONSTITUTION OF DIPHENOXYPHOSPHORYL DERIVATIVES AND PHOSPHONIUM SALTS AS COUPLING REAGENTS FOR PEPTIDE SEGMENT CONDENSATION. Phosphorus, Sulfur and Silicon. 2003, vol. 178, pp. 299-309<br>entire text | 1-15 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 March 2025** | **11 March 2025** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**
**Information on patent family members**

International application No.
**PCT/JP2024/046067**

| Patent document cited in search report | Publication date (day/month/year) | Patent family member(s) | Publication date (day/month/year) |
|---|---|---|---|
| WO 2023/117904 A1 | 29 June 2023 | EP 4201951 A1 | |
| | | JP 2025-500341 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013100132 A
- WO 2023117904 A
- WO 2018225851 A
- WO 2018225864 A
- WO 2019117274 A
- WO 2020111238 A
- WO 2020122182 A
- WO 2021075478 A
- WO 2021090856 A
- WO 2021132545 A
- WO 2021246471 A
- WO 2022097540 A
- WO 2022138891 A
- WO 2022145444 A
- WO 2022234864 A
- WO 2023127869 A


### Non-patent literature cited in the description


- *Acc. Chem. Res.*, 2008, vol. 41, 1331-1342
- *Angew. Chem. Int. Ed.*, 2013, vol. 52, 254-269
- *Chem. Rev.*, 2019, vol. 119, 10360-10391
- Amino Acids, Peptides and Proteins in Organic Chemistry: Building Blocks. *Catalysis and Coupling Chemistry*, 2011, vol. 3
- *Tetrahedron Letters*, 2020, 152299
- *Tetrahedron Letters*, 2021, 153462
- *J. Peptide Res.*, 2005, vol. 65, 153-166
- *Chem. Eur. J.*, 2009, vol. 15, 9394-9403
- *Chem. Eur. J*, 2009, vol. 15, 9394-9403
- *ACS Omega*, 2022, vol. 7, 6007-6023
- *ACS Omega.*, 2022, vol. 7, 6007-6023
- *Tetrahedron Asymmetry*, 2012, vol. 23, 1023-1027
- **Y. OKADA** ; **H. SUZUKI** ; **T. NAKAE** ; **S. FUJITA** ; **H. ABE** ; **K. NAGANO** ; **T. YAMADA** ; **N. EBATA** ; **S. KIM** ; **K. CHIBA**. Tag-Assisted Liquid-Phase Peptide Synthesis Using Hydrophobic Benzyl Alcohols as Supports. *J. Org. Chem.*, 2013, vol. 78, 320-327
- **S. YANO et al.** *Molecules*, 2021, vol. 26 (12), 3497
- **R. SRIVASTAVA**. *J. Mol. Catal. A: Chem.*, 2007, vol. 264, 146-152
- **HALL, H.K., JR.** *J. A.m. Chem. Soc.*, 1957, vol. 79, 5441
- **HALL, H.K., JR.** *J. A.m. Chem. Soc*, 1957, vol. 79, 5441
- *Chemical and Pharmaceutical Bulletin*, 1995, vol. 43, 1872-1877
- **CLARKE, K** ; **ROTHWELL, K.** *J. Chem. Soc.*, 1960, 1885
- **CLARKE, K** ; **ROTHWELL, K**. *J. Chem. Soc.*, 1960, 1885
- *Analytical Sciences*, 1996, vol. 12
- **D.H. RIPIN** ; **D.A. EVANS**. *pKa's of Nitrogen Acids, [online*, 22 September 2023
- **D.H. RIPIN** ; **D.A. EVANS**. *pKa's of Inorganic and Oxo-Acids,[online*, 05 December 2017
- Greene's Protective Groups in Organic Synthesis. 2014
- *Bioorganic & Medicinal Chemistry*, 2009, vol. 17, 6106-6122
- *Communications Chemistry*, 2023, vol. 14, 5790